(19) Europäisches Patentamt
European Patent Office
Office européen des brevets

(11) Publication number: **0 488 301 A1**

(12) # EUROPEAN PATENT APPLICATION

(21) Application number: **91120417.0**

(22) Date of filing: **28.11.91**

(51) Int. Cl.5: **C07D 311/68**, C07D 405/12, C07D 405/14, A61K 31/35, A61K 31/40, A61K 31/445, A61K 31/45, A61K 31/50, A61K 31/535

(30) Priority: **28.11.90 ES 9003264**

(43) Date of publication of application: **03.06.92 Bulletin 92/23**

(84) Designated Contracting States: **AT BE CH DE DK ES FR GB GR IT LI LU NL SE**

(71) Applicant: **J. URIACH & CIA. S.A.**
**Degà Bahi, 59-67**
**E-08026 Barcelona(ES)**

(72) Inventor: **Almansa, Carmen**
**Sta Eulalia 114**
**ES-08902 Hospit. de Llobregat, Barcelona(ES)**
Inventor: **Carmen, Torres Ma**
**Tramontana 14-16**
**ES-08860 Castelldefels, Barcelona(ES)**
Inventor: **Elena, Carceller**
**Arag n 117**
**ES-08015 Barcelona(ES)**
Inventor: **Javier, Bartroli**
**Vives i Tut 55**
**ES-08034 Barcelona(ES)**

(74) Representative: **Zumstein, Fritz, Dr. et al**
**Dr. F. Zumstein Dipl.-Ing. F. Klingseisen**
**Bräuhausstrasse 4**
**W-8000 München 2(DE)**

(54) **New benzopyran compounds with pharmacological activity.**

(57) The present invention relates to new benzopyranes having the formula **I**:

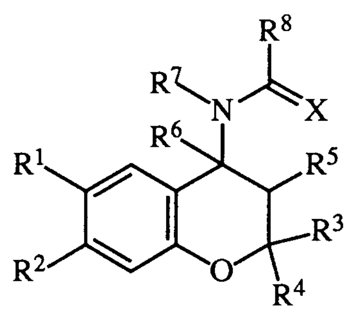

**I**

wherein:
$R^1$ and $R^2$ represent, among others, hydrogen, halogen, cyano, $C_{1-4}$ alkyl, $C_{1-4}$ alkoxy; $R^3$ is hydrogen or $C_{1-4}$ alkyl, and $R^4$ is $C_{1-4}$ alkyl, or $R^3$ and $R^4$ together form a $C_{2-5}$ polymethylene chain; $R^5$ represents hydroxyl or acetoxy and $R^6$ is hydrogen, or $R^5$ together with $R^6$ form a carbonyl group and $R^7$ is hydrogen, or $R^5$ and $R^6$ together form a bond; $R^7$ is hydrogen or $C_{1-6}$ alkyl; $R^8$ is a totally saturated $C_5$-$C_6$ cyclic or heterocyclic group

EP 0 488 301 A1

selected from cyclopentyl, cyclohexyl, pyrrolidinyl, piperidinyl, piperazinyl, imidazolinyl, pyrazolidinyl, isothiazolidinyl, isoxazolidinyl, thiazolidinyl, oxazolidinyl or morpholinyl, which may be optionally substituted by one or two $R^9$ groups; $R^9$ is hydroxyl, oxo, $C_{1-6}$ alkyl, $C_{1-6}$ alkylcarbonyl, $C_{1-6}$ alkoxycarbonyl, $C_{1-6}$ alkoxycarbonylmethyl $C_{1-6}$ alkylaminocarbonyl, $R^{10}$-oxycarbonyl or $R^{10}$-($C_{1-4}$)alkoxycarbonyl, wherein $R^{10}$ is an optionally substituted aryl or heteroaryl group. The invention also relates to a procedure for their preparation and to pharmaceutical compositions containing them. These compounds are antihypertensive and bronchodilator agents.

Field of the invention.

The present invention relates to new benzopyran compounds with pharmacological activity, as well as to a process for their preparation, to pharmaceutical compositions containing them and to their use for the manufacture of medicaments useful in the treatment of mammals, including man. Such benzopyrans have been found to have blood pressure lowering activity, useful in the treatment of hypertension, as well as bronchodilatory activity, useful in the treatment of asthma. They are also indicated in the treatment of other diseases related with the regulation of the smooth muscle contraction in the gastrointestinal, uterus or urinary tract and in the cardiovascular, respiratory or cerebrovascular systems. Such disordes include angina, congestive heart failure, incontinence, irritable bowel syndrome and epilepsy.

Description of the Prior Art.

Several benzopyran compounds which show antihypertensive activity have been described in the literature. However, they are all structurally different from the compounds of the present invention. As examples it may be mentioned:

EP 126311, EP 250077 and EP 139992 describe benzopyran compounds with antihypertensive activity of formula:

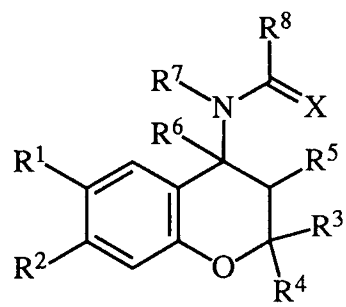

wherein $R^8$ is aryl or heteroaryl optionally substituted by one or more groups or atoms selected from $C_{1-6}$ alkyl, $C_{1-6}$ alkoxy, hydroxyl, halogen, trifluoromethyl, nitro, cyano, $C_{1-12}$ acyl, amino or aminocarbonyl, optionally substituted by one or two $C_{1-6}$ alkyl.

EP 126367 discloses benzopyran compounds with antihypertensive activity of similar formula to the above mentioned, wherein $R^8$ is either $C_{1-6}$ alkyl substituted by an amino group, which may optionally be substituted by one or two $C_{1-6}$ alkyl groups, or $R^8$ is an amino group optionally substituted by a $C_{1-6}$ alkyl, a $C_{2-6}$ alkenyl, a $C_{5-8}$ cycloalkyl or an optionally substituted phenyl group.

EP 95316 describes benzopyran compounds with antihypertensive activity of similar general formula, wherein $R^8$ is hydrogen, $C_{1-6}$ alkyl (optionally substituted by hydroxyl, $C_{1-6}$ alkoxy, $C_{1-6}$ alkoxycarbonyl or carboxyl), $C_{1-2}$ alkyl substituted by halogen, or $C_{2-6}$ alkenyl.

Description of the invention.

The present invention relates to new benzopyran compounds of general formula **I**:

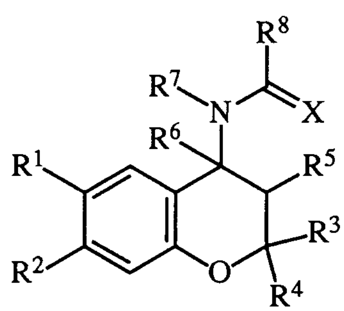

**I**

wherein:

$R^1$ and $R^2$ represent hydrogen, $C_{1-4}$ alkyl, hydroxyl, $C_{1-4}$ alkoxy, formyl, $C_{1-4}$ alkylcarbonyl, $C_{1-4}$ alkylthiocarbonyl, carboxyl, $C_{1-4}$ alkoxycarbonyl, $C_{1-4}$ alkoxythiocarbonyl, $C_{1-4}$ alkylcarbonyloxy, $C_{1-4}$ alkylthiocarbonyloxy, hydroxy-($C_{1-4}$) alkyl, mercapto-($C_{1-4}$) alkyl, perfluoro($C_{1-4}$)alkyl, nitro, amino, cyano, halogen, trifluoromethoxy, ethynyl, trimethylsilylethynyl, $C_{1-4}$ alkylsulphinyl, arylsulphinyl, $C_{1-4}$ alkylsulphonyl, arylsulphonyl, $C_{1-4}$ alkoxysulphinyl, $C_{1-4}$ alkoxysulphonyl, $C_{1-4}$ alkylcarbonylamino, $C_{1-4}$ alkoxycarbonylamino, aminosulphinyl, aminosulphonyl, aminocarbonyl, aminothiocarbonyl, $C_{1-4}$ alkylsulphinylamino, $C_{1-4}$ alkylsulphonylamino, $C_{1-4}$ alkoxysulphinylamino, $C_{1-4}$ alkoxysulphonylamino, ($C_{1-4}$ alkyl)carbonyl($C_{1-4}$ alkyl), nitro-($C_{1-4}$ alkyl), cyano-($C_{1-4}$ alkyl), ($C_{1-4}$ alkyl)C(=NOH), ($C_{1-4}$ alkyl)C(=$NNH_2$) or ($C_{1-4}$ alkoxy)C(=NH), being the above amino groups optionally substituted by one or two $C_{1-4}$ alkyl groups;

$R^3$ is hydrogen or $C_{1-4}$ alkyl; $R^4$ is $C_{1-4}$ alkyl, or $R^3$ and $R^4$ form a $C_{2-5}$ polymethylene chain;

$R^5$ is OH or -$OCOCH_3$ and $R^6$ is hydrogen, or $R^5$ and $R^6$ together form a bond;

$R^7$ is hydrogen or $C_{1-6}$ alkyl;

$R^8$ is a totally saturated $C_5$-$C_6$ cyclic or heterocyclic group selected from cyclopentyl, cyclohexyl, pyrrolidinyl, piperidinyl, piperazinyl, imidazolinyl, pyrazolidinyl, isothiazolidinyl, isoxazolidinyl, thiazolidinyl, oxazolidinyl or morpholinyl, which may be optionally substituted by one or two $R^9$ groups;

$R^9$ is a hydroxyl, oxo, $C_{1-6}$ alkyl, $C_{1-6}$ alkylcarbonyl, $C_{1-6}$ alkoxycarbonyl, $C_{1-6}$ alkoxycarbonylmethyl $C_{1-6}$ alkylaminocarbonyl, $R^{10}$-oxycarbonyl or $R^{10}$-($C_{1-4}$)alkoxycarbonyl group;

$R^{10}$ is a 5- or 6-membered monocyclic or a 9- or 10-membered bicyclic aryl or heteroaryl group, which may be optionally substituted by one or several fluorine, chlorine, bromine or iodine atoms, or hydroxyl, $C_{1-4}$ alkyl, $C_{1-4}$ alkoxy, cyano or nitro groups;

X is an oxygen or sulphur atom;

and the salts thereof.

The invention also provides the use of at least one compound of formula **I** or a pharmaceutically acceptable salt thereof for the manufacture of a medicament for the treatment and/or prevention of the diseases related with the regulation of the smooth muscle contraction at the cardiovascular, respiratory and cerebrovascular systems, and at the gastrointestinal, urinary and uterus tracts, and particularly for the treatment and/or prevention of hypertension and asthma in mammals, including man.

The invention further provides a pharmaceutical composition comprising an effective amount of at least one compound of formula **I** or a pharmaceutically acceptable salt thereof in admixture with a pharmaceutically acceptable excipient.

The invention still further provides processes for preparing the compounds of formula **I**, which in general terms comprise:

(a) when in a compound of general formula **I**, $R^5$ is OH, $R^6$ is hydrogen, X is oxygen and $R^8$ is bound to the C=X group by a carbon atom, reacting a compound of general formula **II**, wherein $R^{1'}$ and $R^{2'}$ are $R^1$ and $R^2$ as above defined or a group or atom convertible thereto, and $R^3$, $R^4$ and $R^7$ have the previously defined meaning,

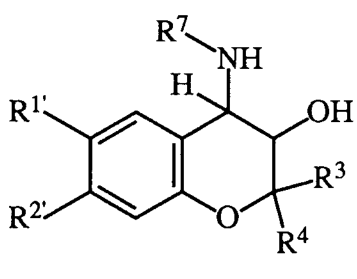

**II**

with an acid of general formula $R^{8a}COOH$ (wherein $R^{8a}$ means an $R^8$ group as above defined optionally substituted by one or two $R^9$ groups and which is bound to the C=X group by a carbon atom) in the presence of dicyclohexylcarbodiimide and 1-hydroxybenzotriazole in a suitable solvent such as dimethylformamide, or alternatively, reacting said compound **II** with an acid chloride of general formula $R^{8a}COCl$ (wherein $R^{8a}$ has the previously defined meaning) in the presence of a base in a suitable solvent such as chloroform, to give a compound of formula **I**, and optionally modifying its substituent $R^{8a}$ by standard chemical reactions involving the manipulation of amine protecting groups and the reaction of said amines with suitable electrophylic reagents; or

(b) when in a compound of general formula **I**, $R^5$ is OH, $R^6$ is hydrogen, X is oxygen and $R^8$ is bound to the C=X group by a nitrogen atom, reacting a compound of formula **II** with phenyl chloroformiate in a suitable solvent such as chloroform, to give a compound of general formula **XII**:

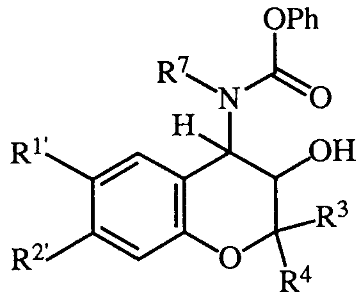

**XII**

wherein $R^{1'}$, $R^{2'}$, $R^3$, $R^4$ and $R^7$ have the previously defined meaning, and then reacting said compound **XII** with a compound of formula $R^{8d}$-H (**XIII**, wherein $R^{8d}$ is an $R^8$ group as above defined optionally substituted by one or two $R^9$ groups and which is bound to the C=X group by a nitrogen atom) in a suitable solvent such as butanol or else using **XIII** itself as solvent; or

(c) when in a compound of general formula **I**, $R^5$ is OH, $R^6$ is hydrogen, X is sulphur and $R^8$ is bound to the C=X group by a nitrogen atom, reacting a compound of formula **II** with carbon sulphide in the presence of a base such as potassium hydroxide in a suitable solvent such as dimethylformamide, and subsequently treating the intermediate obtained with methyl iodide in a suitable solvent such as methanol, to give a compound of general formula **XIV**:

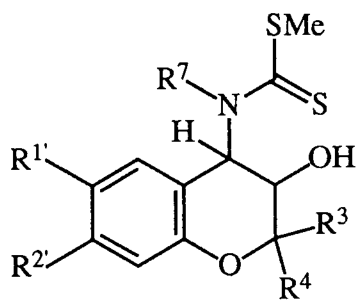

**XIV**

wherein $R^{1'}$, $R^{2'}$, $R^3$, $R^4$ and $R^7$ have the previously defined meaning, and finally reacting said compound **XIV** with a compound of general formula $R^{8d}$-H(**XIII**, wherein $R^{8d}$ has the previously defined meaning) in a suitable solvent such as butanol or else using **XIII** itself as solvent; or alternatively, in all cases wherein X is sulphur treating a compound of formula **I** wherein X is oxygen with a thiation agent such as Lawesson's reagent in a suitable solvent such as toluene;

(d) in all cases wherein $R^5$ is an acetoxy group and $R^6$ is hydrogen, reacting a compound of formula **I** wherein $R^5$ is hydroxyl and $R^6$ is hydrogen with acetic anhydride in the presence of a base such as pyridine;

(e) in all cases wherein $R^5$ and $R^6$ together form a bond, reacting a compound of general formula **I** wherein $R^5$ is OH and $R^6$ is hydrogen with a base such as sodium hydride or sodium hydroxide in a suitable solvent such as toluene or dioxane; or alternatively, reacting a compound of general formula **I** wherein $R^5$ is acetoxy and $R^6$ is hydrogen with a base such as 1,8-diazabicyclo(5.4.0)undec-7-ene (DBU) in an inert solvent such as toluene;

(f) optionally, interconverting the groups $R^1$, $R^2$, $R^{1'}$ and/or $R^{2'}$ in a compound of formula **I** or any intermediate of formula **II, XII** or **XIV** into other groups $R^1$ and/or $R^2$;

(g) and optionally, reacting a compound of formula **I** with an acid to give its corresponding acid addition salt.

In the compounds of the present invention, a "$C_{1-n}$ alkyl" group means, unless otherwise specified, a linear or branched alkyl chain containing from 1 to n carbon atoms. Therefore, when n is 4 this term can be methyl, ethyl, propyl, isopropyl, butyl, isobutyl, *sec*-butyl or *tert*-butyl, of which methyl, ethyl, propyl,

isopropyl, butyl and isobutyl are preferred, methyl and ethyl are more preferred, and methyl is most preferred. Examples of n being 6 include methyl, ethyl, propyl, isopropyl, butyl, isobutyl, sec-butyl, tert-butyl, pentyl, isopentyl, neopentyl, hexyl, isohexyl, 1-methylpentyl, 2-methylpentyl and 3-methylpentyl.

In $R^1$ or $R^2$ a $C_{1-4}$ alkoxy group means a group derived from the union of a $C_{1-4}$ alkyl group to an oxygen atom of an ether functional group. Examples include methoxy, ethoxy, propoxy, isopropoxy, butoxy, isobutoxy, *sec*-butoxy and *tert*-butoxy, of which methoxy, ethoxy, propoxy, isopropoxy, butoxy and isobutoxy are preferred, and methoxy is most preferred.

In $R^1$ or $R^2$ a $C_{1-4}$ alkylcarbonyl group means a group derived from the union of a $C_{1-4}$ alkyl group to a carbonyl group. Examples include acetyl, propanoyl, isopropanoyl, butanoyl, and isobutanoyl, of which acetyl and propanoyl are preferred, and acetyl is most preferred.

In $R^1$ or $R^2$ a $C_{1-4}$ alkylthiocarbonyl group means a group derived from the union of a $C_{1-4}$ alkyl group to a thiocarbonyl group. Examples include thioacetyl, thiopropanoyl, thioisopropanoyl, thiobutanoyl, and thioisobutanoyl, of which thioacetyl and thiopropanoyl are preferred, and thioacetyl is most preferred.

In $R^1$ or $R^2$ a $C_{1-4}$ alkoxycarbonyl group means a group derived from the union of a $C_{1-4}$ alkoxy group, like the above mentioned, to a carbonyl group, and include methoxycarbonyl, ethoxycarbonyl, propoxycarbonyl, isopropoxycarbonyl, butoxycarbonyl, isobutoxycarbonyl, *sec*-butoxycarbonyl and *tert*-butoxycarbonyl, of which methoxycarbonyl, ethoxycarbonyl, propoxycarbonyl, isopropoxycarbonyl, butoxycarbonyl, and isobutoxycarbonyl are preferred, methoxycarbonyl and ethoxycarbonyl are more preferred, and methoxycarbonyl is most preferred.

In $R^1$ or $R^2$ a $C_{1-4}$ alkoxythiocarbonyl group means a group derived from the union of a $C_{1-4}$ alkoxy group, like the above mentioned, to a thiocarbonyl group, and include methoxythiocarbonyl, ethoxythiocarbonyl, propoxythiocarbonyl, isopropoxythiocarbonyl, butoxythiocarbonyl, isobutoxythiocarbonyl, *sec*-butoxythiocarbonyl and *tert*-butoxythiocarbonyl, of which methoxythiocarbonyl, ethoxythiocarbonyl, propoxythiocarbonyl, isopropoxythiocarbonyl, butoxythiocarbonyl, and isobutoxythiocarbonyl are preferred, methoxythiocarbonyl and ethoxythiocarbonyl are more preferred, and methoxythiocarbonyl is most preferred.

In $R^1$ or $R^2$ a $C_{1-4}$ alkylcarbonyloxy group means a group derived from the union of a $C_{1-4}$ alkylcarbonyl group to an oxygen atom. Examples include acetoxy, propanoxy, isopropanoxy, butanoxy, and isobutanoxy, of which acetoxy and propanoxy are preferred, and acetoxy is most preferred.

In $R^1$ or $R^2$ a $C_{1-4}$ alkylthiocarbonyloxy group means a group derived from the union of a $C_{1-4}$ alkylthiocarbonyl group to an oxygen atom. Examples include thioacetoxy, thiopropanoxy, thioisopropanoxy, thiobutanoxy, and thioisobutanoxy, of which thioacetoxy and thiopropanoxy are preferred, and thioacetoxy is most preferred.

In $R^1$ or $R^2$ a hydroxy-$C_{1-4}$ alkyl group means a group resulting from the substitution of one hydrogen atom of the above mentioned "$C_{1-4}$ alkyl" group by an hydroxyl group. Examples include hydroxymethyl, 1-hydroxyethyl, 2-hydroxyethyl, 1-hydroxypropyl, 2-hydroxypropyl, and 3-hydroxypropyl, of which hydroxymethyl, 1-hydroxyethyl and 2-hydroxyethyl are preferred.

In $R^1$ or $R^2$ a mercapto-$C_{1-4}$ alkyl group means a group resulting from the substitution of one hydrogen atom of the above mentioned "$C_{1-4}$ alkyl" group by a mercapto group. Examples include mercaptomethyl, 1-mercaptoethyl, 2-mercaptoethyl, 1-mercaptopropyl, 2-mercaptopropyl, and 3-mercaptopropyl, of which mercaptomethyl, 1-mercaptoethyl and 2-mercaptoethyl are preferred.

In $R^1$ or $R^2$ a perfluoro($C_{1-4}$)alkyl group means a $C_{1-4}$ alkyl group in which all hydrogen atoms have been substituted by fluorine atoms. Examples include trifluoromethyl, pentafluoroethyl, heptafluoropropyl, and nonafluorobutyl, of which trifluoromethyl and pentafluoroethyl are preferred.

In a compound of formula **I**, an amino group may be optionally substituted by one or two $C_{1-4}$ alkyl groups. An amino group substituted by one or two $C_{1-4}$ alkyl groups means a group resulting from the substitution of one or two hydrogen atoms of the amino group by a $C_{1-4}$ alkyl group. When the amino group is substituted by two $C_{1-4}$ alkyl groups, they can be the same or different. Examples include amino, methylamino, dimethylamino, ethylamino, diethylamino, ethylmethylamino, propylamino, dipropylamino, isopropylamino, and diisopropylamino, of which methylamino, dimethylamino, ethylamino and diethylamino are preferred, and methylamino and dimethylamino are most preferred.

The term "halogen" means fluorine, chlorine, bromine or iodine.

In $R^1$ or $R^2$ a $C_{1-4}$ alkylsulphinyl group means a group derived from the union of a $C_{1-4}$ alkyl group to a sulphinyl group. Examples include methylsulphinyl, ethylsulphinyl, propylsulphinyl, isopropylsulphinyl, butylsulphinyl, isobutylsulphinyl, *sec*-butylsulphinyl and *tert*-butylsulphinyl, of which methylsulphinyl, ethylsulphinyl, propylsulphinyl, isopropylsulphinyl, butylsulphinyl and isobutylsulphinyl are preferred, and methylsulphinyl is most preferred.

The term "aryl" represents a phenyl group or a phenyl group substituted by a fluorine, chlorine, bromine or iodine atom, or a methyl, hydroxyl, methoxy, cyano or nitro group. Examples include phenyl, 2-methylphenyl, 4-methylphenyl, 4-chlorophenyl, 4-bromophenyl, 4-methoxyphenyl, 2-methoxyphenyl, and 4-cyanophenyl.

In $R^1$ or $R^2$ an arylsulphinyl group means a group derived from the union of an aryl group, like the above mentioned, to a sulphinyl group. Examples include phenylsulphinyl, 2-methylphenylsulphinyl, 4-methylphenylsulphinyl, 4-chlorophenylsulphinyl, 4-bromophenylsulphinyl, 4-methoxyphenylsulphinyl, 2-methoxyphenylsulphinyl, and 4-cyanophenylsulphinyl,of which phenylsulphinyl is preferred.

In $R^1$ or $R^2$ a $C_{1-4}$ alkylsulphonyl group means a group derived from the union of a $C_{1-4}$ alkyl group to a sulphonyl group. Examples include methylsulphonyl, ethylsulphonyl, propylsulphonyl, isopropylsulphonyl, butylsulphonyl, isobutylsulphonyl, *sec*-butylsulphonyl and *tert*-butylsulphonyl, of which methylsulphonyl, ethylsulphonyl, propylsulphonyl, isopropylsulphonyl, butylsulphonyl and isobutylsulphonyl are preferred, and methylsulphonyl is most preferred.

In $R^1$ or $R^2$ an arylsulphonyl group means a group derived from the union of an aryl group, like the above mentioned, to a sulphonyl group. Examples include phenylsulphonyl, 2-methylphenylsulphonyl, 4-methylphenylsulphonyl, 4-chlorophenylsulphonyl, 4-bromophenylsulphonyl, 4-methoxyphenylsulphonyl, 2-methoxyphenylsulphonyl, and 4-cyanophenylsulphonyl, of which phenylsulphonyl is preferred.

In $R^1$ or $R^2$ a $C_{1-4}$ alkoxysulphinyl group means a group derived from the union of a $C_{1-4}$ alkoxy group to a sulphinyl group. Examples include methoxysulphinyl, ethoxysulphinyl, propoxysulphinyl, isopropoxysulphinyl, butoxysulphinyl, isobutoxysulphinyl, *sec*-butoxysulphinyl and *tert*-butoxysulphinyl, of which methoxysulphinyl, ethoxysulphinyl, propoxysulphinyl, isopropoxysulphinyl, butoxysulphinyl and isobutoxysulphinyl are preferred, and methoxysulphinyl is most preferred.

In $R^1$ or $R^2$ a $C_{1-4}$ alkoxysulphonyl group means a group derived from the union of a $C_{1-4}$ alkoxy group to a sulphonyl group. Examples include methoxysulphonyl, ethoxysulphonyl, propoxysulphonyl, isopropoxysulphonyl, butoxysulphonyl, isobutoxysulphonyl, *sec*-butoxysulphonyl and *tert*-butoxysulphonyl, of which methoxysulphonyl, ethoxysulphonyl, propoxysulphonyl, isopropoxysulphonyl, butoxysulphonyl and isobutoxysulphonyl are preferred, and methoxysulphonyl is most preferred.

In $R^1$ or $R^2$ a $C_{1-4}$ alkylcarbonylamino group means a group derived from the substitution of an hydrogen atom of an amino group, like the above mentioned, by a $C_{1-4}$ alkylcarbonyl group. Examples include acetamido, N-methylacetamido, propanamido, N-methylpropanamido, and isopropanamido, of which acetamido, N-methylacetamido, propanamido and N-methylpropanamido are preferred, and acetamido and N-methylacetamido are most preferred.

In $R^1$ or $R^2$ a $C_{1-4}$ alkoxycarbonylamino group means a group derived from the substitution of an hydrogen atom of an amino group, like the above mentioned, by a $C_{1-4}$ alkoxycarbonyl group. Examples include methoxycarbonylamino, ethoxycarbonylamino, propoxycarbonylamino, isopropoxycarbonylamino, butoxycarbonylamino, and isobutoxycarbonylamino, of which methoxycarbonylamino and ethoxycarbonylamino are preferred, and methoxycarbonylamino is most preferred.

In $R^1$ or $R^2$ an aminosulphinyl group means a group derived from the union of an amino group, like the above mentioned, to a sulphinyl group, and includes, among others, aminosulphinyl, methylaminosulphinyl, dimethylaminosulphinyl, ethylaminosulphinyl, diethylaminosulphinyl, ethylmethylaminosulphinyl, propylaminosulphinyl, dipropylaminosulphinyl, isopropylaminosulphinyl, and diisopropylaminosulphinyl, of which aminosulphinyl, methylaminosulphinyl, dimethylaminosulphinyl, ethylaminosulphinyl, and diethylaminosulphinyl are preferred, and aminosulphinyl, methylaminosulphinyl and dimethylaminosulphinyl are most preferred.

In $R^1$ or $R^2$ an aminosulphonyl group means a group derived from the union of an amino group, like the above mentioned, to a sulphonyl group, and includes, among others, aminosulphonyl, methylaminosulphonyl, dimethylaminosulphonyl, ethylaminosulphonyl, diethylaminosulphonyl, ethylmethylaminosulphonyl, propylaminosulphonyl, dipropylaminosulphonyl, isopropylaminosulphonyl, and diisopropylaminosulphonyl, of which aminosulphonyl, methylaminosulphonyl, dimethylaminosulphonyl, ethylaminosulphonyl, and diethylaminosulphonyl are preferred, and aminosulphonyl, methylaminosulphonyl and dimethylaminosulphonyl are most preferred.

In $R^1$ or $R^2$ an aminocarbonyl group means a group derived from the union of an amino group, like the above mentioned, to a carbonyl group. Examples include aminocarbonyl, methylaminocarbonyl, dimethylaminocarbonyl, ethylaminocarbonyl, diethylaminocarbonyl, ethylmethylaminocarbonyl, propylaminocarbonyl, dipropylaminocarbonyl, isopropylaminocarbonyl, and diisopropylaminocarbonyl, of which aminocarbonyl, methylaminocarbonyl, dimethylaminocarbonyl, ethylaminocarbonyl and diethylaminocarbonyl are preferred, and aminocarbonyl, methylaminocarbonyl and dimethylaminocarbonyl are most preferred.

In $R^1$ or $R^2$ an aminothiocarbonyl group means a group derived from the union of an amino group, like the above mentioned, to a thiocarbonyl group. Examples include aminothiocarbonyl, methylaminothiocarbonyl, dimethylaminothiocarbonyl, ethylaminothiocarbonyl, diethylaminothiocarbonyl, ethylmethylaminothiocarbonyl, propylaminothiocarbonyl, dipropylaminothiocarbonyl, isopropylaminothiocarbonyl, and diisopropylaminothiocarbonyl, of which aminothiocarbonyl, methylaminothiocarbonyl, dimethylaminothiocarbonyl, ethylaminothiocarbonyl and diethylaminothiocarbonyl are preferred, and aminothiocarbonyl, methylaminothiocarbonyl and dimethylaminothiocarbonyl are most preferred.

In $R^1$ or $R^2$ a $C_{1-4}$ alkylsulphinylamino group means a group resulting from the substitution of an hydrogen atom of an amino group, like the above mentioned, by a $C_{1-4}$ alkylsulphinyl group. Examples include methylsulphinylamino, ethylsulphinylamino, propylsulphinylamino, isopropylsulphinylamino, butylsulphinylamino, isobutylsulphinylamino, *sec*-butylsulphinylamino and *tert*-butylsulphinylamino, of which methylsulphinylamino and ethylsulphinylamino are preferred, and methylsulphinylamino is most preferred.

In $R^1$ or $R^2$ a $C_{1-4}$ alkylsulphonylamino group means a group resulting from the substitution of an hydrogen atom of an amino group, like the above mentioned, by a $C_{1-4}$ alkylsulphonyl group. Examples include methylsulphonylamino, ethylsulphonylamino, propylsulphonylamino, isopropylsulphonylamino, butylsulphonylamino, isobutylsulphonylamino, *sec*-butylsulphonylamino and *tert*-butylsulphonylamino, of which methylsulphonylamino and ethylsulphonylamino are preferred, and methylsulphonylamino is most preferred.

In $R^1$ or $R^2$ a $C_{1-4}$ alkoxysulphinylamino group means a group resulting from the substitution of an hydrogen atom of an amino group, like the above mentioned, by a $C_{1-4}$ alkoxysulphinyl group. Examples include methoxysulphinylamino, ethoxysulphinylamino, propoxysulphinylamino, isopropoxysulphinylamino, butoxysulphinylamino, isobutoxysulphinylamino, *sec*-butoxysulphinylamino and *tert*-butoxysulphinylamino, of which methoxysulphinylamino and ethoxysulphinylamino are preferred, and methoxysulphinylamino is most preferred.

In $R^1$ or $R^2$ a $C_{1-4}$ alkoxysulphonylamino group means a group resulting from the substitution of an hydrogen atom of an amino group, like the above mentioned, by a $C_{1-4}$ alkoxysulphonyl group. Examples include methoxysulphonylamino, ethoxysulphonylamino, propoxysulphonylamino, isopropoxysulphonylamino, butoxysulphonylamino, isobutoxysulphonylamino, *sec*-butoxysulphonylamino and *tert*-butoxysulphonylamino, of which methoxysulphonylamino and ethoxysulphonylamino are preferred, and methoxysulphonylamino is most preferred.

In $R^1$ or $R^2$ a ($C_{1-4}$ alkyl)carbonyl($C_{1-4}$ alkyl) group means a group derived from the union of a ($C_{1-4}$ alkyl)carbonyl group, like the above mentioned, to a $C_{1-4}$ alkyl group. Preferred examples are 2-oxopropyl, 2-oxobutyl,3-oxobutyl and 3-oxopentyl.

In $R^1$ or $R^2$ a nitro-($C_{1-4}$ alkyl) group means a group resulting from the substitution of an hydrogen atom of a $C_{1-4}$ alkyl group by a nitro group. Examples include nitromethyl, 1-nitroethyl, 2-nitroethyl, 1-nitropropyl, 2-nitropropyl, and 3-nitropropyl, of which nitromethyl, 1-nitroethyl and 2-nitroethyl are preferred.

In $R^1$ or $R^2$ a cyano-($C_{1-4}$ alkyl) group means a group resulting from the substitution of an hydrogen atom of a $C_{1-4}$ alkyl group by a cyano group. Examples include cyanomethyl, 1-cyanoethyl, 2-cyanoethyl, 1-cyanopropyl, 2-cyanopropyl, and 3-cyanopropyl, of which cyanomethyl, 1-cyanoethyl and 2-cyanoethyl are preferred.

Examples of ($C_{1-4}$ alkyl)C(=NOH) include 1-oximinoethyl, 1-oximinopropyl, 1-oximinobutyl, 2-methyl-1-oximinopropyl, and 1-oximinopentyl, of which 1-oximinoethyl and 1-oximinopropyl are preferred, and 1-oximinoethyl is most preferred.

Examples of ($C_{1-4}$ alkyl)C(=$NNH_2$) include 1-hidrazonoethyl, 1-hidrazonopropyl, 1-hidrazonobutyl, 2-methyl-1-hidrazonopropyl, and 1-hidrazonopentyl, of which 1-hidrazonoethyl and 1-hidrazonopropyl are preferred, and 1-hidrazonoethyl is most preferred.

Examples of ($C_{1-4}$ alkoxy)C(=NH) include methyl imidate, ethyl imidate, propyl imidate, isopropyl imidate, and butyl imidate, of which methyl imidate and ethyl imidate are preferred, and methyl imidate is most preferred.

In a compound of formula **I**, $R^3$ and $R^4$ are preferred to be both $C_{1-4}$ alkyl, more preferably methyl or ethyl, and most preferably methyl.

In a compound of formula **I**, $R^7$ is preferred to be hydrogen or $C_{1-4}$ alkyl, and more preferably hydrogen or methyl.

Examples of $R^8$ include cyclopentyl, cyclohexyl, 1-pyrrolidinyl, 2-pyrrolidinyl, 1-piperidinyl, 2-piperidinyl, 3-piperidinyl, 4-piperidinyl, 1-piperazinyl, 1-imidazolidinyl, 1-pyrazolidinyl, 2-isothiazolidinyl, 2-isoxazolidinyl, 3-thiazolidinyl, 3-oxazolidinyl or 1-morpholinyl, of which cyclopentyl, cyclohexyl, 1-pyrrolidinyl, 2-pyrrolidinyl, 1-piperidinyl, 2-piperidinyl, 3-piperidinyl, 4-piperidinyl, 1-piperazinyl, and 1-morpholinyl are preferred, and cyclohexyl, 1-pyrrolidinyl, 2-pyrrolidinyl, 1-piperazinyl, 1-piperidinyl, and 2-piperidinyl are most preferred. In all cases $R^8$ may optionally be substituted by an $R^9$ group.

In $R^9$, a $C_{1-6}$ alkylcarbonyl group means a group derived from the union of a $C_{1-6}$ alkyl group, like the above mentioned, to a carbonyl group. Examples include acetyl, propanoyl, isopropanoyl, butanoyl,

isobutanoyl, sec-butanoyl, tert-butanoyl, pentanoyl, isopentanoyl, neopentanoyl, hexanoyl, of which acetyl, propanoyl, isopropanoyl, butanoyl, isobutanoyl, sec-butanoyl, tert-butanoyl are preferred, and acetyl and tert-butanoyl are more preferred.

In $R^9$, a $C_{1-6}$ alkoxycarbonyl group means a group derived from the union of a $C_{1-6}$ alkoxy group to a carbonyl group, and includes methoxycarbonyl, ethoxycarbonyl, propoxycarbonyl, isopropoxycarbonyl, butoxycarbonyl, isobutoxycarbonyl, *sec*-butoxycarbonyl, *tert*-butoxycarbonyl, pentoxycarbonyl, isopentoxycarbonyl, neopentoxycarbonyl, hexoxycarbonyl, isohexoxycarbonyl, 1-methylpentoxycarbonyl, 2-methylpentoxycarbonyl, and 3-methylpentoxycarbonyl, of which methoxycarbonyl, ethoxycarbonyl, propoxycarbonyl, isopropoxycarbonyl, butoxycarbonyl, isobutoxycarbonyl, *sec*-butoxycarbonyl, *tert*-butoxycarbonyl and hexoxycarbonyl are preferred, methoxycarbonyl, ethoxycarbonyl, isopropoxycarbonyl, isobutoxycarbonyl, *tert*-butoxycarbonyl and hexoxycarbonyl are more preferred, and *tert*-butoxycarbonyl is most preferred.

In $R^9$, a $C_{1-6}$ alkoxycarbonylmethyl group means a group derived from the substitution of an hydrogen atom of a methyl group by a $C_{1-6}$ alkoxycarbonyl group, like the above mentioned, and includes methoxycarbonylmethyl, ethoxycarbonylmethyl, propoxycarbonylmethyl, isopropoxycarbonylmethyl, butoxycarbonylmethyl, isobutoxycarbonylmethyl, *sec*-butoxycarbonylmethyl, *tert*-butoxycarbonylmethyl, pentoxycarbonylmethyl, isopentoxycarbonylmethyl, neopentoxycarbonylmethyl, hexoxycarbonylmethyl, isohexoxycarbonylmethyl, of which methoxycarbonylmethyl, ethoxycarbonylmethyl, propoxycarbonylmethyl, isopropoxycarbonylmethyl, butoxycarbonylmethyl, isobutoxycarbonylmethyl, *sec*-butoxycarbonylmethyl, *tert*-butoxycarbonylmethyl are preferred, and *tert*-butoxycarbonylmethyl is most preferred.

In $R^9$, a $C_{1-6}$ alkylaminocarbonyl group is a group resulting from the union of a $C_{1-6}$ alkyl group, like the above mentioned, to a NH-CO group. Examples include methylaminocarbonyl, ethylaminocarbonyl, propylaminocarbonyl, isopropylaminocarbonyl, butylaminocarbonyl, isobutylaminocarbonyl, sec-butylaminocarbonyl, *tert*-butylaminocarbonyl, pentylaminocarbonyl, isopentylaminocarbonyl, neopentylaminocarbonyl, hexylaminocarbonyl, isohexylaminocarbonyl, 1-methylpentylaminocarbonyl, 2-methylpentylaminocarbonyl and 3-methylpentylaminocarbonyl, of which methylaminocarbonyl, ethylaminocarbonyl, propylaminocarbonyl, isopropylaminocarbonyl, butylaminocarbonyl, isobutylaminocarbonyl, sec-butylaminocarbonyl, *tert*-butylaminocarbonyl are preferred, and isopropylaminocarbonyl, isobutylaminocarbonyl, and *tert*-butylaminocarbonyl are more preferred.

Examples of $R^{10}$ radicals include phenyl, pyridine, pyrimidine, pyrazine, pyridazine, s-triazine, furane, pyrrole, thiophene, thiazole, imidazole, triazole, oxazoline, isoxazoline, quinoline, isoquinoline, indole, benzimidazole, benzofurane, isobenzofurane, isoindole and benzothiophene, being all of them optionally substituted by one or several fluorine, chlorine, bromine or iodine atoms, or hydroxyl, $C_{1-4}$ alkyl, $C_{1-4}$ alkoxy, cyano or nitro groups. Specific examples for $C_{1-4}$ alkyl and $C_{1-4}$ alkoxy are those as mentioned above in connection with $R^1$ and $R^2$.

In $R^9$, examples of an $R^{10}$-($C_{1-4}$)alkoxycarbonyl group include phenylmethyloxycarbonyl, 2-, 3- or 4-methoxyphenylmethyloxycarbonyl, 2-, 3-or 4-chlorophenylmethyloxycarbonyl, 2-, 3-, or 4-fluorophenylmethyloxycarbonyl, 2-phenylethyloxycarbonyl, 2-(2-, 3- or 4-methoxyphenyl)ethyloxycarbonyl, 2-(2-, 3- or 4-chlorophenyl)ethyloxycarbonyl, 2-(2-, 3- or 4-fluorophenyl)ethyloxycarbonyl, 3-phenylpropyloxycarbonyl, 3-(2-, 3- or 4-methoxyphenyl)propyloxycarbonyl, 3-(2-, 3- or 4-chlorophenyl)propyloxycarbonyl, 3-(2-, 3- or 4-fluorophenyl)propyloxycarbonyl, 4-phenylbutyloxycarbonyl, 2-pyridylmethyloxycarbonyl, 3-pyridylmethyloxycarbonyl, 4-pyridylmethyloxycarbonyl, 2-(2-pyridyl)ethyloxycarbonyl, 2-(3-pyridyl)ethyloxycarbonyl, 2-(4-pyridyl)ethyloxycarbonyl, 2-, 4- or 5-pyrimidinylmethyloxycarbonyl, 2- or 3-pyrazinylmethyloxycarbonyl, 3- or 4-pyridazinylmethyloxycarbonyl.

Preferred embodiments of the present invention are those compounds of formula **I** wherein:

$R^1$ is a cyano or nitro group;

$R^2$ is hydrogen;

$R^3$ and $R^4$ are methyl; and

$R^5$, $R^6$, $R^7$, $R^8$ and X have the previously defined meaning.

More preferred embodiments of the present invention are those compounds of formula **I** wherein:

$R^1$ is a cyano group;

$R^2$ is hydrogen;

$R^3$ and $R^4$ are methyl;

$R^5$ is an hydroxyl group and $R^6$ is hydrogen; and

$R^7$, $R^8$ and X have the previously defined meaning.

Still more preferred embodiments of the present invention are those compounds of formula **I** wherein:

$R^1$ is a cyano group;

$R^2$ is hydrogen;

$R^3$ and $R^4$ are methyl;

$R^5$ is an hydroxyl group and $R^6$ is hydrogen;

$R^7$ represents hydrogen or a methyl group;

$R^8$ represents a cyclohexyl, 1-piperazinyl, 1-piperidinyl, or 1-pyrrolidinyl group, which may be optionally substituted by a $C_{1-6}$ alkyl group; and

X has the previously defined meaning,

or those compounds of formula **I** wherein:

$R^1$ is a cyano group;

$R^2$ is hydrogen;

$R^3$ and $R^4$ are methyl;

$R^5$ is an hydroxyl group and $R^6$ is hydrogen;

$R^7$ represents hydrogen or a methyl group;

$R^8$ represents a 2-piperidinyl or 2-pyrrolidinyl group, which may be optionally substituted by an $R^9$ group, wherein $R^9$ is a $C_{1-6}$ alkoxycarbonyl, $R^{10}$-oxycarbonyl or $R^{10}$-($C_{1-4}$)alkoxycarbonyl group, being $R^{10}$ as above defined; and

X has the previously defined meaning.

The formulae of some specific examples are represented below, together with the number corresponding to the example in which their preparation is described:

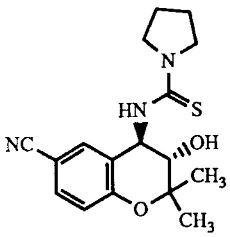

1

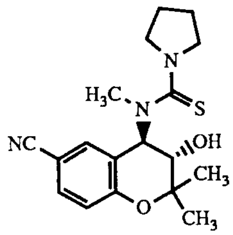

2

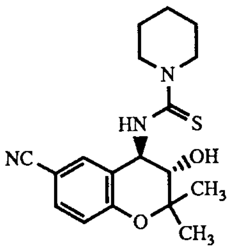

3

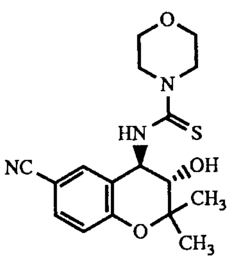

4

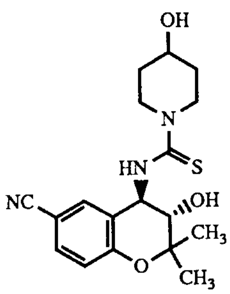

5

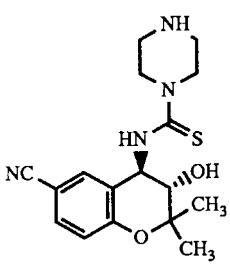

6

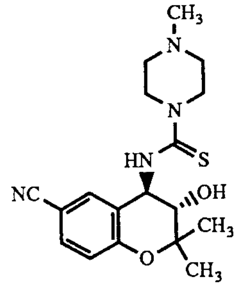

7

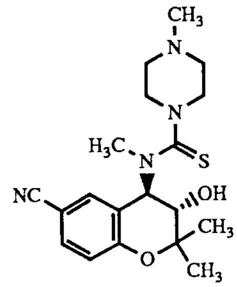

8

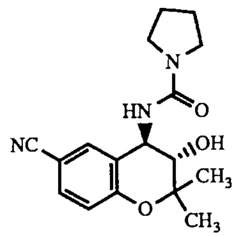

9

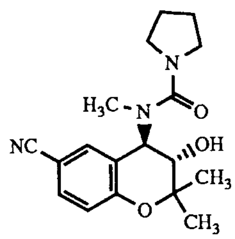

1 0

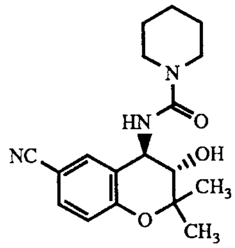

1 1

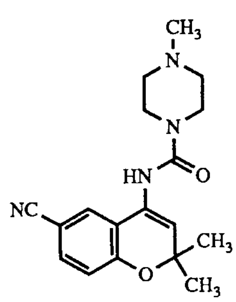

1 2

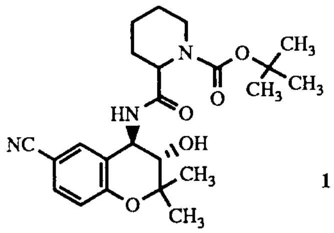

13

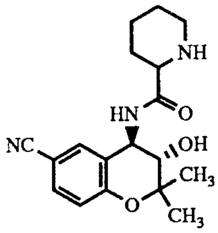

14

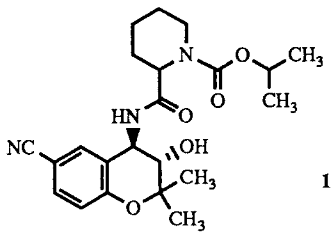

15

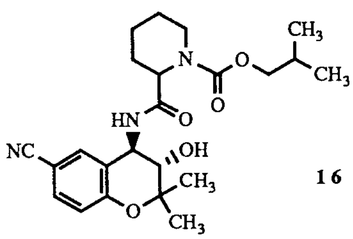

16

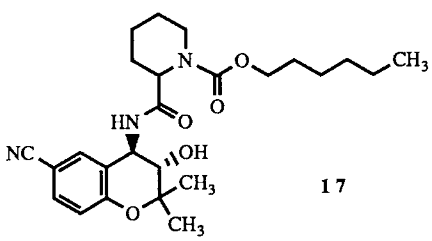

17

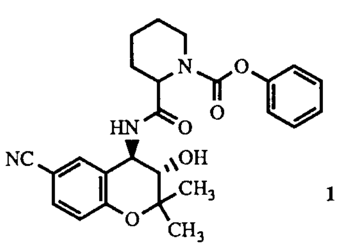

18

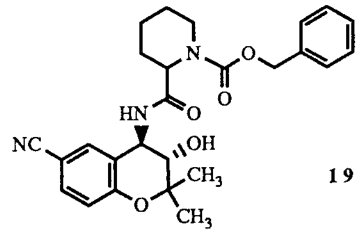

19

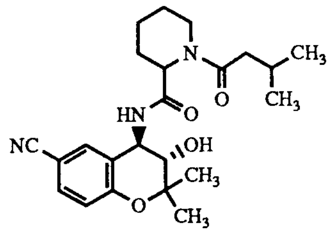

20

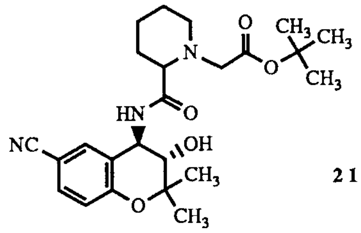

21

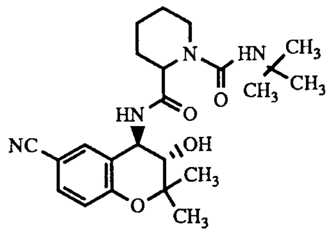

22

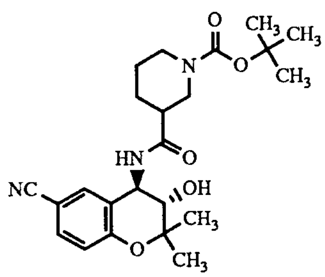

23

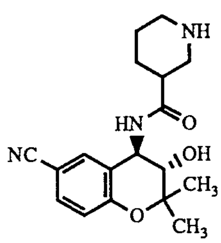

24

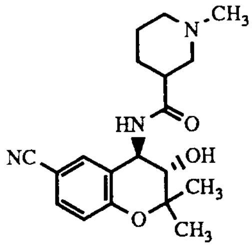

25

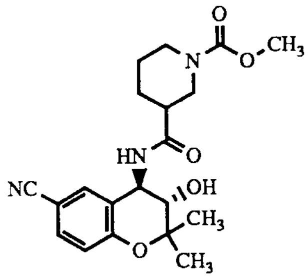

26

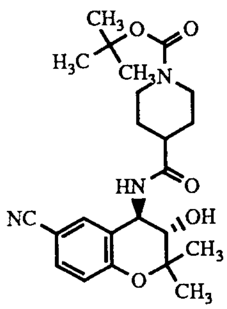

27

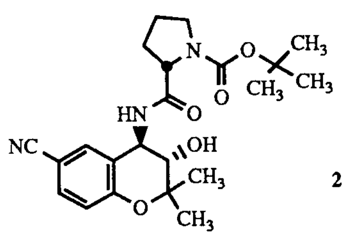

28

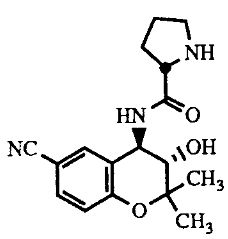

29

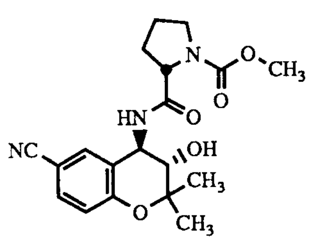

30

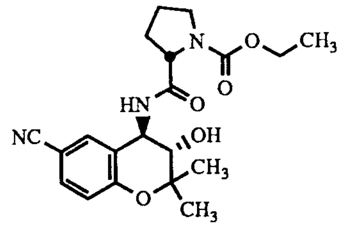

31

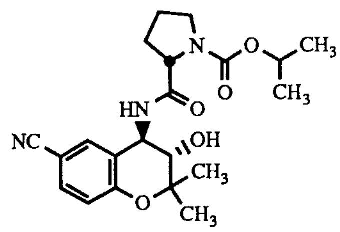

32

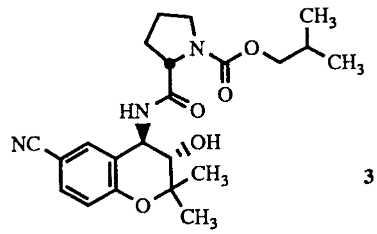

33

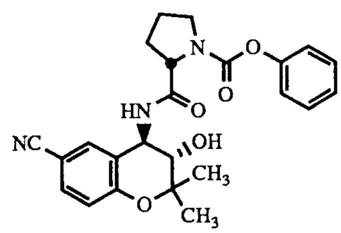

34

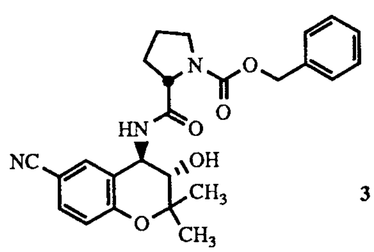

35

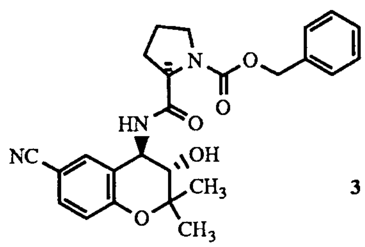

36

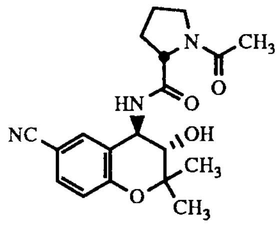
37

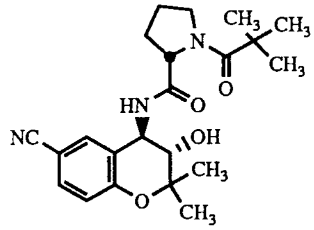
38

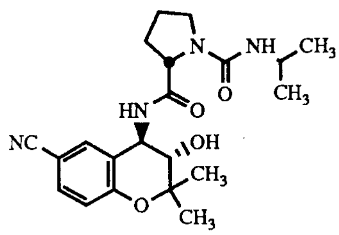
39

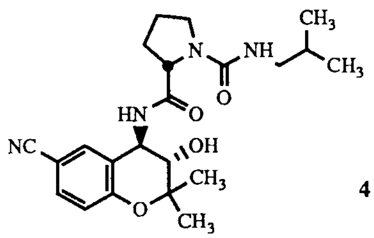
40

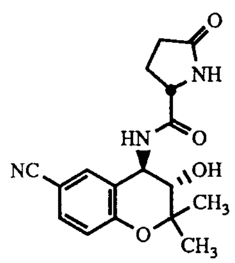
41

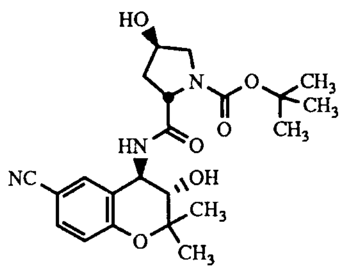
42

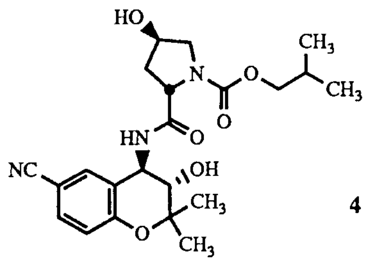
43

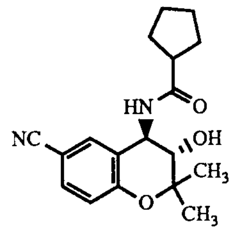
44

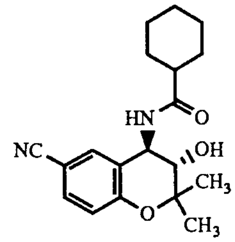
45

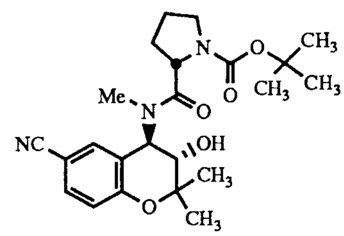
46

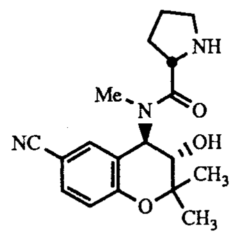
47

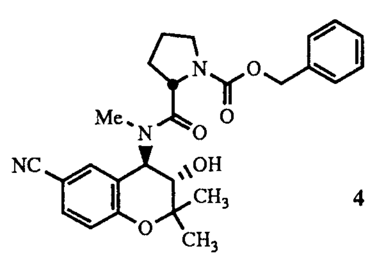
48

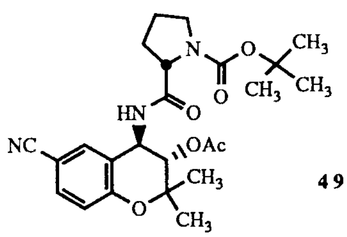

49

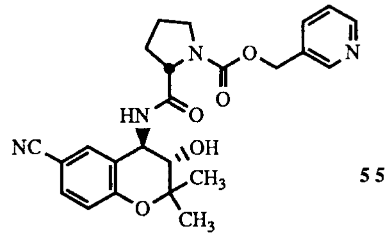

55

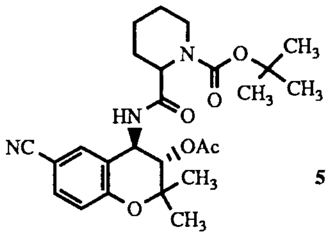

50

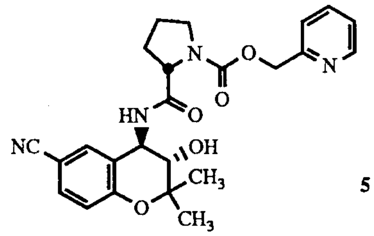

56

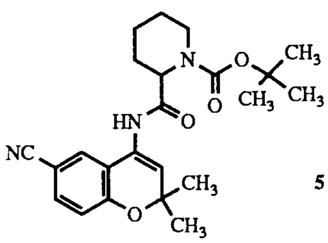

51

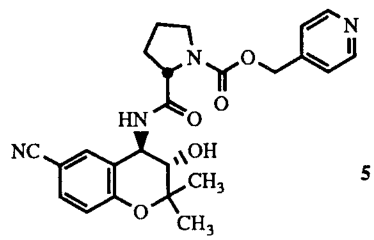

57

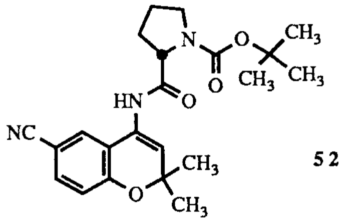

52

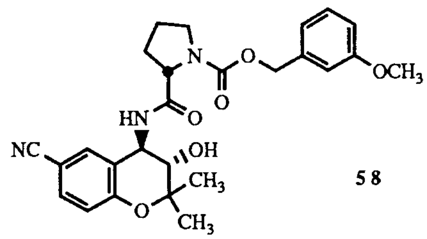

58

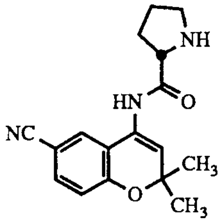

53

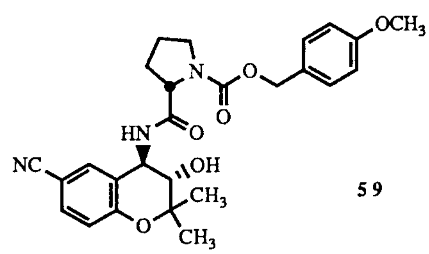

59

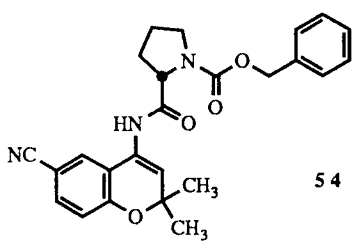

54

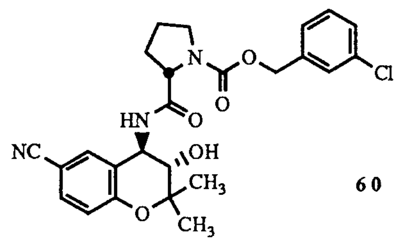

60

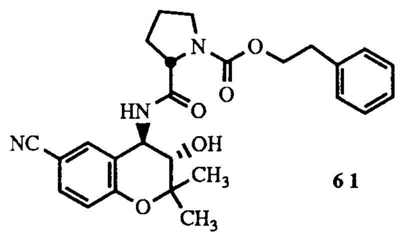

61

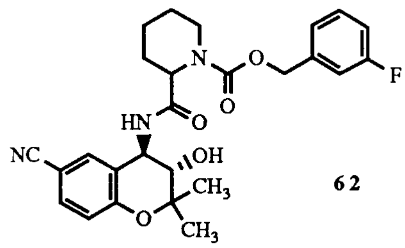

62

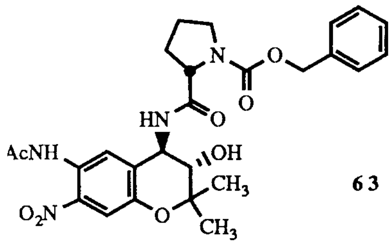

63

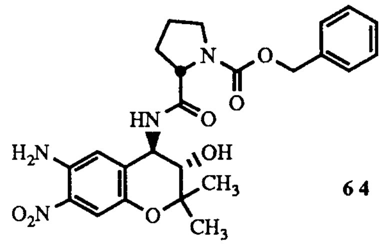

64

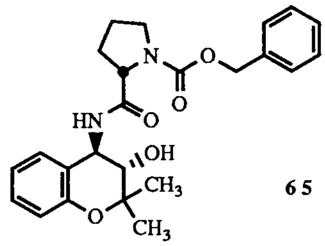

65

Some of the compounds of the present invention contain one or more basic nitrogen atoms and, consequently, they can form salts, which are also included in the present invention. There is no limitation on the nature of these salts but pharmaceutically acceptable ones are preferred. Examples of these salts include: salts with an inorganic acid such as hydrochloric acid, hydrobromic acid, hydriodic acid, nitric acid, perchloric acid, sulfuric acid or phosphoric acid; and salts with an organic acid such as methanesulfonic acid, trifluoromethanesulfonic acid, ethanesulfonic acid, benzenesulfonic acid, *p*-toluenesulfonic acid, fumaric acid, oxalic acid or maleic acid.

The compounds of the present invention can exist as different diastereoisomers and/or optical isomers because the carbons in positions 3 and 4 of the benzopyran skeleton, provided that there is not a double bond between them, are chiral and in some cases there is an additional chiral center. Diastereoisomers can be separated by conventional techniques such as chromatography or fractional crystallization. The optical isomers can be resolved using any of the conventional techniques of optical resolution to give optically pure isomers. Such a resolution can be performed in any chiral synthetic intermediates as well as in the products

of general formula **I**. The optically pure isomers can also be individually obtained using enantiospecific synthesis. The present invention covers the individual isomers as well as their mixtures (e.g. racemic mixtures), being irrelevant if they have been obtained by synthesis or have been prepared physically mixing them up.

The invention also provides processes for preparing the compounds of formula **I**. The precise method used for the preparation of a given compound of the present invention may vary depending on its chemical structure. Schemes 1, 2, 3 and 4 illustrate the general method for their preparation.

SCHEME 1

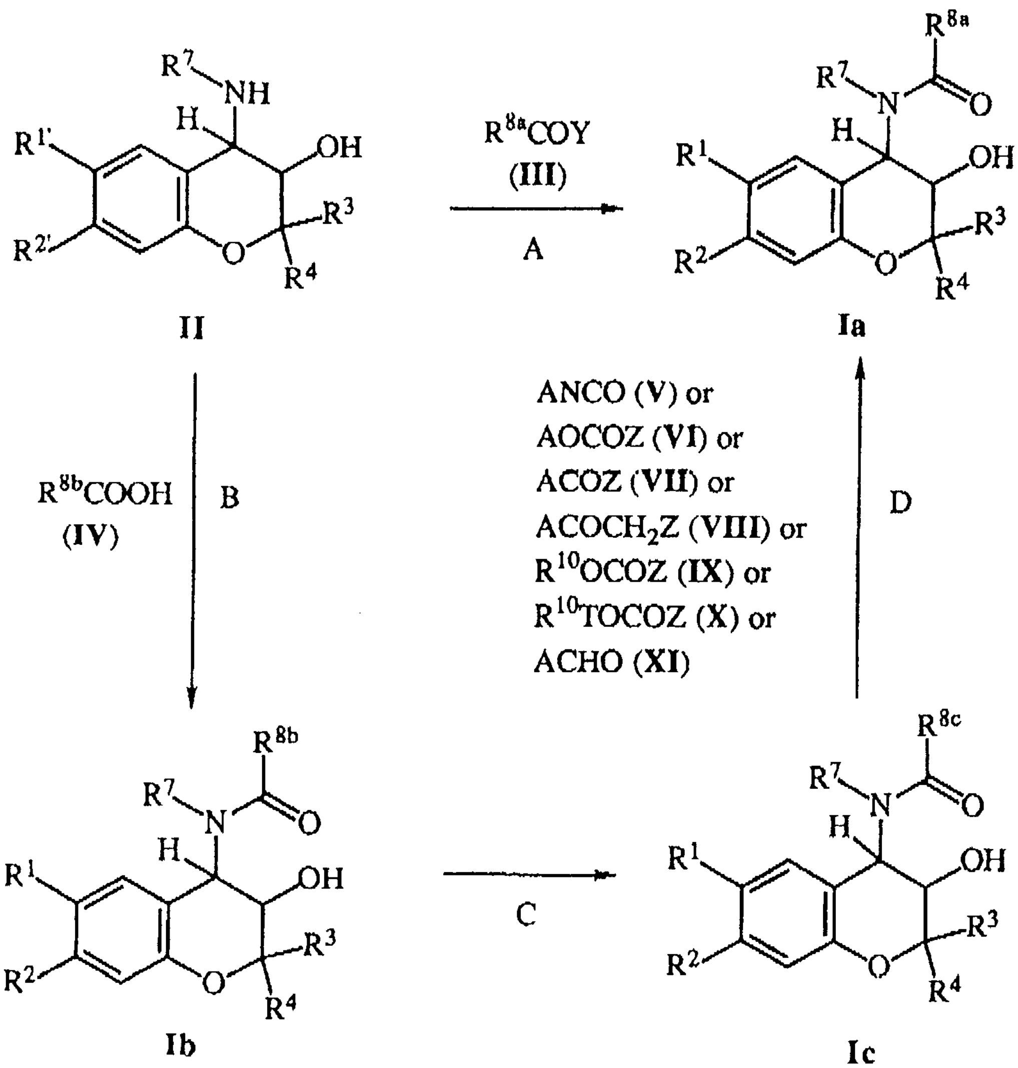

SCHEME 2

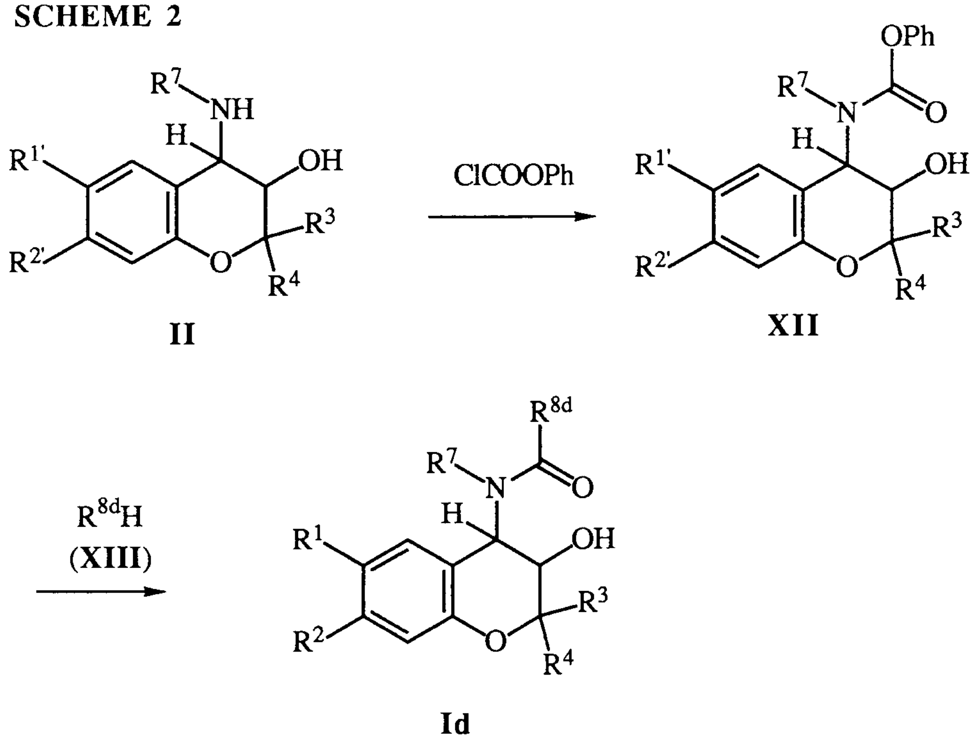


II, XII, Id

SCHEME 3

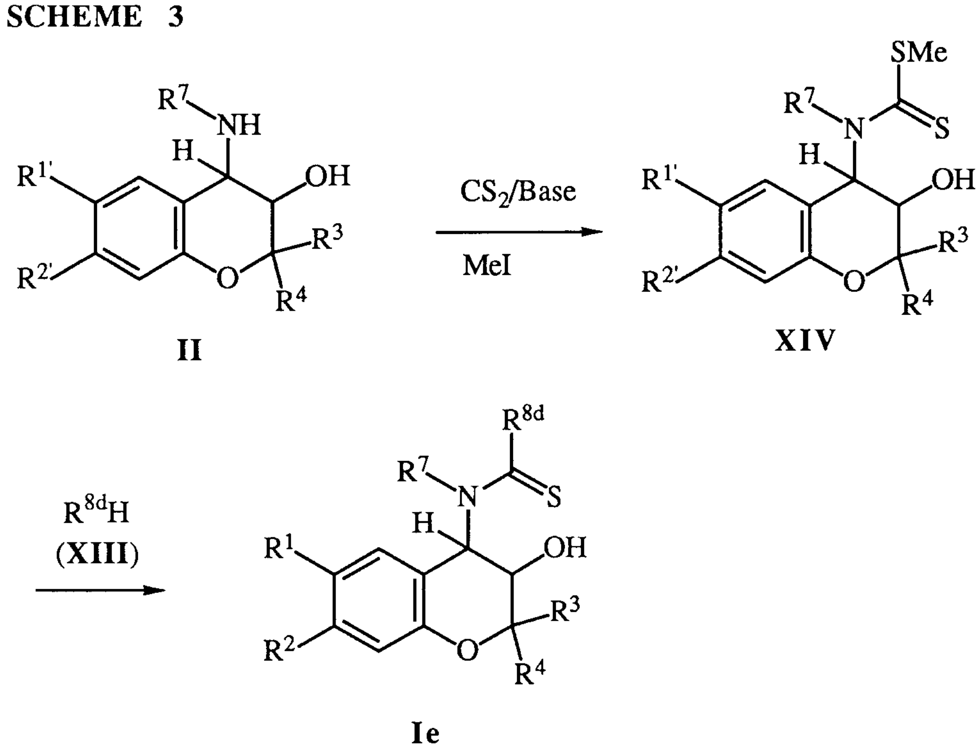


II, XIV, Ie

## SCHEME 4

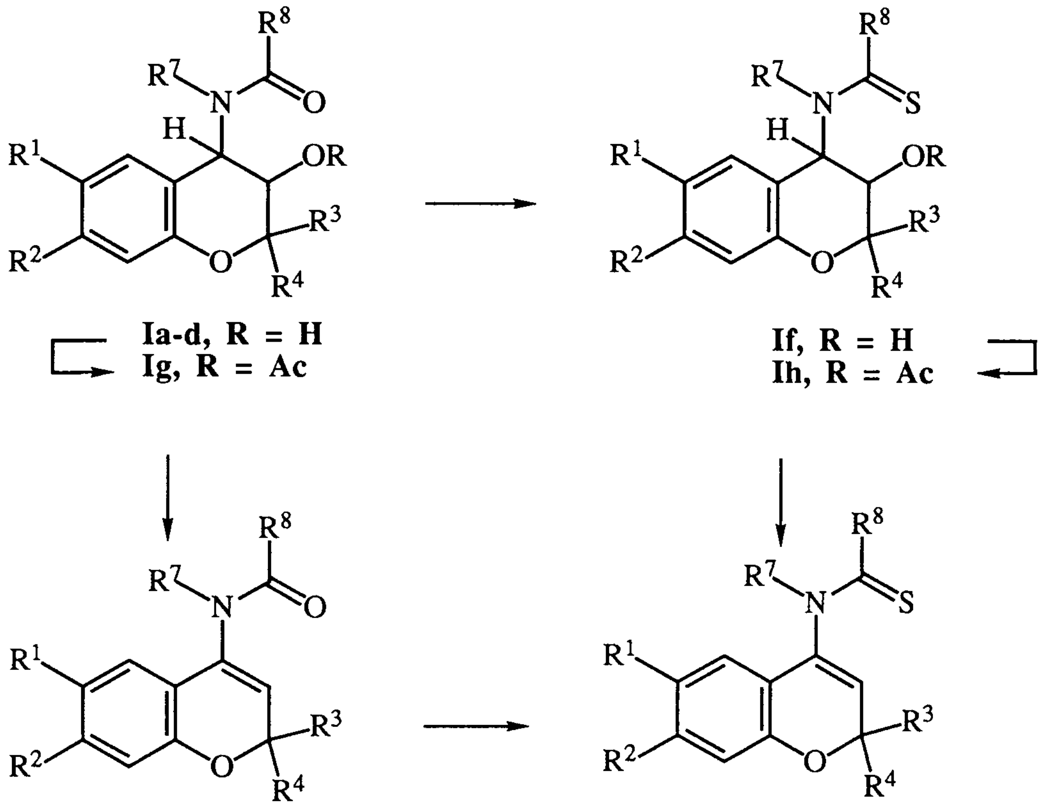

Ia-d, R = H
Ig, R = Ac

If, R = H
Ih, R = Ac

Ii

Ij

Wherein:

$R^{1'}$, $R^{2'}$, $R^1$, $R^2$, $R^3$, $R^4$, $R^7$, $R^8$, $R^{10}$ and X have the previously defined meaning;

Y means a hydroxyl group or an halogen atom, preferably a chlorine or bromine atom;

Z means an halogen atom, preferably a chlorine or bromine atom;

$R^{8a}$ means an $R^8$ group as above defined optionally substituted by one or two $R^9$ groups and which is bound to the C=X group by a carbon atom;

$R^{8b}$ means an $R^8$ group as above defined (optionally substituted by an $R^9$ group) bound to the C=X group by a carbon atom and whose nitrogen atom in position 1 of the ring is protected by a protecting group such as *tert*-butoxycarbonyl;

$R^{8c}$ means an $R^8$ group as above defined (optionally substituted by an $R^9$ group) bound to the C=X group by a carbon atom and being the nitrogen atom in position 1 of the ring unsubstituted;

$R^{8d}$ means an $R^8$ group as above defined optionally substituted by one or two $R^9$ groups and which is bound to the C=X group by a nitrogen atom;

A means a linear or branched alkyl chain of 1 to 6 carbon atoms; and T means a linear or branched chain of 1 to 4 carbon atoms,

Compounds of formula **I**, wherein $R^5$ is OH, $R^6$ is H, X is oxygen and $R^8$ is $R^{8a}$ (that is to say compounds of formula **Ia**), can be prepared by reaction of a compound of general formula **II** with a compound of formula $R^{8a}COY$ (**III**, wherein Y has the previously defined meaning) in a suitable solvent (Step A of Scheme 1).

Preferably, Y is OH and the reaction is carried out in a polar solvent, such as N,N-dimethylformamide, tetrahydrofuran, dioxane, acetonitrile or chloroform, in the presence of a dehydrating agent, such as dicyclohexylcarbodiimide, and 1-hydroxybenzotriazole, at a temperature from 0 °C to 60 °C and during a reaction time from 6 to 48 h.

When Y is a chlorine or bromine atom, the reaction is carried out in a suitable solvent, such as chloroform, dichloromethane or benzene, in the presence of a proton scavenger amine, such as pyridine or

triethylamine, at a temperature between 0 ° C and that of the boiling point of the solvent and during a reaction time from 30 min to 24 h.

Compounds of general formula **II** either are known compounds or, if they have not been described, can be prepared following analogous methods to those described in the literature for these kind of substances, such as those described in EP 76075. In general, these methods involve the alkylation of a phenol of general formula $R^{1'}R^{2'}PhOH$ with a propargyl chloride of general formula $ClR^3R^4CC\equiv CH$, to give an ether of formula $R^{1'}R^{2'}PhOCR^3R^4C\equiv CH$, whose cyclization leads to a cromene, which is converted into an epoxide by treatment with a peracid or by hydrobromination followed by treatment with a base. Finally, the opening of the epoxide by ammonia or an amine of formula $R^7NH_2$ leads to the compounds of formula **II** with the right regiochemistry and trans configuration.

Alternatively, compounds of general formula **Ia** may also be prepared by reaction of a compound of formula **II** with an acid of formula $R^{8b}COOH$ (**IV**, wherein $R^{8b}$ has the previously defined meaning) (Step B of Scheme 1) in the same experimental conditions described above for the reaction of a compound of formula **III**, to give a compound of general formula **Ib** (**I**, wherein $R^5$ is OH, $R^6$ is H, X is oxygen and $R^8$ is $R^{8b}$). In a compound of formula **Ib**, the nitrogen atom of the heterocyclic ring $R^{8b}$ is protected by a protecting group such as *tert*-butoxycarbonyl. The reaction of said compound **Ib** in the suitable conditions for the deprotection of the *tert*-butoxycarbonyl group, i.e. treatment with an acid such as trifluoroacetic acid, hydrochloric acid in dioxane solution, hydrochloric acid in ethyl acetate solution or hydrobromic acid in ethyl acetate solution, leads to a compound of general formula **Ic** (**I**, wherein $R^5$ is OH, $R^6$ is H, X is oxygen and $R^8$ is $R^{8c}$) (Step C of Scheme 1). Finally, the reaction of **Ic** with a suitable electrophylic reagent of general formula **V**, **VI**, **VII**, **VIII**, **IX**, **X** or **XI** leads to a compound of formula **Ia** (Step D of Scheme 1). When the electrophylic reagent used is a compound of formula **V** to **X**, the reactions are preferably performed in an inert solvent, such as chloroform or dichloromethane, in the presence of a proton scavenger base, such as pyridine or triethylamine, at a temperature from 0 ° C to that of the boiling point of the solvent and during a reaction time from 30 min to 24 h. When a compound of formula **XI** is used, the reaction is carried out in an alcoholic solvent, such as methanol or ethanol, in the presence of a reducing agent, such as sodium cyanoborohydride, or in catalytic hydrogenation conditions in the presence of Pd/C.

Compounds of formula **I**, wherein $R^5$ is OH, $R^6$ is hydrogen, X is oxygen and $R^8$ is $R^{8d}$ (i.e. compounds of formula **Id**), can be obtained following the procedure described in Scheme 2. The reaction of a compound of formula **II** with phenyl chloroformiate in an inert solvent, such as chloroform or dichloromethane, in the presence of a proton scavenger amine, such as pyridine or triethylamine, at a temperature between 0 ° C and that of the boiling point of the solvent and during a reaction time from 30 min to 24 h, leads to a compound of formula **XII**. The subsequent reaction of said compound **XII** with a compound of general formula $R^{8d}$-H (**XIII**, wherein $R^{8d}$ has the previously defined meaning) in an alcoholic solvent, such as *n*-butanol or isopropanol, or using **XIII** itself as solvent, at a temperature between room temperature and that of the boiling point of the solvent and during a reaction time from 6 to 72 h, leads to the compounds of general formula **Id**.

The compounds of formula **I**, wherein $R^5$ is OH, $R^6$ is hydrogen, X is sulphur and $R^8$ is $R^{8d}$ (i.e. compounds of formula **Ie**), may be obtained following the method described in Scheme 3. The reaction of a compound of formula **II** with carbon sulphide in a polar solvent, such as N,N-dimethylformamide, in the presence of a base, such as potassium hydroxide or sodium hydroxide, at a temperature from 0 ° C to 60 ° C and during a reaction time from 6 to 48 h, followed by treatment of the intermediate obtained with methyl iodide in an alcoholic solvent, such as methanol or ethanol, at the temperature of the boiling point of the solvent and during a reaction time from 30 min to 3 h, leads to a compound of formula **XIV**. The reaction of said compound **XIV** with a compound of general formula **XIII** (above mentioned) in an alcoholic solvent, such as *n*-butanol or isopropanol, or else using **XIII** itself as solvent, at a temperature between room temperature and that of the boiling point of the solvent and during a reaction time from 6 to 72 h, leads to the compounds of formula **Ie**.

As shown in Scheme 4, compounds of general formula **I** wherein X is sulphur (i.e. compounds of formula **If**, **Ih** and **Ij**) may also be obtained by thiation of the corresponding oxygen derivatives (**Ia-d**, **Ig** and **Ii**) with conventional reagents such as hydrogen sulphide, phosphorous pentasulphide or Lawesson's reagent (*p*-methoxyphenylthiophosphine disulphide) in an inert apolar solvent, such as toluene, at the temperature of the boiling point of the solvent and during a reaction time from 1 to 24 h.

Compounds of general formula **I** wherein $R^5$ is acetoxy and $R^6$ is hydrogen (i.e. compounds of formula **Ig** and **Ih**) can be obtained (Scheme 4) by acetylation of the corresponding hydroxy derivatives with acetic anhydride in the presence of a base, such as pyridine, at room temperature and during a reaction time from 24 to 96 hours.

Compounds of general formula **I** wherein $R^5$ and $R^6$ together form a bond (i.e. compounds of formula **Ii**

and **Ij**) can be obtained (Scheme 4) from the corresponding hydroxy derivatives (**Ia-d** and **If**) using conventional reactions of dehydration such as treatment with sodium hydride or sodium hydroxide in an inert solvent, such as tetrahydrofuran or dioxane, at a temperature between room temperature and that of the boiling point of the solvent and during a reaction time from 6 to 48 h. Alternatively, the compounds of formula **Ii** and **Ij** can be obtained by treatment of the corresponding acetoxy derivatives (**Ig** and **Ih**) with a base, such as 1,8-diazabicyclo(5.4.0)undec-7-ene, in an inert solvent, such as toluene, at a temperature between room temperature and that of the boiling point of the solvent and during a reaction time from 6 to 48 h.

Furthermore, it is also possible to transform the groups $R^1$ and/or $R^2$ in a compound of formula I or $R^{1'}$ and/or $R^{2'}$ in one of its synthetic intermediates into other groups $R^1$ and/or $R^2$.

For instance, a cyano group may be transformed into a carboxyl group (e.g. with HCl in water, at 20-100 °C), into a carbamoyl group (e.g. with KOH in *t*-BuOH), into a methyl carboximidate group (e.g. with sodium methoxide in MeOH, at room temperature), or into a methyl carboxylate (e.g. with HCl gas in MeOH, at reflux); a bromine atom may be converted into a trifluoromethyl or a pentafluoroethyl group (e.g. with trifluoroacetate or pentafluoropropionate resp./cuprous iodide in N-methylpyrrolidone, at 160 °C), or into a trimethylsilylethynyl group (e.g. with Pd(II) acetate/ethynyltrimethylsilane/triphenylphosphine in $NEt_3$), which may be subsequently transformed into an ethynyl group (e.g. with potassium carbonate in MeOH, at room temperature); a methoxy group may be transformed into a hydroxyl group (e.g. with 48% HBr, at reflux), and this may be then converted into a bromine atom (e.g. with triphenylphosphonium bromide, at 185 °C); an acetamido group may be alkylated to an N-methylacetamido group (e.g. MeI/NaH in bencene, at 50 °C and then at reflux), which may be deacetylated to a methylamino group (e.g. with HCl, at reflux), and this transformed into a N-methylmethanesulphonamide (e.g. with methanesulphonyl chloride in $CHCl_3$/pyridine, at 0 °C-room temperature).

The compounds of formula **I** may be transformed into their corresponding acid addition salts by treatment with an acid, such as hydrochloric acid, sulphuric acid, nitric acid, oxalic acid or methanesulphonic acid.

The compounds of general formula **I** are useful as antihypertensive agents, as shown by their ability to inhibit the contraction induced by noradrenaline in portal vein isolated from rat, according to test 1, and their ability to lower the blood pressure in hypertense rats, according to test 2.

<u>Test 1: Inhibition of the contraction induced by noradrenaline in portal vein isolated from rat.</u>

Portal vein was extracted from adult male rats (between 200 and 250 g of body weight), which had been stunned by a blow in the head. Portal vein was installed into an isolated organ bath (Letica) containing a physiological saline solution (Hamilton et al., *Br. J. Pharmacol.,* **1986,** <u>88</u>, 103-111) at 37 °C and a gas bubbler (5% $CO_2$ and 95% $O_2$). Contractions were induced by noradrenaline (3µM) and were reverted after thoroughly washing with physiological saline solution. Portal vein contraction was measured with an isometric force transducer and with an initial tension of 1 g. After two contractions with noradrenaline, performed in order to measure the tissue's basal response, the tested compounds were incubated for 30 minutes and a new contraction was induced. The concentration that produces a 50% inhibition ($IC_{50}$) versus the basal response was calculated. The results are shown in table I.

TABLE I

| Compound Nº | $IC_{50}$(μM) |
|---|---|
| 1 | 3.1 |
| 3 | 4.2 |
| 7 | 5.3 |
| 13 | 31 |
| 18 | 0.8 |
| 19 | 0.8 |
| 28(A) | >10(28%) |
| 28(B) | 2.6 |
| 33(A) | >10(28%) |
| 33(B) | 0.3 |
| 34 | 2.5 |
| 35(A) | 2.4 |
| 35(B) | 0.2 |
| 45 | 3.5 |
| 58 | 0.5 |
| 60 | 0.2 |

Test 2: Lowering of the arterial pressure in conscious hypertense rats.

Spontaneously hypertense rats (between 200 and 250 g of body weight) of more than 8 weeks of age have been used. Diastolic and systolic arterial pressure of the rat was measured at the caudal artery using a special sphygnomanometer (Letica 5007 and 5007/4) attached to the animal's tail. To ensure rapid and reliable data, animals were placed in a heating plate at 37°C, with the aim to produce a vasodilatation that ensured a better fixation of the rat tail to the transducer chamber. During the experiment, rats are conscious and fixed to a clamp. The tested products were administered orally. Arterial pressure was measured every 30 minutes and 10 minutes before the administration of the tested compound. The $ED_{30}$ (dose which produces a decrease of 30 mm Hg of the systolic arterial pressure) was calculated for each compound, using 3 doses and 3 animals per dose. The results are shown in table II.

TABLE II

| Compound Nº | $ED_{30}$ mg/Kg p.o. |
|---|---|
| 1 | >3.0 |
| 3 | 0.5 |
| 7 | 0.17 |
| 13 | 0.18 |
| 18 | 0.70 |
| 19 | 0.70 |
| 28(A) | - |
| 28(B) | 1.0 |
| 33(A) | 0.3 |
| 33(B) | <0.1 |
| 33(B) | <0.1 |
| 34 | 3.0 |
| 35 | 0.05 |
| 45 | 0.65 |
| 58 | <1 |
| 60 | <0.5 |

Furthermore, we have found that compounds of general formula **I** are potent bronchodilator agents, according to test 3.

Test 3 -Direct relaxation of the trachea isolated from guinea pig.

This test was performed according to the experimental model described by Emmerson, J. and Mackay, D. (*J. Pharm. Pharmacol.*,**1979**,31, 798). Tracheas were extracted from male guinea pigs of 400 g of body weight, which had been stunned by a blow in the head. Then, tracheas were cut in zigzag and placed into an isolated organ bath (Letica) containing Krebs-Henseleit solution at 37°C and were bubbled with carbogen (95% $O_2$ and 5% $CO_2$). The relaxation of the trachea was measured using an isometric force transducer. The basal tension was 0.5 g. The tested compounds were accumulatively added to the bath and the effective concentration which produces a 50% of the maximum relaxation ($EC_{50}$) was calculated. The maximum relaxation was considered to be the relaxation induced by isoproterenol at $1x10^{-6}$ M. Results are shown in table III.

TABLE III

| Compound Nº | $EC_{50}$(μM) |
|---|---|
| 3 | 0.8 |
| 7 | 1.3 |
| 13 | 6.0 |
| 18 | 1.0 |
| 19 | 4.5 |
| 28(A) | - |
| 28(B) | 4.8 |
| 33(A) | - |
| 33(B) | 9.1 |
| 34 | 2.5 |
| 35 | 0.9 |
| 45 | 0.4 |
| 60 | 0.1 |

Solid compositions according to the present invention for oral administration include compressed tablets, dispersible powders, granules and capsules. In tablets, one or more of the active component(s) is admixed with at least one inert diluent such as lactose, starch, mannitol, microcrystalline cellulose or calcium phosphate; granulating and disintegrating agents for example corn starch, gelatine, microcrystalline cellulose or polyvinylpyrrolidone; and lubricating agents for example magnesium stearate, stearic acid or talc. The tablets may be coated by known techniques to delay disintegration and absorption in the gastrointestinal tract and, thereby, provide a sustained action over a longer period. Gastric film-coated or enteric film-coated can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as ethoxylated saturated glycerides that could exhibit controlled liberation. Soft gelatin capsules are possible wherein the active ingredient is mixed with water or an oily medium, for example coconut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, methylcellulose, hydroxypropylmethylcellulose, sodium alginate, polyvinylpyrrolidone, gum tragacanth, xantham gum, gum acacia, and one or more preservatives, such as methyl or *n*-propyl-*p*-hydroxybenzoate. Additional excipients, for example sweetening, flavoring and coloring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavouring, perfuming, preserving agents and buffers.

Other compositions for oral administration include spray compositions, which may be prepared by known methods and which comprise one or more active compound(s). The spray compositions will contain a suitable propellent.

Preparations for injection according to the present invention for parenteral administration include sterile

aqueous or non-aqueous solutions, suspensions or emulsions, in a non-toxic parentally-acceptable diluent or solvent. Examples of aqueous solvents or suspending media are distilled water for injection, the Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately before use. When all of the components are sterile, the injectables will maintain the sterility if they are manufactured in sterile environment.

A compound of the present invention may also be administered in the form of suppositories for rectal administration of the drug, or as creams, ointments jellies, solutions or suspensions for topical use and pessaries for vaginal administration.

Following are some representative preparations for tablets capsules, syrups, aerosols and injectables. They can be prepared following standard procedures and they are useful in the treatment of diseases related with the regulation of the smooth muscle contraction in the cardiovascular, respiratory and cerebrovascular systems and in the gastrointestinal, urinary and uterus tracts, and particularly as antihypertensive and bronchodilator agents.

| Tablets | |
|---|---|
| Compound of formula **I** | 100 mg |
| Dibasic calcium phosphate | 125 mg |
| Sodium starch glycolate | 10 mg |
| Talc | 12.5 mg |
| Magnesium stearate | 2.5 mg |
| | 250.0 mg |

| Hard gelatin capsules | |
|---|---|
| Compound of formula **I** | 100 mg |
| Lactose | 197 mg |
| Magnesium stearate | 3 mg |
| | 300 mg |

| Syrup | |
|---|---|
| Compound of formula **I** | 0.4 g |
| Sucrose | 45 g |
| Flavouring agent | 0.2 g |
| Sweetening agent | 0.1 g |
| Water to | 100 mL |

| Aerosol | |
|---|---|
| Compound of formula **I** | 4 g |
| Flavouring agent | 0.2 g |
| Propylene glycol to | 100 mL |
| Suitable propellent to | 1 unit |

| Injectable preparation | |
|---|---|
| Compound of formula **I** | 100 mg |
| Benzylic alcohol | 0.05 mL |
| Propylene glycol | 1 mL |
| Water to | 5 mL |

The following examples illustrate, but do not limit, the scope of the preparation of the compounds of the present invention.

**REFERENCE EXAMPLE 1**

**Potassium (6-cyano-3,4-dihydro-2,2-dimethyl-3-hydroxy-2H-1-benzopyran-4-yl)dithiocarbamate.**

To a solution of 1.55 g (7.1 mmol) of 4-amino-6-cyano-2,2-dimethyl-2H-1-benzopyran-3-ol (EP 76075) in 5 mL of anhydrous dimethylformamide, were added under nitrogen atmosphere 0.555 g (0.44 mL, 7.3 mmol) of carbon sulphide and 0.404 g (7.2 mmol) of KOH. The mixture was stirred overnight at room temperature and the solution was poured into 50 mL of diethyl ether. The precipitate obtained was filtered and dried under vacuum, yielding 1.93 g of the desired product as a yellow solid (yield: 81%).
M.p.: 205.4-213.7 ° C;
IR(KBr)$\nu$: 3600-3000, 2967, 2881, 2228, 1656, 1603, 1481, 1260, 1118, 1068 $cm^{-1}$;
$^1H$ NMR (80 MHz, $D_2O$) $\delta$ (TMS): 7.58 (m, 2H, Ar), 6.92 (d, J = 8Hz, 1H, Ar), 5.80 (d, J = 8.7Hz, 1H, CHN), 4.70 ($H_2O$), 3.93 (d, J = 8.8Hz, 1H, CHOH), 1.51 (s, 3H, $CH_3$), 1.32 (s, 3H, $CH_3$).

**REFERENCE EXAMPLE 2**

**3,4-dihydro-2,2-dimethyl-3-hydroxy-4-methylthiothiocarbonylamino-2H-1-benzopyran-6-carbonitrile**

To a solution of 1.93 g (5.8 mmol) of the product obtained in reference example 1 in 20 mL of MeOH, were added 0.39 mL (6.1 mmol) of methyl iodide and the mixture was stirred at reflux for 1 hour. The solvent was removed and the residue was chromatographed on silica gel eluting with hexane-ethyl acetate mixtures of increasing polarity, yielding 1.60 g of a white solid (yield: 89%). A sample was recrystallized from ether-hexane.
M.p.: 59.6-63.8 ° C;
IR (KBr) $\nu$: 3600-3200, 2966, 2883, 2219, 1605, 1485, 1351, 1266, 1098, 1072 $cm^{-1}$;
$^1H$ NMR (80 MHz, $CDCl_3$) $\delta$ (TMS): 7.50 (m, 3H, 2Ar + NH), 6.88 (d, J = 8.1Hz, 1H, Ar), 6.11 (broad t, J = 8.8Hz, 1H, CHN), 3.82 (d, J = 8.8Hz, 1H, CHOH), 3.27 (broad s, 1H, OH), 2.72 (s, 3H, $SCH_3$), 1.51 (s, 3H, $CH_3$), 1.33 (s, 3H, $CH_3$).
Analysis Calcd. for $C_{14}H_{16}N_2O_2S_2$ . $Et_2O$: C 56.54%; H 6.81%, N 7.33%.Found: C 56.36%; H 6.75%; N 7.29%.

**REFERENCE EXAMPLE 3**

**Potassium N-methyl-N-(6-cyano-3,4-dihydro-2,2-dimethyl-3-hydroxy-2H-1-benzopyran-4-yl)-dithiocarbamate**

Following the procedure described in reference example 1, but starting from 4-methylamino-6-cyano-2,2-dimethyl-2H-1-benzopyran-3-ol, the title compound of this example was obtained as a yellow solid (yield: 78%), which was directly used in the next step.

**REFERENCE EXAMPLE 4**

**3,4-dihydro-2,2-dimethyl-3-hydroxy-N-methyl-4-methylthiothiocarbonylamino-2H-1-benzopyran-6-carbonitrile**

Following the procedure described in reference example 2, but starting from the product obtained in reference example 3, the title compound of this example was obtained as a white solid (yield: 97%).
M.p.: 138.2-142.0 ° C;

IR (KBr) ν: 3600-3200, 2974, 2902, 2221, 1605, 1483, 1468, 1382, 1306, 1260, 1068 $cm^{-1}$;
$^1H$ NMR (80 MHz, $CDCl_3$) δ (TMS): 7.35 (m, 3H, 2Ar + CHN), 6.91 (d, J = 8Hz, 1H, Ar), 3.90 (d, J = 10Hz, 1H, CHOH), 3.09 (s, 3H, $CH_3N$), 2.76 (s, 3H, $SCH_3$), 2.50 (broad s, 1H, OH), 1.55 (s, 3H, $CH_3$), 1.34 (s, 3H, $CH_3$);
Analysis Calcd. for $C_{15}H_{18}N_2O_2S_2$: C 55.88%; H 5.63%; N 8.69%. Found: C 55.78%; H 5.58%; N 8.66%.

## REFERENCE EXAMPLE 5

### 3,4-dihydro-2,2-dimethyl-3-hydroxy-4-phenyloxycarbonylamino-2H-1-benzopyran-6-carbonitrile

To a solution of 1.5 g (6.9 mmol) of 4-amino-2,2-dimethyl-3-hydroxy-2H-1-benzopyran-6-carbonitrile in 25 mL of anhydrous $CH_2Cl_2$, were added under argon atmosphere 0.95 mL (6.9 mmol) of triethylamine and the mixture was cooled to 0 ° C. 0.87 mL (6.9 mmol) of phenyl chloroformiate were added and the resulting mixture was stirred for 1 h at room temperature. The mixture was poured into 75 mL of $H_2O$ and the layers were separated. The organic phase was washed with $H_2O$ and dried over $Na_2SO_4$. The solvent was removed, yielding 1.32 g of a colorless oil, which was directly used in the next step (yield: 57%).
IR (film)ν: 3436, 3250, 2976, 2230, 1775, 1691, 1485, 1262, 1210, 1182, 1128, 1969 $cm^{-1}$;
$^1H$ NMR (80 MHz, $CDCl_3$) δ (TMS): 7.35 (m,7H), 6.86 (d, J = 8.8Hz, 1H, Ar), 4.71 (m, 1H, CHN), 4.14 (broad s, 2H, NH + OH), 3.67 (d, J = 10Hz, 1H, CHOH), 1.52 (s,3H, $CH_3$), 1.29(s, 3H, $CH_3$).

## REFERENCE EXAMPLE 6

### 3,4-dihydro-2,2-dimethyl-3-hydroxy-N-methyl-4-phenyloxycarbonylamino-2H-1-benzopyran-6-carbonitrile

Following the procedure described in reference example 5, but starting from 4-methylamino-6-cyano-2,2-dimethyl-2H-1-benzopyran-3-ol, the title compound of this example was obtained as a white solid (yield: 85%).
IR (KBr) ν: 3431, 3037, 2987, 2224, 1686, 1694, 1483, 1328, 1265, 1219, 1196, 1119 $cm^{-1}$;
$^1H$ NMR (80 MHz, $CDCl_3$) δ (TMS): 7.33 (m, 7H), 6.87 (d, J = 9Hz, 1H, Ar), 5.35 (m, 1H, CHN), 3.9-3.2 (m, 2H), 2.81, 2.63 (s, 3H, $CH_3N$), 1.50 (s, 3H, $CH_3$), 1,15 (s, 3H, $CH_3$).

## REFERENCE EXAMPLE 7

### a) 3-pyridylmethyl phenylcarbonate

To a solution of 2 g (18 mmol) of 3-hydroxymethylpyridine in 15 mL of chloroform, cooled at 0 ° C, were added 2.27 mL (18 mmol) of phenyl chloroformiate and the mixture was stirred under argon atmosphere for 1 h at room temperature. 20 mL of chloroform were added and the mixture was washed with 1N NaOH solution. The organic layer was dried over $MgSO_4$ and the solvent was removed, yielding 4.02 g of the desired product as a colorless oil (yield: 98%).
IR (KBr) ν: 2955, 1756, 1588, 1486, 1376, 1234, 1208, 1022 $cm^{-1}$;
$^1H$ NMR (80 MHz, $CDCl_3$) δ (TMS): 8.65 (m, 2H), 7.78 (d, J = 8Hz, 1H), 7.30 (m, 6H), 5.28 (s, 2H).

### b) N-(3-pyridylmethyloxycarbonyl)-L-Proline

To a solution of 0.5 g (2.18 mmol) of the product obtained in reference example 7a in 10 mL of pyridine, were added 0.25 g (2.18 mmol) of L-proline and the mixture was stirred overnight at reflux and under argon atmosphere. Pyridine was removed, and the residue was redissolved in chloroform. The resulting solution was acidified with a solution of HCl in ether. The solvent was removed and the residue obtained, impurified with phenol, was used in the next reaction without further purification.
IR (KBr) ν: 3600-2400, 1695, 1588, 1469, 1410, 1238 $cm^{-1}$;
$^1H$ NMR (80 MHz, $CDCl_3$) δ (TMS): 10.14 (s, 1H), 8.63 (broad s., phenol), 7.75 (m, 2H), 7.00 (m, 2H + phenol), 5.28 (s, 2H), 4.39 (m, 1H), 3.58 (m, 2H), 2.10 (m, 4H).

## REFERENCE EXAMPLE 8

### a) 2-pyridylmethyl phenylcarbonate

Following the procedure described in reference example 7a, but starting from 2-hydroxymethylpyridine, the title compound of this example was obtained as a colorless oil (yield: 80%).
IR (KBr) ν: 2951, 1758, 1588, 1469, 1374, 1265, 1238, 1208 $cm^{-1}$;
$^1H$ NMR (80 MHz, $CDCl_3$) δ (TMS): 8.61 (m, 2H), 7.30 (m, 7H), 5.44 (s, 2H).

**b) N-(2-pyridylmethyloxycarbonyl)-L-Proline**

Following the procedure described in reference example 7b, but starting from the product obtained in reference example 8a, the title compound of this example was obtained impurified with phenol, which was used in the next reaction without further purification (yield: 80%).
IR (KBr) ν: 3600-2400, 1697, 1588, 1469, 1410, 1245 $cm^{-1}$;
$^1H$ NMR (80 MHz, $CDCl_3$) δ (TMS): 10.14 (s, 1H), 8.63 (broad s., phenol), 7.75 (m, 1H), 7.00 (m, 3H + phenol), 5.28 (s, 2H), 4.39 (m, 1H), 3.58 (m, 2H), 2.10 (m, 4H).

**REFERENCE EXAMPLE 9**

**a) 4-pyridylmethyl phenylcarbonate**

Following the procedure described in reference example 7a, but starting from 4-hydroxymethylpyridine, the title compound of this example was obtained as a colorless oil (yield: 80%).
IR (KBr) ν: 2963, 1758, 1488, 1242, 1206, 1155 $cm^{-1}$;
$^1H$ NMR (80 MHz, $CDCl_3$) δ (TMS): 8.63 (m, 1H), 7.32 (m, 8H), 5.27 (s, 2H).

**b) N-(4-pyridylmethyloxycarbonyl)-L-Proline**

Following the procedure described in reference example 7b, but starting from the product obtained in reference example 9a, the title compound of this example was obtained impurified with phenol, which was used in the next reaction without further purification.

**REFERENCE EXAMPLE 10**

**a) 3-methoxyphenylmethyl phenylcarbonate**

Following the procedure described in reference example 7a, but starting from 3-hydroxymethyl-1-methoxybenzene, the title compound of this example was obtained as a colorless oil (yield: 36%).
IR (KBr) ν: 3054, 2955, 1754, 1487, 1452, 1236, 1207 $cm^{-1}$;
$^1H$ NMR (80 MHz, $CDCl_3$) δ (TMS): 7.10 (m, 9H), 5.24 (m, 2H), 3.82 (s, 3H).

**b) N-(3-methoxyphenylmethyloxycarbonyl)-L-Proline**

Following the procedure described in reference example 7b, but starting from the product obtained in reference example 10a, the title compound of this example was obtained impurified with phenol, which was used in the next reaction without further purification (yield: 100%).
$^1H$ NMR (80 MHz, $CDCl_3$) δ (TMS): 9.71 (s, 1H), 8.63 (broad s., phenol), 7.0 (m, 4H + phenol), 5.11 (s, 2H), 4.39 (m, 1H), 3.75 (s, 3H), 3.55 (m, 2H), 2.03 (m, 4H).

**REFERENCE EXAMPLE 11**

**a) 4-methoxyphenylmethyl phenylcarbonate**

Following the procedure described in reference example 7a, but starting from 4-hydroxymethyl-1-methoxybenzene, the title compound of this example was obtained as a colorless oil (yield: 24%).
IR (KBr) ν: 2929, 2832, 1753, 1510, 1243, 1207, 1173 $cm^{-1}$;
$^1H$ NMR (80 MHz, $CDCl_3$) δ (TMS): 7.13 (m, 9H), 5.19 (m, 2H), 3.80 (s, 3H).

**b) N-(4-methoxyphenylmethyloxycarbony])-L-Proline**

Following the procedure described in reference example 7b, but starting from the product obtained in

reference example 11a, the title compound of this example was obtained impurified with phenol, which was used in the next reaction without further purification (yield: 86%).
IR (KBr) $\nu$: 3600-2400, 1671, 1589, 1508, 1496, 1448, 1428, 1202, 1172 $cm^{-1}$;
$^1H$ NMR (80 MHz, $CDCl_3$) $\delta$ (TMS): 7.50 (s, 1H), 7.0 (m, 4H + phenol), 5.07 (s, 2H), 4.51 (m, 1H), 3.76 (s, 3H), 3.46 (m, 2H), 2.10 (m, 4H).

**REFERENCE EXAMPLE 12**

**a) 3-chlorophenylmethyl phenylcarbonate**

Following the procedure described in reference example 7a, but starting from 3-hydroxymethyl-1-chlorobenzene, the title compound of this example was obtained as a colorless oil (yield: 78%).
IR (KBr) $\nu$: 2951, 1758, 1587, 1488, 1237, 1209, 1179, 1157 $cm^{-1}$;
$^1H$ NMR (80 MHz, $CDCl_3$) $\delta$ (TMS): 7.23 (m, 9H), 5.19 (m, 2H).

**b) N-(3-chlorophenylmethyloxycarbonyl)-L-Proline**

Following the procedure described in reference example 7b, but starting from the product obtained in reference example 12a, the title compound of this example was obtained impurified with phenol, which was used in the next reaction without further purification (yield: 80%).

**REFERENCE EXAMPLE 13**

**a) 2-phenylethyl phenylcarbonate**

Following the procedure described in reference example 7a, but starting from 1-hydroxy-2-phenylethane, the title compound of this example was obtained as a colorless oil (yield: 100%).
IR (KBr) $\nu$: 3052, 2933, 2891, 1773, 1745, 1233, 1204, 1178, 1157 $cm^{-1}$;
$^1H$ NMR (80 MHz, $CDCl_3$) $\delta$ (TMS): 7.27 (m, 10H), 4.45 (t, J = 8Hz, 2H), 3.05 (t, J = 8Hz, 2H).

**b) N-[(2-phenylethyl)oxycarbonyl]-L-Proline**

Following the procedure described in reference example 7b, but starting from the product obtained in reference example 13a, the title compound of this example was obtained impurified with phenol, which was used in the next reaction without further purification (yield: 32%).
IR (KBr) $\nu$: 3600-2400, 1702, 1590, 1469, 1409, 1227 $cm^{-1}$;
$^1H$ NMR (80 MHz, $CDCl_3$) $\delta$ (TMS): 8.51 (s, 1H), 7.22 (m, 5H + phenol), 4.39 (m, 1H), 4.30 (t, J = 8Hz, 2H), 3.58 (m, 2H), 2.92 (t, J = 8Hz, 2H), 2.10 (m, 4H).

**EXAMPLE 1**

***Trans* 3,4-dihydro-2,2-dimethyl-3-hydroxy-4-(1-pyrrolidinylthiocarbonylamino)-2H-1-benzopyran-6-carbonitrile**

A solution of 0.5 g (1.6 mmol) of the compound obtained in reference example 2, 0.218 g (3.1 mmol) of pyrrolidine and 10 mL of n-BuOH was heated to reflux overnight. The solvent was removed and the residue was chromatographed on silica gel, eluting with hexane-ethyl acetate mixtures of increasing polarity, to yield 0.42 g of a white solid (yield: 78%).
M.p.: 249.1-255.0 °C;
IR (KBr) $\nu$: 3422, 3351, 2969, 2228, 1605, 1536, 1483, 1421, 1350, 1267, 1073 $cm^{-1}$;
$^1H$ NMR (80 MHz, $CDCl_3$) $\delta$ (TMS): 7.65 (m, 1H, Ar), 7.44 (dd, J = 9Hz, J = 2.5Hz, 1H, Ar), 6.86 (d, J = 9Hz, 1H, Ar), 6.05 (d, J = 10Hz, 1H, CHN), 4.24 (broad s, 2H), 3.68 (m, 5H), 2.05 (m, 4H, pyrr), 1.52 (s, 3H, $CH_3$), 1.34 (s, 3H, $CH_3$).
Analysis Calcd. for $C_{17}H_{21}N_3O_2S$. 0.25 $H_2O$: C 60.80%; H 6.26%; N 12.51%. Found: C 60.96%; H 6.50%; N 12.11%.

**EXAMPLE 2**

**Trans 3,4-dihydro-2,2-dimethyl-3-hydroxy-4-(N-methyl-1-pyrrolidinylthiocarbonylamino)-2H-1-benzopyran-6-carbonitrile**

Following the procedure described in example 1, but starting from the product obtained in reference example 4, the title compound of this example was obtained as a white solid (yield: 25%).
M.p.: 197.3 ° C;
IR (KBr) ν: 3600-3200, 2971, 2925, 2878, 2221, 1603, 1483, 1378, 1265, 1165 $cm^{-1}$;
$^1H$ NMR (80 MHz, $CDCl_3$) δ (TMS): 7.42 (m, 2H, Ar), 6.88 (d, J = 8.8Hz, 1H, Ar), 6.36 (d, J = 10Hz, 1H, CHN), 3.98 (d, J = 10Hz, 1H, CHOH), 3.70 (m, 5H), 2.78 (s, 3H, $CH_3N$), 2.04 (m, 4H, pyrr), 1.55 (s, 3H, $CH_3$), 1.35 (s, 3H, $CH_3$).
Analysis Calcd. for $C_{18}H_{23}N_3O_2S$: C 62.58%; H 6.71%; N 12.16%. Found: C 62.38%; H 6.76%; N 11.89%.

**EXAMPLE 3**

**Trans 3,4-dihydro-2,2-dimethyl-3-hydroxy-4-(1-piperidinylthiocarbonylamino)-2H-1-benzopyran-6-carbonitrile**

Following the procedure described in example 1, but using piperidine instead of pyrrolidine, the title compound of this example was obtained as a white solid (yield: 54%).
M.p.: 123.1-124.7 ° C;
IR (KBr) ν: 3600-3000, 2936, 2221, 1605, 1529, 1482, 1416, 1351, 1289, 1258, 1240, 1125 $cm^{-1}$;
$^1H$ NMR (80 MHz, $CDCl_3$) δ (TMS): 7.45 (m, 2H, Ar), 6.87 (d, J = 8.8Hz, 1H, Ar), 6.19 (broad t, J = 9.2Hz, 1H, CHN), 5.66 (broad d, J = 9.2Hz, 1H, NH), 3.78 (m, 6H), 1.72 (m, 6H, pip), 1.50 (s, 3H, $CH_3$), 1.35 (s, 3H, $CH_3$).
Analysis Calcd. for $C_{18}H_{23}N_3O_2S$. 0.5 $Et_2O$: C 62.83%; H 7.33%; N 10.99%. Found: C 63.23%; H 7.21%; N 10.73%.

**EXAMPLE 4**

**Trans 3,4-dihydro-2,2-dimethyl-3-hydroxy-4-(1-morpholinylthiocarbonylamino)-2H-1-benzopyran-6-carbonitrile**

Following the procedure described in example 1, but using morpholine instead of pyrrolidine, the title compound of this example was obtained as a white solid (yield: 46%).
M.p.: 208.1-213.5 ° C;
IR (KBr) ν: 3500-3100, 2966, 2863. 2213, 1604, 1529, 1480, 1337, 1261, 1122, 1070 $cm^{-1}$;
$^1H$ NMR (80 MHz, $CDCl_3$) δ (TMS): 7.59 (m, 1H, Ar), 7.45 (broad d, J = 9Hz, 1H, Ar), 6.85 (d, J = 9Hz, 1H, Ar), 6.10 (d, J = 10Hz, 1H, CHN), 3.87 (m, 11H), 1.51 (s, 3H, $CH_3$), 1.29 (s, 3H, $CH_3$).
Analysis Calcd. for $C_{17}H_{21}N_3O_3S$: C 58.77%; H 6.09%; N 12.09%. Found: C 59.04%; H 6.10%; N 11.79%.

**EXAMPLE 5**

**Trans 3,4-dihydro-2,2-dimethyl-3-hydroxy-4-(4-hydroxy-1-piperidinylthiocarbonylamino)-2H-1-benzopyran-6-carbonitrile**

Following the procedure described in example 1, but using 4-hydroxypiperidine instead of pyrrolidine, the title compound of this example was obtained as a white solid (yield: 48%).
M.p.: 119.5-124.6 ° C;
IR (KBr) ν: 3600-3100, 2970, 2922, 2222, 1605, 1534, 1483, 1338, 1263, 1065 $cm^{-1}$;
$^1H$ NMR (80 MHz, $CDCl_3$) δ (TMS): 7.50 (m, 2H, Ar), 6.87 (d, J = 9Hz, 1H, Ar), 6.18 (t, J = 8Hz, 1H, CHN), 5.77 (d, J = 8Hz, 1H, NH), 4.6-3.4 (m, 8H), 1.70 (m, 4H), 1.50 (s, 3H, $CH_3$), 1.35 (s, 3H, $CH_3$).
Analysis Calcd. for $C_{18}H_{23}N_3O_3S$. 0.75 $H_2O$: C 57.68%; H 6.54%; N 11.21%. Found: C 57.85%; H 6.57%; N 10.88%.

**EXAMPLE 6**

**Trans 3,4-dihydro-2,2-dimethyl-3-hydroxy-4-(1-piperazinylthiocarbonylamino)-2H-1-benzopyran-6-carbonitrile**

Following the procedure described in example 1, but using piperazine instead of pyrrolidine, the title compound of this example was obtained as a white solid (yield: 54 %).
M.p.: 139.6-146.8 ° C;
IR (KBr) ν: 3600-2800, 2215, 1605, 1541, 1485, 1351, 1316, 1300, 1266, 1123 cm$^{-1}$;
$^1$H NMR (80 MHz, $CDCl_3$) δ (TMS): 7.5 (m, 2H, Ar), 6.86 (d, J = 8Hz, 1H Ar), 6.10 (d, J = 10Hz, 1H, CHN), 3.91 (m, 4H, pip), 3.74 (m, 4H), 2.93 (m, 4H, pip), 1.52 (s, 3H, $CH_3$), 1.34 (s, 3H, $CH_3$).
Analysis Calcd. for $C_{17}H_{22}N_4O_2S$. 1 $H_2O$: C 56.04%; H 6.59%; N 15.39%. Found: C 55.74%; H 6.74%; N 15.32%.

## EXAMPLE 7

***Trans* 3,4-dihydro-2,2-dimethyl-3-hydroxy-4-(4-methyl-1-piperazinylthiocarbonylamino)-2H-1-benzopyran-6-carbonitrile**

Following the procedure described in example 1, but using 1-methylpiperazine instead of pyrrolidine, the title compound of this example was obtained as a white solid (yield: 51%).
M.p.: 85.3-94.0 ° C;
IR (KBr) ν: 3600-3200, 2971, 2931, 2221, 1530, 1483, 1337, 1263, 1141 cm$^{-1}$;
$^1$H NMR (80 MHz, $CDCl_3$) δ (TMS): 7.47 (m, 2H, Ar), 6.88 (d, J = 8Hz, 1H, Ar), 6.19 (t, J = 10Hz, 1H, CHN), 5.66 (d, J = 10Hz, 1H, NH), 3.93 (m, 4H, pip), 3.73 (d, J = 10Hz, 1H, CHOH), 2.54 (m, 4H, pip), 2.36 (s, 3H, $CH_3N$), 1.70 (m, 1H, OH), 1.51 (s, 3H, $CH_3$), 1.35 (s, 3H, $CH_3$).
Analysis Calcd. for $C_{18}H_{24}N_4O_2S$. 1.5 $H_2O$: C 55.81%; H 6.98%; N 14.47%. Found: C 56.02%; H 6.44%; N 14.44%.

## EXAMPLE 8

***Trans* 3,4-dihydro-2,2-dimethyl-3-hydroxy-4-(N-methyl-4-methyl-1-piperazinylthiocarbonylamino)-2H-1-benzopyran-6-carbonitrile**

Following the procedure described in example 1, but starting from the product obtained in reference example 4 and using 1-methylpiperazine instead of pyrrolidine, the title compound of this example was obtained as a white solid (yield: 37%).
M.p.: 243.1-244.1 ° C;
IR (KBr) ν: 3497, 3077, 2931, 2220, 1486, 1361, 1301, 1261, 1055 cm$^{-1}$;
$^1$H NMR (80 MHz, $CDCl_3$) δ (TMS): 7.42 (m, 2H, Ar), 6.88 (d, J = 8Hz, 1H, Ar), 6.16 (d, J = 10Hz, 1H, CHN), 3.73 (m, 5H), 2.93 (s, 1H, OH), 2.83 (s, 3H, $CH_3N$), 2.62 (m, 4H, pip), 2.39 (s, 3H, $CH_3N$), 1.54 (s, 3H, $CH_3$), 1.31 (s, 3H, $CH_3$).
Analysis Calcd. for $C_{19}H_{26}N_4O_2S$. 0.25 $H_2O$: C 60.23%; H 7.00%; N 14.80%. Found: C 60.13%; H 7.20%; N 14.71%.

## EXAMPLE 9

***Trans* 3,4-dihydro-2,2-dimethyl-3-hydroxy-4-(1-pyrrolidinylcarbonylamino)-2H-1-benzopyran-6-carbonitrile**

A mixture of 0.65 g (1.9 mmol) of the product obtained in reference example 5 and 3 mL of pyrrolidine was stirred under reflux overnight. The excess of pyrrolidine was removed and the residue was chromatographed on silica gel, eluting with 40% hexane-ethyl acetate mixtures. 0.380 g of a solid were obtained, which were recrystallized from ethyl acetate-hexane, to give 0.253 g of a white solid (yield: 42%).
M.p.: 199.6-200.4 ° C;
IR (KBr) ν: 3600-3200, 2975, 2869, 2216, 1620, 1525, 1479, 1266, 1102 cm$^{-1}$;
$^1$H NMR (80 MHz, $CDCl_3$) δ (TMS): 7.66 (broad s, 1H, Ar), 7.46 (d, J = 8.8Hz, 1H, Ar), 6.87 (d, J = 8.8Hz, 1H, Ar), 4.96 (broad t, J = 9Hz, 1H, CHN), 4.50 (m, 2H, NH + OH), 3.60 (d, J = 9Hz, 1H, CHOH), 3.40 (m, 4H, pyrr), 1.98 (m, 4H, pyrr), 1.50 (s, 3H, $CH_3$), 1.26 (s, 3H, $CH_3$).
Analysis Calcd. for $C_{17}H_{21}N_3O_3$ . 0.25 $H_2O$: C 63.85%; H 6.73%; N 13.14%. Found: C 63.62%; H 6.91%; N 12.64%.

## EXAMPLE 10

**_Trans_ 3,4-dihydro-2,2-dimethyl-3-hydroxy-4-(N-methyl-1-pyrrolidinylcarbonylamino)-2H-1-benzopyran-6-carbonitrile**

Following the procedure described in example 9, but starting from the product obtained in reference example 6, the title compound of this example was obtained as a white solid (yield: 43%).
M.p.: 206.0 °C;
IR (KBr) ν: 3420, 2964, 2218, 1597, 1481, 1401, 1262, 1120 $cm^{-1}$;
$^1H$ NMR (80 MHz, $CDCl_3$) δ (TMS): 7.45 (m, 2H, Ar), 6.88 (d, J = 9Hz, 1H, Ar), 5.10 (d, J = 10Hz, 1H, CHN), 4.30 (broad s, 1H, OH), 3.84 (d, J = 10Hz, 1H, CHOH), 3.50 (m, 4H, pyrr), 2.60 (s, 3H, $CH_3N$), 1.95 (m, 4H, pyrr), 1.54 (s, 3H, $CH_3$), 1.27 (s, 3H, $CH_3$).
Analysis Calcd. for $C_{18}H_{23}N_3O_3$: C 65.63%; H 7.04%; N 12.76%. Found: C 65.66%; H 7.32%; N 12.64%.

**EXAMPLE 11**

**_Trans_ 3,4-dihydro-2,2-dimethyl-3-hydroxy-4-(1-piperidinylcarbonylamino)-2H-1-benzopyran-6-carbonitrile**

Following the procedure described in example 9, but using piperidine instead of pyrrolidine, the title compound of this example was obtained as a white solid (yield: 39%).
M.p.: 178.5-179.0 °C;
IR (KBr) ν: 3400-3200, 2933, 2855, 2218, 1607, 1581, 1524, 1486, 1268, 1255, 1131 $cm^{-1}$;
$^1H$ NMR (80 MHz, $CDCl_3$) δ (TMS): 7.62 (broad s, 1H, Ar), 7.45 (dd, J = 9Hz, J = 2.5Hz, 1H, Ar), 6.87 (d, J = 9Hz, 1H, Ar), 4.95 (broad t, J = 10Hz, 1H, CHN), 4.64 (broad d, J = 10Hz, 1H, NH), 3.58 (d, J = 10Hz, 1H, CHOH), 3.40 (m, 5H, OH, pip), 1.63 (m, 6H, pip), 1.49 (s, 3H, $CH_3$), 1.26 (s, 3H, $CH_3$).
Analysis Calcd. for $C_{18}H_{23}N_3O_3$ . 0.25 $H_2O$: C 64.77%; H 7.05%; N 12.59%. Found: C 64.78%; H 6.93%; N 12.54%.

**EXAMPLE 12**

**2,2-dimethyl-4-(4-methyl-1-piperazinylcarbonylamino)-2H-1-benzopyran-6-carbonitrile**

To a solution of 1.05 g (2.9 mmol) of the product obtained in example 7 in 45 mL of dioxane, were added, under argon atmosphere, 1.05 g of NaOH on silica, and the mixture was stirred at reflux for 3h and then at room temperature overnight. The solution was filtered and the solvent was removed. The residue was redissolved in $CH_2Cl_2$ and washed with $H_2O$. The organic phase was dried over $Na_2SO_4$ and the solvent was removed. The residue was chromatographed on silica gel, eluting with $CHCl_3$/MeOH/$NH_3$ - (60:2:0.2) mixtures. 0.150 g of an oil were obtained, which was recrystallized from ethanol-ether, to give 0.130 g of the desired product as a white solid (yield: 13%).
M.p.: 154.3 °C;
IR (KBr) ν: 2975, 2933, 2221, 1631, 1473, 1288, 1255, 1122 $cm^{-1}$;
$^1H$ NMR (80 MHz, $CDCl_3$) δ (TMS): 7.75 (m, 1H, Ar), 7.40 (m, 2H, Ar + NH), 6.85 (d, J = 9Hz, 1H, Ar), 5.75 (s, 1H, CH), 3.60 (m, 4H, pip), 2.54 (m, 4H, pip), 2.39 (s, 3H, $CH_3N$), 1.59 (s, 3H, $CH_3$), 1.49 (s, 3H, $CH_3$).
Analysis Calcd. for $C_{18}H_{22}N_4O_2$ . 1.25 $H_2O$: C 61.98%; H 7.03%; N 16.06%. Found: C 62.21%; H 6.67%; N 15.66%.

**EXAMPLE 13**

**_Trans_ 3,4-dihydro-2,2-dimethyl-3-hydroxy-4-(1-_tert_-butoxycarbonyl-2-piperidinyl carbonylamino)-2H-1-benzopyran-6-carbonitrile**

To a solution of 0.6 g (2.75 mmol) of 4-amino-3,4-dihydro-2,2-dimethyl-3-hydroxy-2H-1-benzopyran-6-carbonitrile in 8 mL of dimethylformamide, were added under argon atmosphere 0.630 g (2.75 mmol) of N-*tert*-butoxycarbonyl homoproline, 0.367 g (2.7 mmol) of 1-hydroxybenzotriazole and 0.558 g (2.7 mmol) of dicyclohexylcarbodiimide. The mixture was stirred overnight at room temperature and then poured into ethyl acetate. The precipitate obtained was filtered and the filtrate was washed with a saturated solution of $NaHCO_3$ and $H_2O$. The organic phase was dried over $Na_2SO_4$, the solvent was removed and the residue was chromatographed on silica gel eluting with hexane-ethyl acetate mixtures of increasing polarity. 0.96 g of the desired product were obtained as a white solid (yield: 81%).

M.p.: 61.6-75.0 ° C;
IR (KBr) ν: 3600-3100, 2973, 2931, 2850, 2223, 1649, 1485, 1364, 1265, 1160 $cm^{-1}$;
$^1$H NMR (80 MHz, $CDCl_3$) δ (TMS): 7.40 (m, 2H, Ar), 6.87 (d, J = 8.8Hz, 1H, Ar), 6.50 (m, 1H, NH), 5.06 (t, J = 9Hz, 1H, CHN), 4.74 (m, 1H, OH), 4.0 (m, 1H, N-CH-CO), 3.61 (d, J = 9Hz, 1H, CHOH), 2.93 (m, 1H, $CH_2N$), 2.25 (m, 1H, $CH_2N$), 1.67 (m, 6H, pip), 1.50 (s, 12H), 1.27 (s, 3H).
Analysis Calcd. for $C_{23}H_{31}N_3O_5S$ . 1 $H_2O$: C 61.74%; H 7.38%; N 9.39%. Found: C 62.13%; H 7.33%; N 9.10%.

**EXAMPLE 14**

**a) *Trans* 3,4-dihydro-2,2-dimethyl-3-hydroxy-4-(2-piperidinylcarbonylamino)-2H-1-benzopyran-6-carbonitrile, monohydrochloride.**

To a solution of 0.8 g (1.86 mmol) of the product obtained in example 13 in 2 mL of dioxane, were added 15 mL of a solution ca. 7M of HCl in dioxane, and the mixture was stirred for 30 min at room temperature. The resulting solid was filtered, washed with ether and dried under vacuum overnight, to give 0.582 g of the desired product as a white solid (yield: 85%).
M.p.: >300 ° C;
IR (KBr) ν: 3600-2400, 2220, 1659, 1483, 1281, 1260, 1125 $cm^{-1}$;
$^1$H NMR (80 MHz, $D_2O$) δ (TMS): 7.40 (m, 2H, Ar), 6.83 (d, J = 9Hz, 1H, Ar), 4.9-4.4 (m, 3H + s-$D_2O$), 3.9-2.8 (m, 5H), 1.71 (m, 6H, pip), 1.50 (s, 3H, $CH_3$), 1.14 (s, 3H, $CH_3$).
Analysis Calcd. for $C_{18}H_{23}N_3O_3$ . HCl . 1 $H_2O$: C 56.32%; H 6.78%; N 10.95%. Found: C 56.35%; H 6.49%; N 10.84%.

**b) *Trans* 3,4-dihydro-2,2-dimethyl-3-hydroxy-4-(2-piperidinylcarbonylamino)-2H-1-benzopyran-6-carbonitrile**

A solution of 8.70 g (24 mmol) of the product obtained in example 14a in 20 mL of $H_2O$, was basified with 2N NaOH and extracted with $CHCl_3$. The organic phase was dried over $Na_2SO_4$ and the solvent was removed, yielding 7.32 g of the title product of this example as a white solid (yield: 93%).
M.p.: 102.5-128.0 ° C;
IR (KBr) ν: 3600-3000, 2927, 2221, 1642, 1484, 1265, 1125, 1072 $cm^{-1}$;
$^1$H NMR (80 MHz, DMSO) δ (TMS): 7.41 (m, 3H, Ar + NH), 6.86 (d, J = 9Hz, 1H, Ar), 5.01 (m, 1H, CHN), 3.8-2.5 (complex signal, 6H), 2.2-1.0 (complex signal, 6H). 1.49 (s, 3H, $CH_3$), 1.26 (s, 3H, $CH_3$);
$^{13}$C-NMR (80 MHz, DMSO) δ (TMS): 175.24 (s), 174.78 (s'), 156 (s), 131.86 (d), 131.85 (d), 123.69 (s), 123.59 (s'), 118.51 (s), 117.44 (d), 102.87 (s), 79.64 (s), 79.58 (s'), 72.15 (d), 71.99 (d'), 60.50 (d), 59.33 (d'), 48.63 (d), 48.42 (d'), 44.93 (t), 29.66 (t), 29.12 (t'), 26.02 (q), 25.29 (t'), 25.03 (t), 23.55 (t), 23.38 (t'), 18.26 (q).

**EXAMPLE 15**

***Trans* 3,4-dihydro-2,2-dimethyl-3-hydroxy-4-(1-isopropyloxycarbonyl-2-piperidinylcarbonylamino)-2H-1-benzopyran-6-carbonitrile**

To a solution of 0.5 g (1.5 mmol) of the product obtained in example 14b in 10 mL of anhydrous $CHCl_3$, were added under argon atmosphere 0.21 mL (1.5 mmol) of triethylamine and 1.5 mL (1.5 mmol) of isopropyl chloroformiate, and the resulting solution was stirred at room temperature overnight. The solvent was removed and the residue was chromatographed on silica gel eluting with hexane-ethyl acetate mixtures of increasing polarity, to give 0.43 g of a compound which was recrystallized from ethanol-ether, yielding 0.246 g of the desired product as a white solid (yield: 40%).
M.p.: 208.2-209.9 ° C;
IR (KBr) ν: 3477, 3362, 3255, 3072, 2977, 2935, 2221, 1684, 1644, 1485, 1275, 1125 $cm^{-1}$;
$^1$H NMR (80 MHz, $CDCl_3$) δ (TMS): 7.39 (m, 2H, Ar), 8.87 (d, J = 8Hz, 1H, Ar), 6.54 (m, 1H, NH), 5.05 (m, 1H, CHN), 4.84 (m, 1H, OH), 4.10 (m, 1H, N-CH-CO), 3.63 (double d, J = 9Hz, 1H, CHOH), 3.1-2.0 (complex signal, 3H), 1.67 (m, 6H), 1.49 (s, 3H, $CH_3$), 1.30 (s, 3H, $CH_3$), 1.26 (d, J = 5Hz, 6H, $CH(CH_3)_2$).
Analysis Calcd. for $C_{22}H_{29}N_3O_5$: C 63.60%; H 7.04%; N 10.11%. Found: C 63.78%; H 7.13%; N 9.92%.

**EXAMPLE 16**

**_Trans_3,4-dihydro-2,2-dimethyl-3-hydroxy-4-(1-isobutyloxycarbonyl-2 piperidinylcarbonylamino)-2H-1-benzopyran-6-carbonitrile**

Following the procedure described in example 15, but using isobutyl chloroformiate instead of isopropyl chloroformiate, the title compound of this example was obtained as a white solid (yield: 70%).
M.p.: 165.6-171.5 °C;
IR (KBr) $\nu$: 3600-3200, 2931, 2222, 1658, 1605, 1484, 1416, 1263, 1124 $cm^{-1}$;
$^1$H NMR (80 MHz, $CDCl_3$) $\delta$ (TMS): 7.42 (m, 2H, Ar), 6.88 (d, J = 9Hz, 1H, Ar), 6.20 (m, 1H, NH), 5.02 (m, 1H, CHN), 4.83 (m, 1H, OH), 4.1 (m, 1H, N-CH-CO), 3.92 (d, J = 6.5Hz, 2H, $CH_2O$), 3.64 (double d, J = 9Hz, 1H, CHOH), 3.0 (m, 2H, $CH_2N$), 2.5-1.5 (m, 7H), 1.50 (s, 3H, $CH_3$), 1.28 (s, 3H, $CH_3$), 0.94 (d, J = 6.5Hz, 6H, $CH(CH_3)_2$).
Analysis Calcd. for $C_{23}H_{31}N_3O_5$ . 0.25 $H_2O$: C 63.67%; H 7.27%; N 9.69%. Found: C 63.81%; H 7.51%; N 9.43%.

**EXAMPLE 17**

**_Trans_ 3,4-dihydro-2,2-dimethyl-3-hydroxy-4-(1-hexyloxycarbonyl-2-piperidinylcarbonylamino)-2H-1-benzopyran-6-carbonitrile**

Following the procedure described in example 15, but using hexyl chloroformiate instead of isopropyl chloroformiate, the title compound of this example was obtained as a white solid (yield: 59%).
M.p.: 129.0-140.5 °C;
IR (KBr) $\nu$: 3492, 3242, 2927, 2220, 1692, 1642, 1483, 1415, 1267, 1255, 1069 $cm^{-1}$;
$^1$H NMR (80 MHz, $CDCl_3$) $\delta$ (TMS): 7.49 (m, 2H, Ar), 6.87 (d, J = 9Hz, 1H, Ar), 6.54 (m, 1H, NH), 5.04 (m, 1H, CHN), 4.08 (m, 1H, OH), 4.10 (m, 1H, N-CH-CO), 4.10 (t, J = 7Hz, 2H, $CH_2O$), 3.63 (double d, J = 9Hz, 1H, CHOH), 3.11 (m, 1H, $CH_2N$), 2.25 (m, 1H, $CH_2N$), 1.67 (m, 6H), 1.49 (s, 3H), 1.29 (m, 11H), 0.97 (m, 3H).
Analysis Calcd. for $C_{25}H_{35}N_3O_5$: C 65.62%; H 7.71%; N 9.18%. Found: C 66.24%; H 7.96%; N 9.21%.

**EXAMPLE 18**

**_Trans_ 3,4-dihydro-2,2-dimethyl-3-hydroxy-4-(1-phenyloxycarbonyl-2-piperidinylcarbonylamino)-2H-1-benzopyran-6-carbonitrile** Following the procedure described in example 15, but using phenyl chloroformiate instead of isopropyl chloroformiate, the title compound of this example was obtained as a white solid (yield: 75%).
M.p.: 184.3-193.7 °C;
IR (KBr) $\nu$: 3381, 3277, 3070, 2961, 2929, 2222, 1692, 1642, 1485, 1264, 1202 $cm^{-1}$;
$^1$H NMR (80 MHz, $CDCl_3$) $\delta$ (TMS): 7.5-6.7 (m, 8H, Ar), 6.55 (m, 1H, NH), 5.14 (m, 2H), 4.22 (m, 1H, N-CH-CO), 3.60 (d, J = 10Hz, 0.5H, CHOH), 3.53 (d, J = 10Hz, 0.5H, CHOH), 3.17 (m, 2H), 2.27 (m, 1H), 1.74 (m, 5H), 1.46 (s, 3H, $CH_3$), 1.25 (s, 3H, $CH_3$).
Analysis Calcd. for $C_{25}H_{27}N_3O_5$: C 66.80%; H 6.05%; N 9.35%. Found: C 66.81%; H 6.28%; N 9.14%.

**EXAMPLE 19**

**_Trans_ 3,4-dihydro-2,2-dimethyl-3-hydroxy-4-(1-benzyloxycarbonyl-2-piperidinylcarbonylamino)-2H-1-benzopyran-6-carbonitrile**

Following the procedure described in example 15, but using benzyl chloroformiate instead of isopropyl chloroformiate, the title compound of this example was obtained as a white solid (yield: 71%).
M.p.: 37.5-54.3 °C;
IR (KBr) $\nu$: 3700-3200, 2933, 2222, 1685, 1658, 1523, 1484, 1416, 1263, 1124, 1073 $cm^{-1}$;
$^1$H NMR (80 MHz, $CDCl_3$) $\delta$ (TMS): 7.31 (m, 7H, Ar), 6.87 (d, J = 9Hz, 1H), 6.35 (m, 1H, NH), 5.19 (s, 2H, $OCH_2$), 5.02 (m, 1H), 4.83 (m, 1H), 4.08 (m, 1H), 3.60 (m, 4H, CHOH + $H_2O$), 3.02 (m, 1H), 2.29 (m, 1H), 1.66 (m, 6H), 1.49 (s, 3H, $CH_3$), 1.26 (s, 3H, $CH_3$).
Analysis Calcd. for $C_{26}H_{29}N_3O_5$ . 1.25 $H_2O$: C 64.26%; H 6.49%; N 8.65%. Found: C 64.23%; H 6.66%; N 8.21%.

**EXAMPLE 20**

**Trans 3,4-dihydro-2,2-dimethyl-3-hydroxy-4-(1-isopentanoyl-2 piperidinylcarbonylamino)-2H-1-benzopyran-6-carbonitrile**

Following the procedure described in example 15, but using isopentanoyl chloride instead of isopropyl chloroformiate, the title compound of this example was obtained as a white solid (yield: 56%).
M.p.: 169.9-180.3 °C;
IR (KBr) ν: 3479, 3257, 3070, 2949, 2863, 2220, 1644, 1622, 1483, 1266, 1125, 1068 $cm^{-1}$;
$^1H$ NMR (80 MHz, $CDCl_3$) δ (TMS): 7.36 (m, 2H, Ar), 6.84 (m, 2H, Ar + NH), 5.05 (m, 2H), 3.81 (m, 1H), 3.60 (m, 1H), 3.0 (m, 2H), 2.24 (m, 2H), 2.3-1.5 (m, 7H), 1.49 (s, 3H, $CH_3$), 1.26 (s,3H, $CH_3$), 0.82 (d, J = 3Hz, 6H, $CH(CH_3)_2$).
Analysis Calcd. for $C_{23}H_{31}N_3O_4$ . 0.25 $H_2O$: C 66.11%; H 7.54%; N 10.06%. Found: C 66.31%; H 7.78%; N 9.68%.

**EXAMPLE 21**

**Trans 3,4-dihydro-2,2-dimethyl-3-hydroxy-4-(1-*tert*-butoxycarbonylmethyl-2-piperidinylcarbonylamino)-2H-1-benzopyran-6-carbonitrile**

Following the procedure described in example 15, but using *tert*-butyl bromoacetate instead of isopropyl chloroformiate, the title compound of this example was obtained as a white solid (yield: 62%).
M.p.: 128.7-131.8 °C;
IR (KBr) ν: 3497, 3259, 2931, 2226, 1739, 1720, 1642, 1484, 1416, 1267, 1156, 1127 $cm^{-1}$;
$^1H$ NMR (80 MHz, $CDCl_3$) δ (TMS): 7.50 (m, 3H), 6.86 (d, J = 9Hz, 1H), 5.05 (broad t, J = 9Hz, 1H), 3.8-2.7 (m, 7H), 2.5-1.5 (m, 6H), 1.51 (s, 3H, $CH_3$), 1.44 (s, 9H, $^tBu$), 1.28 (s, 3H, $CH_3$).
Analysis Calcd. for $C_{24}H_{33}N_3O_5$ . 0.5 $H_2O$: C 63.72%; H 7.52%; N 9.29%. Found: C 63.62%; H 7.60%; N 9.30%.

**EXAMPLE 22**

**Trans 3,4-dihydro-2,2-dimethyl-3-hydroxy-4-(1-*tert*-butylaminocarbonyl-2-piperidinylcarbonylamino)-2H-1-benzopyran-6-carbonitrile**

To a solution of 0.5 g (1.5 mmol) of the product obtained in example 14 in 9 mL of chloroform, were added at 0 °C and under argon atmosphere 0.17 mL (1.5 mmol) of *tert*-butyl isocyanate dissolved in 5 mL of chloroform, and the mixture was stirred at room temperature overnight. The solvent was removed and the residue was chromatographed on silica gel eluting with hexane-ethyl acetate mixtures of increasing polarity. 0.36 g of a white solid were obtained (yield: 58%).
M.p.: 215.4-217.0 °C;
IR (KBr) ν: 3485, 3361, 3323, 2969, 2931, 2228, 1642, 1615, 1522, 1483, 1363, 1279, 1262, 1209, 1125, 1071 $cm^{-1}$;
$^1H$ NMR (80 MHz, $CDCl_3$) δ (TMS): 7.44 (m, 2H), 7.13 (m, 1H), 6.85 (d, J = 9Hz, 1H), 5.00 (broad t, J = 9Hz, 1H), 4.73 (m, 1H), 3.57 (d, J = 9Hz, 1H), 3.37 (m, 2H), 2.3-1.4 (m, 8H), 1.50 (s, 3H), 1.30 (s, 12H).
Analysis Calcd. for $C_{23}H_{32}N_4O_4$ . $1H_2O$: C 61.86%; H 7.67%; N 12.55%. Found: C 62.02%; H 7.44%; N 12.15%.

**EXAMPLE 23**

**Trans 3,4-dihydro-2,2-dimethyl-3-hydroxy-4-(1-*tert*-butoxycarbonyl-3-piperidinylcarbonylamino)-2H-1-benzopyran-6-carbonitrile**

Following the procedure described in example 13, but using N-*tert*-butoxycarbonyl-3-piperidincarboxylic acid instead of N-*tert*-butoxycarbonylhomoproline, the title compound of this example was obtained as a white solid (yield: 86%).
M.p.: 205.9-212.9 °C;
IR (KBr) ν: 3417, 3273, 3983, 2972, 2925, 2210, 1658, 1642, 1483, 1267, 1140. 1125 $cm^{-1}$;
$^1H$ NMR (80 MHz, $CDCl_3$) δ (TMS): 7.44 (m, 2H), 6.95 (m, 1H), 6.85 (d, J = 9Hz, 1H), 5.09 (broad t, J = 9Hz, 1H), 4.25 (broad s, 1H), 3.5 (m, 5H), 2.53 (m, 1H), 2.3-1.4 (m, 4H), 1.49 (s, 3H), 1.38 (s, 9H), 1.26 (s, 3H).
Analysis Calcd. for $C_{23}H_{31}N_3O_5$: C 64.32%; H 7.27%; N 9.78%. Found: C 64.48%; H 7.34%; N 9.76%.

**EXAMPLE 24**

***Trans* 3,4-dihydro-2,2-dimethyl-3-hydroxy-4-(3-piperidinylcarbonylamino)-2H-1-benzopyran-6-carbonitrile**

Following the procedure described in example 14, but starting from the compound obtained in example 23, the title compound of this example was obtained as a white solid (yield: 58%).
M.p.: 202.5 ° C;
IR (KBr) ν: 3700-3600, 2929, 2222, 1636, 1484, 1263, 1125, 1076 $cm^{-1}$;
$^1$H NMR (80 MHz, $CDCl_3$) δ (TMS): 8.70 (m, 1H), 7.46 (m, 2H), 6.88 (d, J= 9Hz, 1H), 5.50 (broad t, J= 9Hz, 1H), 3.63 (d, J= 9Hz, 1H), 3.0 (m, 4H), 2.56 (m, 1H), 1.79 (m, 6H+$H_2O$), 1.49 (s, 3H, $CH_3$), 1.28 (s, 3H, $CH_3$).
Analysis Calcd. for $C_{18}H_{23}N_3O_3$ . 1 $H_2O$: C 62.25%; H 7.20%; N 12.10%. Found: C 62.19%; H 6.90%; N 11.88%.

**EXAMPLE 25**

***Trans* 3,4-dihydro-2,2-dimethyl-3-hydroxy-4-(1-methyl-3-piperidinylcarbonylamino)-2H-1-benzopyran-6-carbonitrile**

Following the procedure described in example 13, but using N-methyl-3-piperidincarboxylic acid instead of N-*tert*-butoxycarbonylhomoproline, the title compound of this example was obtained as a white solid (yield: 64%).
M.p.: 150.8-155.4 ° C;
IR (KBr) ν: 3453, 3232, 3067, 2929, 2220, 1678, 1625, 1605, 1484, 1280, 1268, 1124, 1071$cm^{-1}$;
$^1$H NMR (80 MHz, $CDCl_3$) δ (TMS): 9.25 (m, 1H), 7.52 (m, 2H), 6.89 (d, J= 9Hz, 1H), 4.99 (broad t, J= 10Hz, 1H), 3.66 (broad d, J= 10Hz, 1H), 3.1-1.5 (complex signal, 10H + 1.6$H_2O$), 2.23 (s, 3H, N-$CH_3$), 1.51 (s, 3H, $CH_3$), 1.29 (s, 3H, $CH_3$).
Analysis Calcd. for $C_{19}H_{25}N_3O_3$ . 1.6 $H_2O$: C 61.32%; H 7.58%; N 11.30%. Found: C 61.10%; H 7.34%; N 11.38%.

**EXAMPLE 26**

***Trans* 3,4-dihydro-2,2-dimethyl-3-hydroxy-4-(1-methoxycarbonyl-3-piperidinylcarbonylamino)-2H-1-benzopyran-6-carbonitrile**

Following the procedure described in example 15, but starting from the product obtained in example 24 and using methyl chloroformiate instead of isopropyl chloroformiate, the title compound of this example was obtained as a white solid (yield: 86%).
M.p.: 97.0-100.3 ° C;
IR (KBr) ν: 3600-3200, 2971, 2933, 2859, 2223, 1671, 1483, 1263, 1242, 1124 $cm^{-1}$;
$^1$H NMR (80 MHz, $CDCl_3$) δ (TMS): 7.45 (m, 2H), 6.85 (d, J= 9Hz, 1H), 6.54 (broad d, J= 9Hz, 1H), 5.09 (t, J= 9Hz, 1H), 4.12 (m, 1H), 3.9-3.2 (m, 4H), 3.64 (s, 3H, $OCH_3$), 2.53 (m, 1H), 2.2-1.5 (complex signal, 5H), 1.50 (s, 3H, $CH_3$), 1.27 (s, 3H, $CH_3$).
Analysis Calcd. for $C_{20}H_{25}N_3O_5$ . 0.25 $H_2O$: C 61.30%; H 6.51%; N 10.73%. Found: C 61.07%; H 6.86%; N 10.06%.

**EXAMPLE 27**

***Trans* 3,4-dihydro-2,2-dimethyl-3-hydroxy-4-(1-*tert*-butoxycarbonyl-4-piperidinylcarbonylamino)-2H-1-benzopyran-6-carbonitrile**

Following the procedure described in example 13, but using N-*tert*-butoxycarbonyl-4-piperidincarboxylic acid instead of N-*tert*-butoxycarbonylhomoproline, the title compound of this example was obtained as a white solid (yield: 78%).
M.p.: 189-3-192.8 ° C;
IR (KBr) ν: 3600-3200, 2972, 2925, 2229, 1695, 1626, 1485, 1417, 1362, 1279, 1214, 1161 $cm^{-1}$;
$^1$H NMR (80 MHz, $CDCl_3$) δ (TMS): 7.46 (m, 2H), 6.88 (d, J= 9Hz, 1H), 6.05 (d, J= 9Hz, 1H), 5.07 (t, J=

9Hz, 1H), 4.17 (m, 2H), 3.62 (d, J = 9Hz, 1H), 3.4 (broad s, 1H), 2.81 (m, 2H), 2.30 (m, 1H), 2.0-1.5 (m, 4H), 1.50 (s, 3H), 1.46 (s, 9H), 1.27 (s, 3H).
Analysis Calcd. for $C_{23}H_{31}N_3O_5$: C 64.32%; H 7.27%; N 9.78%. Found: C 64.50%; H 7.73%; N 9.92%.

## EXAMPLE 28

**<u>*Trans*3,4-dihydro-2,2-dimethyl-3-hydroxy-4-[(2S)-1-*tert*-butoxycarbonyl-2-pyrrolidinylcarbonylamino]-2H-1-benzopyran-3-ol</u>**

Following the procedure described in example 13, but using N-*tert*-butoxycarbonyl-L-proline instead of N-*tert*-butoxycarbonylhomoproline, a crude was obtained, which was subsequently chromatographed on silica gel using hexane-ethyl acetate mixtures of increasing polarity as eluent. The chromatography yielded three fractions:

1- **Isomer A: <u>Trans (3R,4S)-3,4-dihydro-2,2-dimethyl-3-hydroxy-4-[(2S)-1-*tert*-butoxycarbonyl-2-pyrrolidinylcarbonylamino]-2H-1-benzopyran-6-carbonitrile</u>**
Yield: 29%:
M.p.: 186.1-190.0 ° C;
$(\alpha)^{20}$ (2, $CHCl_3$) = -134.97 °
IR (KBr) $\nu$: 3600-3200, 2971, 2931, 2222, 1649, 1410, 1262, 1125 $cm^{-1}$;
$^1H$ NMR (80 MHz, $CDCl_3$) $\delta$ (TMS): 7.40 (m, 2H), 7.20 (m, 1H), 6.85 (d, J = 9Hz, 1H), 5.03 (t, J = 9Hz, 1H), 4.33 (m, 1H), 3.60 (d, J = 9Hz, 1H), 3.45 (m, 2H), 2.4-1.5 (complex signal, 5H), 1.47 (s, 12H), 1.23 (s, 3H).
Analysis Calcd. for $C_{22}H_{29}N_3O_5$ . 0.25 $H_2O$: C 62.93%; H 7.03%; N 10.01%. Found: C 63.23%; H 7.16%; N 9.64%.

2- Mixture of isomers (yield: 31%).

3- **Isomer B: <u>*Trans* (3S,4R)-3,4-dihydro-2,2-dimethyl-3-hydroxy-4-[(2S)-1-*tert*-butoxycarbonyl-2-pyrrolidinylcarbonylamino]-2H-1-benzopyran-6-carbonitrile</u>**

Yield: 18%:
M.p.: 86.1-95.9 ° C;
$(\alpha)^{20}$ (2, $CHCl_3$) = -27.42 °
IR (KBr) $\nu$: 3600-3200, 2973, 2223, 1664, 1484, 1388, 1264, 1160, 1125 $cm^{-1}$;
$^1H$ NMR (80 MHz, $CDCl_3$) $\delta$ (TMS): 7.43 (m, 2H), 6.85 (d, J = 9Hz, 1H), 6.85 (m, 1H), 5.06 (t, J = 9Hz, 1H), 4.30 (m, 1H), 3.63 (d, J = 9Hz, 1H), 3.45 (t, J = 6Hz, 2H), 2.10 (m, 5H), 1.49 (s, 12H), 1.28 (s, 3H).
Analysis Calcd. for $C_{22}H_{29}N_3O_5$ . 0.5 $H_2O$: C 62.26%; H 7.07%; N 9.90%. Found: C 62.59%; H 7.20%; N 9.52%.

## EXAMPLE 29

**<u>*Trans* 3,4-dihydro-2,2-dimethyl-3-hydroxy-4-[(2S)-2-pyrrolidinylcarbonylamino]-2H-1-benzopyran-6-carbonitrile. monohydrochloride</u>**

Following the procedure described in example 14a, but starting from the several isomers of the product obtained in example 28, the isomers of the title compound of this example were obtained as white solids.

1-**Isomer A: <u>*Trans* (3R,4S)-3,4-dihydro-2,2-dimethyl-3-hydroxy-4-[(2S)-2-pyrrolidinylcarbonylamino]-2H-1-benzopyran-6-carbonitrile. monohydrochloride</u>**

Yield: 73%.
M.p.: >300 ° C;
$(\alpha)^{20}$(0.2, $H_2O$) = + 23.69 °;
IR (KBr) $\nu$: 3600-2400, 2221, 1654, 1482, 1378, 1367, 1305, 1280, 1259, 1125, 1076 $cm^{-1}$;
$^1H$ NMR (80 MHz, $D_2O$) $\delta$ (TMS): 7.60 (m, 2H), 6.98 (d, J = 9Hz, 1H), 5.01 (d, J = 10Hz, 1H), 4.69 (s, 4H + $H_2O$), 4.51 (m, 1H), 3.80 (d, J = 10Hz, 1H), 3.48 (t, J = 6Hz, 2H), 2.70-1.90 (complex signal, 4H), 1.51 (s, 3H, $CH_3$), 1.29 (s, 3H, $CH_3$).
Analysis Calcd. for $C_{17}H_{22}ClN_3O_3$ . 0.25 $H_2O$: C 57.30%; H 6.32%; N 11.79%. Found: C 57.35%; H 6.40%;

N 11.51%.

2-Mixture of isomers (yield: 98%).

3-**Isomer B:** ***Trans* (3S,4R)-3,4-dihydro-2,2-dimethyl-3-hydroxy-4-[(2S)-2 pyrrolidinylcarbonylamino]-2H-1-benzopyran-6-carbonitrile. monohydrochlo-ride**

Yield: 62%.
M.p.: 280.2-282.8 °C;
$(\alpha)^{20}$ (0.2, $H_2O$) = -50.24 °;
IR (KBr) $\nu$: 3600-2500, 2222, 1671, 1483, 1366, 1262, 1125 $cm^{-1}$;
$^1H$ NMR (80 MHz, $CDCl_3$) $\delta$ (TMS): 9.64 (m, 1H), 8.25 (m, 2H), 7.30 (m, 2H), 6.84 (d, J= 9Hz, 1H), 5.03 (m, 1H), 4.0-3.0 (complex signal, 5H), 2.8-1.9 (complex signal, 4H), 1.30 (s, 3H, $CH_3$), 1.15 (s, 3H, $CH_3$).
Analysis Calcd. for $C_{17}H_{22}ClN_3O_3$ . 0.75 $H_2O$: C 55.89%; H 6.43%; N 11.51%. Found: C 55.78%; H 5.95%; N 11.34%.

## EXAMPLE 30

***Trans* 3,4-dihydro-2,2-dimethyl-3-hydroxy-4-[(2S)-1-methoxycarbonyl-2-pyrrolidinylcarbonylamino]-2H-1-benzopyran-6-carbonitrile**

Following the procedure described in example 15, but starting from the product obtained in example 29 (mixture of isomers), after having been basified, and using methyl chloroformiate instead of isopropyl chloroformiate, the title compound of this example was obtained as a white solid (yield: 73%).
M.p.: 200.8-201.8 °C;
IR (KBr) $\nu$: 3431, 3387, 3239, 2972, 2233, 1706, 1683, 1642, 1529, 1484, 1446, 1384, 1267, 1119 $cm^{-1}$;
$^1H$ NMR (80 MHz, $CDCl_3$) $\delta$ (TMS): 7.45 (m, 2H), 6.84 (d, J= 9Hz, 1H), 6.6 (m, 1H), 5.05 (m, 1H), 4.65 (m, 2H), 3.77 (s, 0.5 3H), 3.75 (s, 0.5 3H), 3.65 (m, 1H), 3.51 (t, J= 6Hz, 2H), 2.6-1.6 (m, 4H), 1.51 (s, 3H), 1.28 (s, 3H)
Analysis Calcd. for $C_{19}H_{23}N_3O_5$ . 0.25 $H_2O$: C 60.40%; H 6.23%; N 11.12%. Found: C 60.34%; H 6.25%; N 10.67%.

## EXAMPLE 31

***Trans* 3,4-dihydro-2,2-dimethyl-3-hydroxy-4-[(2S)-1-ethoxycarbonyl-2-pyrrolidinylcarbonylamino]-2H-1-benzopyran-6-carbonitrile**

Following the procedure described in example 30, but using ethyl chloroformiate instead of methyl chloroformiate, the title compound of this example was obtained as a white solid (yield: 65%).
M.p.: 88.5-89.1 °C;
IR (KBr) $\nu$: 3600-3200, 2973, 2223, 1666, 1605, 1484, 1416, 1264 $cm^{-1}$;
$^1H$ NMR (80 MHz, $CDCl_3$) $\delta$ (TMS): 7.45 (m, 2H), 6.85 (m, 2H), 5.05 (m, 1H), 4.70 (m, 1H), 4.15 (broad q, J= 7.5Hz, 2H, $\underline{CH_2}CH_3$), 3.63 (d, J= 10Hz, 1H), 3.51 (t, J= 6Hz, 2H), 2.03 (m, 5H), 1.50 (s, 3H, $CH_3$), 1.28 (s, 3H, $CH_3$), 1.28 (t, J= 7.5Hz, 3H, $CH_2CH_3$).
Analysis Calcd. for $C_{20}H_{25}N_3O_3$ . 0.75 $H_2O$: C 59.93%; H 6.62%; N 10.48%. Found: C 59.94%; H 6.69%; N 10.04%.

## EXAMPLE 32

***Trans* 3,4-dihydro-2,2-dimethyl-3-hydroxy-4-[(2S)-1-isopropoxycarbonyl-2-pyrrolidinylcarbonylamino]-2H-1-benzopyran-6-carbonitrile**

Following the procedure described in example 30, but using isopropyl chloroformiate instead of methyl chloroformiate, the title compound of this example was obtained as a white solid (yield: 33%).
M.p.: 101.0-121.0 °C;
IR (KBr) $\nu$: 3600-3200, 2975, 2220, 1658, 1483, 1421, 1267, 1125 $cm^{-1}$;
$^1H$ NMR (80 MHz, $CDCl_3$) $\delta$ (TMS): 7.45 (m, 2H), 7.26 (m, 1H), 6.85 (d, J= 9Hz, 1H), 5.00 (m, 2H), 4.37 (m, 1H), 3.50 (m, 4H), 2.4-1.3 (m, 4H), 1.50 (s, 3H), 1.28 (m, 9H).

Analysis Calcd. for $C_{21}H_{27}N_3O_5$: C 62.83%; H 6.78%; N 10.47%. Found: C 62.97%; H 7.08%; N 10.44%.

## EXAMPLE 33

**_Trans_ 3,4-dihydro-2,2-dimethyl-3-hydroxy-4-[(2S)-1-isobutoxycarbonyl-2-pyrrolidinylcarbonylamino]-2H-1-benzopyran-6-carbonitrile**

Isomer A

Following the procedure described in example 30, but starting from stereoisomer A of the product obtained in example 29, after having been basified, and using isobutyl chloroformiate instead of methyl chloroformiate, the title compound of this example was obtained as a white solid (yield: 80%).
M.p.: 82.6-87.9 ° C;
$(\alpha)^{20}$(0.2, $CHCl_3$) = -119.0 ° ;
IR (KBr) $\nu$: 3600-3200, 2967, 2222, 1670, 1605, 1484, 1421, 1264, 1125 $cm^{-1}$;
$^1$H NMR (80 MHz, $CDCl_3$) $\delta$ (TMS): 7.45 (m, 2H), 7.25 (m, 1H), 6.85 (d, J = 9Hz, 1H), 5.00 (t, J = 9Hz, 1H), 4.45 (m, 1H), 3.94 (d, J = 6.5Hz, 2H, $OCH_2$, 3.58 (m, 3H), 2.5-1.3 (complex signal, 6H), 1.50 (s, 3H, $CH_3$), 1.28 (s, 3H, $CH_3$), 0.95 (d, J = 6.5Hz, 6H, $CH(CH_3)_2$).
Analysis Calcd. for $C_{22}H_{29}N_3O_5$: C 63.60%; H 7.04%; N 10.11%. Found: C 63.41%; H 7.20%; N 9.40%.

Isomer B

Following the procedure described in example 30, but starting from stereoisomer B of the product obtained example 29, after having been basified, and using isobutyl chloroformiate instead of methyl chloroformiate, the title compound of this example was obtained as a white solid (yield: 79%).
M.p.: 84.6-89.4 ° C;
$(\alpha)^{20}$(0.2, $CHCl_3$) = -39.5 ° ;
IR (KBr) $\nu$: 3600-3200, 2967, 2222, 1670, 1605, 1484, 1421, 1264, 1125 $cm^{-1}$;
$^1$H NMR (80 MHz, $CDCl_3$) $\delta$ (TMS): 7.45 (m, 2H), 6.85 (d, J = 9Hz, 1H), 6.60 (m, 1H), 5.06 (t, J = 9Hz, 1H), 4.30 (m, 1H), 3.89 (d, J = 6.5Hz, 2H, $OCH_2$), 3.59 (m,3H), 2.10 (complex signal, 6H), 1.51 (s, 3H, $CH_3$), 1.28 (s, 3H, $CH_3$), 0.94 (d, J = 6.5Hz, 6H, $CH(CH_3)_2$).
Analysis Calcd. for $C_{22}H_{29}N_3O_5$: C 63.60%; H 7.04%; N 10.11%. Found: C 63.57%; H 7.24%; N 9.78%.

## EXAMPLE 34

**_Trans_ 3,4-dihydro-2,2-dimethyl-3-hydroxy-4-[(2S)-1-phenoxycarbonyl-2-pyrrolidinylcarbonylamino]-2H-1-benzopyran-6-carbonitrile**

Following the procedure described in example 30, but using phenyl chloroformiate instead of methyl chloroformiate, the title compound of this example was obtained as a white solid (yield: 75%).
M.p.: 83.0-92.5 ° C;
IR (KBr) $\nu$: 3600-3200, 3062, 2971, 2222, 1701, 1658, 1484, 1392, 1198 $cm^{-1}$;
$^1$H NMR (80 MHz, $CDCl_3$) $\delta$ (TMS): 7.28 (m, 7H), 16.83 (d, J = 9Hz:, 1H), 6.65 (m, 1H), 5.06 (m, 1H), 4.43 (m, 1H), 3.60 (m, 3H), 2.6-1.7 (m, 5H), 1.46 (s, 3H, $CH_3$), 1.24 (s, 3H, $CH_3$).
Analysis Calcd. for $C_{24}H_{25}N_3O_5$ . 0.5 $H_2O$: C 64.86%; H 5.86%; N 9.46%. Found: C 65.04%; H 5.97%; N 9.08%.

## EXAMPLE 35

**_Trans_3,4-dihydro-2,2-dimethyl-3-hydroxy-4-[(2S)-1-benzyloxycarbonyl-2-pyrrolidinylcarbonylamino]-2H-1-benzopyran-6-carbonitrile**

Following the procedure described in example 30, but using benzyl chloroformiate instead of methyl chloroformiate, the title compound of this example was obtained as a white solid (yield: 70%).
M.p.: 73.5-88.1 ° C;
IR (KBr) $\nu$: 3600-3200, 2971, 2929, 2873, 2222, 1665, 1605, 1484, 1415, 1354, 1264, 1125, 1076 $cm^{-1}$;
$^1$H NMR (80 MHz, $CDCl_3$) $\delta$ (TMS): 7.35 (m, 7H), 6.85 (d, J = 9Hz, 1H), 6.69 (m, 1H), 5.20 (s, 0.5 2H), 5.17 (s, 0.5 2H,), 5.03 (m, 1H), 4.44 (m, 1H), 3.55 (m, 3H), 2.4-1.4 (complex signal, 5H), 1.49 (s,3H), 1.26 (s, 3H).

Analysis Calcd. for $C_{25}H_{27}N_3O_5$: C 66.80%; H 6.05%; N 9.35%. Found: C 66.67%; H 6.30%; N 8.98%.

Isomer A: ***Trans* (3R,4S)-3,4-dihydro-2,2-dimethyl-3-hydroxy-4-[(2S)-1-benzyloxy-carbonyl-2-pyrrolidinylcarbonylamino]-2H-1-benzopyran-6-carbonitrile**

Method 1

Following the procedure described in example 30, but starting from stereoisomer A of the product obtained in example 29, after having been basified, the title compound of this example was obtained as a white solid (yield: 92%).
M.p.: 190.0-191.5 ° C;
$(\alpha)^{20}$(2, $CHCl_3$) = -105 ° ;
IR (KBr) ν: 3600-3200, 2975, 2926, 2890, 2227, 1672, 1607, 1484, 1444, 1414, 1360, 1268, 1121, 1072 $cm^{-1}$;
$^1$H NMR (80 MHz, $CDCl_3$) δ (TMS): 7.35 (m, 7H), 7.00 (broad s., 1H), 6.85 (d, J = 9Hz, 1H), 5.20 (s, 0.5 2H), 5.17 (s, 0.5 2H), 4.97 (t, J = 8Hz, 1H), 4.31 (m, 1H), 3.52 (m, 3H), 2.4-1.8 (complex signal, 5H), 1.48 (s, 3H), 1.26 (s, 3H).
$^{13}$C NMR (80 MHz, $CDCl_3$) δ (TMS): 18.71 (q), 24.69 (2t), 26.46 (q), 29.27 (t), 47.42 (t), 50.66 (d), 61.00 (d), 67.91 (t), 75.07 (d), 80.16 (s), 104.15 (s), 115.54 (d), 118.53 (d), 128.13 (d), 128.36 (d), 128.63 (d), 130.02 (s), 132.13 (d), 133.12 (s), 136.16 (s), 156.92 (s), 174.32 (s).
Analysis Calcd. for $C_{25}H_{27}N_3O_5$: C 66.80%; H 6.05%; N 9.35%. Found: C 66.81%; H 6.16%; N 9.11%.

Method 2

Following the procedure described in example 13, but using N-benzyloxycarbonyl-L-proline instead of N-*tert*-butoxycarbonylhomoproline and starting from (3**R**,4**S**)-4-amino-3,4-dihydro-2,2-dimethyl-3-hydroxy-2H-1-benzopyran-6-carbonitrile (EP 344747), the title compound of this example was obtained as a white solid, identical to the one obtained following Method 1 (yield: 73%).

Isomer B: **Trans (3S,4R)-3,4-dihydro-2,2-dimethyl-3-hydroxy-4-[(2S)-1-benzyloxy-carbonyl-2-pyrrolidinylcarbonylamino]-2H-1-benzopyran-6-carbonitrile**

Method 1

Following the procedure described in example 30, but starting from stereoisomer B of the product obtained in example 29, after having been basified, the title compound of this example was obtained as a white solid (yield: 76%).
M.p.: 172.7-175.7 ° C;
$(\alpha)^{20}$(2, $CHCl_3$) = -42.2 ° ;
IR (KBr) ν: 3600-3200, 3082, 2973, 2933, 2871, 2222, 1686, 1665, 1617, 1536, 1485, 1426, 1405, 1354, 1263, 1126, 1089 cmΠ9;
9H NMR (80 MHz, CDCl ) δ (TMS): 7.35 (m, 7H), 6.85 (d, J = 9Hz, 1H), 6.79 (m, 1H), 5.11 (s, 2H), 5.05 (m, 1H), 4.33 (m, 1H), 3.52 (m, 4H), 2.4-1.4 (complex signal, 4H), 1.50 (s, 3H), 1.26 (s, 3H).
93C NMR (80 MHz, CDCl ) δ (TMS): 18.75 (q), 24.68 (t), 26.55 (q), 29.69 (t), 47.39 (t), 50.44 (d), 61.08 (d), 67.77 (t), 74.98 (d), 80.25 (s), 104.21 (s), 118.51 (d), 119.00 (s), 122.84 (d), 128.00 (d), 128.32 (d), 128.63 (d), 132.26 (s), 133.14 (d), 136.16 (s), 156.01 (s), 156.92 (s), 174.32 (s).
Analysis Calcd. for C♂′H♂<N O′ . 0.25 H♂O: C 66.15%; H 6.06%; N 9.26%. Found: C 66.27%; H 6.11%; N 9.15%.

Method 2

Following the procedure described in example 13, but using N-benzyloxycarbonyl-L-proline instead of N-*tert*-butoxycarbonylhomoproline and starting from (3**S**,4**R**)-4-amino-3,4-dihydro-2,2-dimethyl-3-hydroxy-2H-1-benzopyran-6-carbonitrile (EP 344747; EP 385584), the title compound of this example was obtained as a white solid, identical to the one obtained following Method 1 (yield: 76%).

**EXAMPLE 36**

**_Trans_ 3,4-dihydro-2,2-dimethyl-3-hydroxy-4-[(2R)-1-benzyloxycarbonyl-2-pyrrolidinilcarbonylamino]-2H-1-benzopyran-6-carbonitrile**

Isomer A: **_Trans_(3R,4S)-3,4-dihydro-2,2-dimethyl-3-hydroxy-4-[(2R)-1-benzyloxy-carbonyl-2-pyrrolidinilcarbonylamino]-2H-1-benzopyran-6-carbonitrile**

Following the procedure described in example 13, but using N-benzyloxycarbonyl-D-proline instead of N-*tert*-butoxycarbonylhomoproline and starting from (3**R**,4**S**)-4-amino-3,4-dihydro-2,2-dimethyl-3-hydroxy-2H-1-benzopyran-6-carbonitrile (EP 344747), the title compound of this example was obtained as a white solid (yield: 73%).
M.p.: 155.8-159.10C;
(α)2⁰(2, CHCl ) = +38.10;
IR (KBr) ν: 3600-3200, 2973, 2923, 2846, 2231, 2222, 1686, 1666, 1617, 1484, 1426, 1405, 1354, 1262, 1126 cmΠ9;
9H NMR (80 MHz, CDCl ) δ (TMS): 7.35 (m, 7H), 6.85 (d, J= 9Hz, 1H), 6.69 (m, 1H), 5.15 (s, 2H), 5.05 (m, 1H), 4.30 (m, 1H), 4.04 (m, 1H), 3.54 (m, 3H), 2.4-1.4 (complex signal, 4H), 1.50 (s, 3H), 1.28 (s, 3H).
Analysis Calcd. for C♂′H♂<N O′: C 66.80%; H 6.05%; N 9.35%. Found: C 66.76%; H 6.12%; N 9.01%.

Isomer B: **_Trans_(3S,4R)-3,4-dihydro-2,2-dimethyl-3-hydroxy-4-[(2R)-1-benzyloxy-carbonyl-2-pyrrolidinilcarbonylamino]-2H-1-benzopyran-6-carbonitrile**

Following the procedure described in example 13, but using N-benzyloxycarbonyl-D-proline instead of N-*tert*-butoxycarbonylhomoproline and starting from (3**S**,4**R**)-4-amino-3,4-dihydro-2,2-dimethyl-3-hydroxy-2H-1-benzopyran-6-carbonitrile (EP 344747; EP 385584), the title compound of this example was obtained as a white solid (yield: 59%).
M.p.: 186.5-187.70C;
(α)2⁰(2, CHCl ) = +94.40;
IR (KBr) ν: 3600-3200, 2971, 2929, 2873, 2222, 1665, 1605, 1484, 1415, 1354, 1264, 1125, 1076 cmΠ9;
9H NMR (80 MHz, CDCl ) δ (TMS): 7.34 (m, 7H), 6.85 (d, J= 9Hz, 1H), 6.69 (m, 1H), 5.20 (s, 0.5 2H), 5.17 (s, 0.5 2H), 4.98 (t, J= 8Hz, 1H), 4.43 (m, 1H), 3.55 (m, 3H), 2.4-1.4 (complex signal, 5H), 1.49 (s, 3H), 1.26 (s, 3H).
Analysis Calcd. for C♂′H♂<N O′: C 66.80%; H 6.05%; N 9.35%. Found: C 66.91%; H 6.05%; N 9.35%.

## EXAMPLE 37

**_Trans_ 3,4-dihydro-2,2-dimethyl-3-hydroxy-4-[(2S)-1-acetyl-2-pyrrolidinylcarbonylamino]-2H-1-benzopyran-6-carbonitrile**

Following the procedure described in example 13, but using N-acetil-L-proline instead of N-*tert*-butoxycarbonylhomoproline, the title compound of this example was obtained as a white solid (yield: 37%).
M.p.: 161.3-197.10C;
IR (KBr) ν: 3600-3200, 3071, 2974, 2221, 1643, 1483, 1416, 1265, 1123 cmΠ9;
9H NMR (80 MHz, CD OD) δ (TMS): 7.5 (m, 2H), 6.83 (m, 1H), 4.95 (d, J= 10Hz, 1H), 4.75 (s, 2H + H♂O), 4.45 (m, 1H), 3.68 (m, 3H), 2.13 (m, 7H), 1.49 (s, 3H), 1.27 (s, 3H).
Analysis Calcd. for C±>H♂ N OΔ . 0.5 H♂O: C 62.29%; H 6.56%; N 11.47%. Found: C 62.64%; H 6.38%; N 11.24%.

## EXAMPLE 38

**_Trans_3,4-dihydro-2,2-dimethyl-3-hydroxy-4-[(2S)-1-_tert_-butanoyl-2-pyrrolidinilcarbonylamino]-2H-1-benzopyran-6-carbonitrile**

Following the procedure described in example 30, but using *tert*-butiryl chloride instead of methyl chloroformiate, the following 3 fractions were obtained after chromatography:
1-Isomer A (yield: 25%).
M.p.: 219.8-220.00C;
IR (KBr) ν: 2973, 2218, 1668, 1649, 1594, 1482, 1364, 1264, 1122 cmΠ9;
$^1$H NMR (80 MHz, CDCl$_3$) δ (TMS): 7.38 (m, 1H), 7.27 (d, J= 10Hz, 1H), 6.85 (d, J= 10Hz, 1H), 6.48 (m,

1H), 5.05 (t, J= 10Hz, 1H), 4.35 (m, 1H), 3.75 (m, 3H), 3.2 (broad s, 1H), 2.1 (m, 4H), 1.48 (s, 3H), 1.24 (s, 3H), 1.20 (s, 9H).
Analysis Calcd. for $C_{22}H_{29}N_3O_4$: C 66.14%; H 7.32%; N 10.52%. Found: C 66.39%; H 7.54%; N 10.32%.
2-Mixture of isomers (yield: 39%).
3-Isomer B (yield: 18%).
M.p.: 67.5-99.6 °C;
IR (KBr) ν: 3600-3200, 2971, 2222, 1658, 1605, 1484, 1264, 1125 $cm^{-1}$;
$^1H$ NMR (80 MHz, $CDCl_3$) δ (TMS): 7.45 (m, 3H), 6.83 (d, J= 10Hz, 1H), 4.85 (t, J= 10Hz, 1H), 4.60 (m, 1H), 3.60 (m, 3H), 2.70 (broad s, OH + $H_2O$), 2.5-1.8 (complex signal, 4H), 1.50 (s, 3H), 1.32 (s, 9H), 1.26 (s, 3H).
Analysis Calcd. for $C_{22}H_{29}N_3O_4$ . 0.75 $H_2O$: C 64.00%; H 7.39%; N 10.18%. Found: C 63.86%; H 7.44%; N 9.75%.

**EXAMPLE 39**

***Trans* 3,4-dihydro-2,2-dimethyl-3-hydroxy-4-[(2S)-1-isopropylaminocarbonyl-2-pyrrolidinylcarbonylamino]-2H-1-benzopyran-6-carbonitrile**

To a solution of 0.5 g (1.6 mmol) of the product obtained in example 29 (mixture of isomers), after having been basified, in 5mL of chloroform, were added at 0 °C and under argon atmosphere 0.16 mL (1.6 mmol) of isopropyl isocyanate dissolved in 5 mL of chloroform, and the mixture was stirred at room temperature overnight. The solvent was removed and the residue was chromatographed on silica gel eluting with hexane-ethyl acetate mixtures of increasing polarity. 0.44 g of a solid were obtained, which was recrystallized from ethyl acetate-ether yielding 0.100 g of a white solid (yield: 16%).
M.p.: 135.5-137.3 °C;
IR (KBr) ν: 3600-3200, 2969, 2219, 1671, 1606, 1536, 1483, 1265, 1126 $cm^{-1}$;
$^1H$ NMR (80 MHz, $CDCl_3$) δ (TMS): 7.89 (m, 1H), 7.38 (m, 2H), 6.83 (d, J= 10Hz, 1H), 4.96 (broad t, J= 10Hz, 1H), 4.5-1.8 (complex signal, 11H), 1.49 (s, 3H), 1.21 (m, 9H).
Analysis Calcd. for $C_{21}H_{28}N_4O_4$ . $1H_2O$: C 60.29%; H 7.18%; N 13.40%. Found: C 60.69%; H 6.97%; N 13.25%.

**EXAMPLE 40**

***Trans* 3,4-dihydro-2,2-dimethyl-3-hydroxy-4-[(2S)-1-isobutylaminocarbonyl-2-pyrrolidinylcarbonylamino]-2H-1-benzopyran-6-carbonitrile**

Following the procedure described in example 39, but using isobutyl isocyanate instead of isopropyl isocyanate, the title compound of this example was obtained as a white solid (yield: 42%).
M.p.: 104.1-113.1 °C;
IR (KBr) ν: 3600-3200, 2953, 2222, 1680, 1625, 1537, 1483, 1264, 1125 $cm^{-1}$;
$^1H$ NMR (80 MHz, $CDCl_3$) δ (TMS): 7.84 (m, 0.5H), 7.39 (m, 2H), 7.05 (m, 0.5H), 6.84 (d, J= 9Hz, 1H), 4.96 (m, 1H), 4.53 (m, 2H), 3.7-3.5 (complex signal, 11H), 1.50 (s, 3H), 1.27 (s, 3H), 0.90 (d, J= 6.8Hz, 6H).
Analysis Calcd. for $C_{22}H_{30}N_4O_5$ . 0.75 $H_2O$ . 0,25 $Et_2O$ : C 61.88%; H 7.62%; N 12.56%. Found: C 61.99%; H 7.51%; N 12.56%.

**EXAMPLE 41**

***Trans* 3,4-dihydro-2,2-dimethyl-3-hydroxy-4-[(5S)-5-pyrrolidonylcarbonylamino]-2H-1-benzopyran-6-carbonitrile**

Following the procedure described in example 13, but using L-pyroglutamic acid instead of N-*tert*-butoxycarbonylhomoproline, the title compound of this example was obtained as a white solid (yield: 20%).
M.p.: 151.0-157.7 °C;
IR (KBr) ν: $cm^{-1}$
$^1H$ NMR (80 MHz, $CDCl_3$) δ (TMS): 7.60 (m, 0.5H), 7.36 (m, 2H), 7.0 (m, 0.5H), 6.83 (d, J= 10Hz, 1H), 5.15 (m, 1H), 4.59 (m, 1H), 3.73 (m, 1H), 3.0-1.5 (complex signal, 6H), 1.43 (s, 3H), 1.25 (s, 3H).
Analysis Calcd. for $C_{17}H_{19}N_3O_4$ . 1.5 $H_2O$: C 57.30%; H 6.18%; N 11.80%. Found: C 57.59%; H 6.23%; N 11.31%.

## EXAMPLE 42

***Trans* 3,4-dihydro-2,2-dimethyl-3-hydroxy-4-[(3R,5S)-1-*tert*-butoxycarbonyl-3-hydroxy-5-pyrrolidinylcarbonylamino]-2H-1-benzopyran-6-carbonitrile**

Following the procedure described in example 13, but using N-*tert*-butoxycarbonyl-L-4-hydroxyproline instead of N-*tert*-butoxycarbonylhomoproline, the title compound of this example was obtained as a white solid (yield: 59%).
M.p.: 100.6-100.8 °C;
IR (KBr) $\nu$: 3600-3200, 2973, 2223, 1664, 1605, 1484, 1408, 1365, 1264, 1159, 1126 $cm^{-1}$;
$^1H$ NMR (80 MHz, $CDCl_3$) $\delta$ (TMS): 7.45 (m, 2H), 7.06 (m, 1H), 6.85 (d, J = 9Hz, 1H), 5.01 (m, 1H), 4.52 (m, 2H), 3.58 (m, 3H), 2.7-2.0 (complex signal, 4H + $H_2O$), 1.48 (s, 12H), 1.29 (s, 3H).
Analysis Calcd. for $C_{22}H_{29}N_3O_6$ . $1H_2O$: C 58.79%; H 6.95%; N 9.35%. Found: C 59.14%; H 6.91%; N 8.80%.

## EXAMPLE 43

***Trans* 3,4-dihydro-2,2-dimethyl-3-hydroxy-4-[(3R,5S)-1-isobutoxycarbonyl-3-hydroxy-5-pyrrolidinylcarbonylamino]-2H-1-benzopyran-6-carbonitrile a) *Trans* 3,4-dihydro-2,2-dimethyl-3-hydroxy-4-[(3R,5S)-3-hydroxy-5-pyrrolidinylcarbonylamino]-2H-1-benzopyran-6-carbonitrile**

Following the procedure described in example 14, but starting from the product obtained in example 42, the title compound of this example was obtained as a white solid (yield: 62%), which was directly used in the next step.

**b) *Trans* 3,4-dihydro-2,2-dimethyl-3-hydroxy-4-[(3R,5S)-1-isobutoxycarbonyl-3-hydroxy-5-pyrrolidinylcarbonylamino]-2H-1-benzopyran-6-carbonitrile**

Following the procedure described in example 30, but starting from the product obtained in example 43a and using isobutyl chloroformiate instead of methyl chloroformiate, the title compound of this example was obtained as a white solid (yield: 69%).
M.p.: 97.5-103.4 °C;
IR (KBr) $\nu$: 3600-3200, 2959, 2871, 2223, 1671, 1606, 1536, 1484, 1426, 1264, 1126 $cm^{-1}$;
$^1H$ NMR (80 MHz, $CDCl_3$) $\delta$ (TMS): 7.43 (m, 2H), 7.09 (m, 1 H), 6.85 (d, J = 10 Hz, 1H), 5.00 (m, 1H), 4.61 (m, 2H), 4.12 (d, J = 9Hz, 2H), 3.61 (m, 3H), 2.8-1.8 (complex signal, 5H), 1.50 (s, 3H), 1.28 (s, 3H), 0.92 (d, J = 6.5Hz, 6H).
Analysis Calcd. for $C_{22}H_{29}N_3O_6$ . 0.25 $H_2O$: C 60.62%; H 6.77%; N 9.64%. Found: C 60.52%; H 7.07%; N 8.96%.

## EXAMPLE 44

***Trans* 4-cyclopentylcarbonylamino-3,4-dihydro-2,2-dimethyl-3-hydroxy-2-H-1-benzopyran-6-carbonitrile**

Following the procedure described in example 13, but using cyclopentanecarboxylic acid instead of N-*tert*-butoxycarbonylhomoproline, the title compound of this example was obtained as a white solid. (yield: 84%).
M.p.: 176.4-177.9 °C;
IR (KBr) $\nu$: 3469, 3239, 2939, 2228, 1730, 1627, 1607, 1546, 1485, 1365, 1395, 1241 $cm^{-1}$;
$^1H$ NMR (80 MHz, DMSO) $\delta$ (TMS): 8.23 (m, 1H), 7.40 (m, 2H), 6.91 (d, J = 9Hz, 1H), 4.79 (m, 1H), 3.6 (m, 2H + $H_2O$), 2.4-1.0 (m, 9H), 1.41 (s, 3H), 1.17 (s, 3H).
Analysis Calcd. for $C_{18}H_{22}N_2O_3$ . 1.5 $H_2O$: C 63.71%; H 7.37%; N 8.26%. Found: C 63.94%; H 6.97%; N 8.08%.

## EXAMPLE 45

***Trans* 4-cyclohexylcarbonylamino-3,4-dihydro-2,2-dimethyl-3-hydroxy-2H-1-benzopyran-6-carbonitrile**

Following the procedure described in example 13, but using cyclohexanecarboxylic acid instead of N-*tert*-butoxycarbonylhomoproline, the title compound of this example was obtained as a white solid (yield: 42%).
M.p.: 208.1-210.3 ° C;
IR (KBr) ν: 3457, 3229, 2929, 2229, 1625, 1606, 1485, 1443, 1364, 1306, 1270, 1123 $cm^{-1}$;
$^1H$ NMR (80 MHz, $CDCl_3$) δ (TMS): 7.47 (m, 2H), 6.89 (d, J= 10Hz, 1H), 5.68 (m, 1H), 5.05 (t, J= 10Hz, 1H ), 2.4-1.0 (complex signal, 13H), 1.50 (s, 3H), 1.29 (s, 3H).
Analysis Calcd. for $C_{19}H_{24}N_2O_3$ . 1 $H_2O$: C 65.89%; H 7.51%; N 8.09%. Found: C 65.85%; H 7.09%; N 7.83%.

**EXAMPLE 46**

***Trans* 3,4-dihydro-2,2-dimethyl-3-hydroxy-4-[(2S)-N-methyl-1-*tert*-butoxycarbonyl-2-pyrrolidinylcarbonylamino]-2H-1-benzopyran-6-carbonitrile**

Following the procedure described in example 13, but using 2,2-dimethyl-3-hydroxy-4-methylamino-2H-1-benzopyran-6-carbonitrile instead of 4-amino-2,2-dimethyl-3-hydroxy-2H-1-benzopyran-6-carbonitrile and N-*tert*-butoxycarbonyl-L-proline instead of N-*tert*-butoxycarbonyl homoproline, and after chromatography of the resulting residue, two fractions corresponding to two stereoisomers of the title compound of this example were obtained as white solids.
1-Isomer A (yield: 10%).
M.p.: 101.3-106.5 ° C;
IR (KBr) ν: 3600-3200, 2929, 2222, 1745, 1684, 1481, 1396, 1363, 1259, 1156, 1032 $cm^{-1}$;
$^1H$ NMR (80 MHz, $CDCl_3$) δ (TMS): 7.91 (m, 1H), 7.45 (d, J= 10Hz, 1H), 6.86 (d, J= 10Hz, 1H), 5.27 (d, J = 9Hz, 1H), 4.36 (m, 1H), 3.87 (d, J =9 Hz, 1H), 3.48 (m, 2H), 2.4-1.5 (m, 8H), 1.47 (s, 12H), 1.29 (s, 3H).
Analysis Calcd. for $C_{23}H_{31}N_3O_5$: C 64.32%; H 7.27%; N 9.78%. Found: C 64.64%; H 7.76%; N 9.39%.
2-Isomer B (yield: 25%).
M.p.: 49.7-55.8 ° C;
IR (KBr) ν: 3600-3200, 2972, 2927, 2223, 1680, 1606, 1483, 1402, 1263, 1162 $cm^{-1}$;
$^1H$ NMR (80 MHz, $CDCl_3$) δ (TMS): 7.43 (d, J= 10Hz, 1H), 7.26 (s, 1H), 6.89 (d, J= 10Hz, 1H), 5.89 (d, J= 9.6Hz, 1H), 4.63 (m, 1H), 3.77 (d, J= 9.6Hz, 1H), 3.48 (m, 2H), 2,87 (s, 3H), 2.3-1.7 (m, 5H), 1.55 (s, 3H), 1.45 (s, 9H), 1.29 (s, 3H).
Analysis Calcd. for $C_{23}H_{31}N_3O_5$: C 64.32%; H 7.27%; N 9.78%. Found: C 64.64%; H 7.76%; N 9.39%.

**EXAMPLE 47**

***Trans* 3,4-dihydro-2,2-dimethyl-3-hydroxy-4-[(2S)-N-methyl-(2-pyrrolidinylcarbonyl)amino]-2H-1-benzopyran-6-carbonitrile** Following the procedure described in example 14, but starting from isomer B of the product obtained in example 46, the title compound of this example was obtained as a white solid (yield: 97%).
M.p.: 91.2-101.0 ° C;
IR (KBr) ν: 3600-3200, 2969, 2929, 2222, 1632, 1483, 1392, 1263, 1120, 1076 $cm^{-1}$;
$^1H$ NMR (80 MHz, $CDCl_3$) δ (TMS): 7.45 (d of d, J= 10Hz, J= 2Hz, 1H), 7.14 (s, 1H), 6.89 (d, J= 10Hz, 1H), 5.83 (d, J= 10.5Hz, 1H), 4.17 (m, 1H), 3.77 (d, J= 10.5Hz, 1H), 3.6-2.8 (m, 4H), 2,80 (s, 3H), 2.3-1.7 (m, 4H), 1.53 (s, 3H), 1.28 (s, 3H).

**EXAMPLE 48**

***Trans* 3,4-dihydro-2,2-dimethyl-3-hydroxy-4-[(2S)-N-methyl-1-benzyloxycarbonyl-2-pyrrolidinylcarbonylamino]-2H-1-benzopyran-6-carbonitrile**

Following the procedure described in example 30, but starting from the product obtained in example 47 and using benzyl chloroformiate instead of methyl chloroformiate, the title compound of this example was obtained as a white solid (yield: 76%).
M.p.: 93.0-108.2 ° C;
IR (KBr) ν: 3600-3200, 2971, 2875, 2222, 1691, 1636, 1483, 1414, 1354, 1262, 1119 $cm^{-1}$;
$^1H$ NMR (80 MHz, $CDCl_3$) δ (TMS): 7.34 (m, 7H), 6.89 (d, J= 10Hz, 1H), 5.92 (d, J= 10.5Hz, 1H), 5.16 (AB system, δν = 0.16, J= 12Hz, 2H), 4.69 (m, 1H), 3.80 (d, J= 10.5Hz, 1H), 3.48 (m, 2H), 2.89 (s, 3H), 2.6-1.8

(m, 5H), 1.57 (s, 3H), 1.31 (s,3H).
Analysis Calcd. for $C_{25}H_{29}N_3O_5$ . 0.5 $H_2O$: C 65.22%; H 6.52%; N 9.13%. Found: C 65.47%; H 6.51%; N 9.31%.

**EXAMPLE 49**

***Trans* 3-acetoxy-3,4-dihydro-2,2-dimethyl-4-[(2S)-1-*tert*-butoxycarbonyl-2-pyrrolidinylcarbonylamino]-2H-1-benzopyran-6-carbonitrile**

To a solution of 0.5 g (1.2 mmol) of the product obtained in example 28 in 4 mL of pyridine, were added 2 mL of acetic anhydride and the mixture was stirred at room temperature for 3 days. The solvent was removed and the residue was redissolved in $CH_2Cl_2$ and washed with $H_2O$. The organic layer was dried over $MgSO_4$. The solvent was removed and the resulting residue was chromatographed on silica gel, eluting with hexane-ethyl acetate mixtures of increasing polarity, to give 0.42 g of a white solid (yield: 76%).
M.p.: 183.6-194.1 ° C;
IR (KBr) $\nu$: 3298, 2966, 2927, 2222, 1746, 1691, 1649, 1518, 1379, 1259, 1235, 1218, 1133 1059 $cm^{-1}$;
$^1H$ NMR (80 MHz, $CDCl_3$) $\delta$ (TMS): 7.49 (m, 2H), 7.1-6.5 (broad s., 1H), 6.86 (d, J= 10Hz, 1H), 5.20 (m, 2H), 4.30 (m, 1H), 3.45 (m, 2H), 2,11 (s, 3H), 2.4-1.7 (m, 4H), 1.46 (s, 9H), 1.40 (s, 3H), 1.34 (s, 3H).
Analysis Calcd. for $C_{24}H_{31}N_3O_6$ . 0.5 $H_2O$: C 61.80%; H 6.87%; N 9.01%. Found: C 61.98%; H 7.04%; N 8.82%.

**EXAMPLE 50**

***Trans* 3-acetoxy-3,4-dihydro-2,2-dimethyl-4-(1-*tert*-butoxycarbonyl-2-piperidinylcarbonylamino)-2H-1-benzopyran-6-carbonitrile**

Following the procedure described in example 49, but starting from the product obtained in example 13, the title compound of this example was obtained as a white solid (yield: 85%).
M.p.: 71.7-82.7 ° C;
IR (KBr) $\nu$: 3307, 2975, 2932, 1745, 1679, 1485, 1365, 1262, 1219, 1159, 1045 $cm^{-1}$;
$^1H$ NMR (80 MHz, $CDCl_3$) $\delta$ (TMS): 7.45 (m, 2H), 6.86 (d, J= 10Hz, 1H), 6.39 (m, 1H), 5.25 (m, 2H), 4.72 (m, 1H), 4.07 (m, 1H), 3.0-1.4 (m, 7H), 2,11 (s, 3H), 1.47 (s, 9H), 1.41 (s, 3H), 1.34 (s, 3H).
Analysis Calcd. for $C_{25}H_{33}N_3O_6$ . 1.5 $H_2O$: C 60.79%; H 7.19%; N 8.51%. Found: C 60.85%; H 7.19%; N 8.15%.

**EXAMPLE 51**

**2,2-dimethyl-4-(1-*tert*-butoxycarbonyl-2-piperidinylcarbonylamino)-2H-1-benzopyran-6-carbonitrile**

To a solution of 1 g (2.12 mmol) of the product obtained in example 50 in 15 mL of toluene, were added 0.38 mL (2.55 mmol) of 1,8-diazabicyclo(5.4.0)undec-7-ene and the mixture was stirred at reflux for 3 h. The solvent was removed and the residue was redissolved in ethyl acetate and washed with water. The organic phase was dried over $MgSO_4$. The solvent was removed and the resulting residue was chromatographed on silica gel, eluting with hexane-ethyl acetate mixtures of increasing polarity, to give 0.65 g of the starting material (yield: 65%) and 0.26 g of the title compound of this example as a white solid (yield: 29%).
M.p.: 187.4 ° C;
IR (KBr) $\nu$: 3363, 2968, 2228, 1696, 1677, 1530, 1483, 1376, 1361, 1281, 1190, 1161, 1124 $cm^{-1}$;
$^1H$ NMR (80 MHz, $CDCl_3$) $\delta$ (TMS): 8.10 (m, 1H), 7.45 (m, 2H), 6.86 (d, J= 10Hz, 1H), 6.51 (s, 1H), 4.82 (m, 1H), 4.07 (m, 1H), 2.86 (t of d, J= 13Hz, J= 3Hz, 1H), 2,24 (m, 2H), 1.55 (m, 19H).
Analysis Calcd. for $C_{23}H_{29}N_3O_4$ . 0.5 $H_2O$: C 65.71%; H 7.14%; N 10.00%. Found: C 65.43%; H 7.06%; N 9.61%.

**EXAMPLE 52**

**2,2-dimethyl-4-[(2S)-1-*tert*-butoxycarbonyl-2-pyrrolidinylcarbonylamino]-2H-1-benzopyran-6-carbonitrile**

Following the procedure described in example 51, but starting from the product obtained in example 49,

the title compound of this example was obtained as a white solid (yield: 63%).
M.p.: 57.7-80.0 °C;
IR (KBr) ν: 3290, 3063, 2971, 2925, 2223, 1695, 1539, 1483, 1399, 1362, 1162, 1129 $cm^{-1}$;
$^1H$ NMR (80 MHz, $CDCl_3$) δ (TMS): 9.30 (m, 1H), 7.60 (broad s, 1H), 7,43 (d of d, J = 10Hz, J = 2Hz, 1H), 6.84 (d, J = 10Hz, 1H), 6.54 (s, 1H), 4.47 (m, 1H), 3.43 (m, 2H), 2,59 (m, 1H), 1.93 (m, 3H), 1.56 (s, 9H), 1.48 (s, 6H).
Analysis Calcd. for $C_{22}H_{27}N_3O_4$: C 66.48%; H 6.85%; N 10.57%. Found: C 66.40%; H 7.07%; N 10.17%.

**EXAMPLE 53**

**2,2-dimethyl-4-[(2S)-2-pyrrolidinylcarbonylamino]-2H-1-benzopyran-6-carbonitrile**

Following the procedure described in example 14, but starting from the product obtained in example 52, the title compound of this example was obtained as a white solid (yield: 75%).
M.p.: 87.7-89.6 °C;
IR (KBr) ν: 3320, 3239, 2969, 2931, 2867, 2223, 1671, 1518, 1478, 1360, 1275, 1210, 1195, 1170, 1134 $cm^{-1}$;
$^1H$ NMR (80 MHz, $CDCl_3$) δ (TMS): 9.72 (m, 1H), 7.39 (m, 2H), 6.87 (d, J = 10Hz, 1H), 6.55 (s, 1H), 3.92 (d of d, J = 8Hz, J = 5.5Hz, 1H), 3.20 (m, 2H), 2.0 (m, 5H), 1.49 (s, 6H).

**EXAMPLE 54**

**2,2-dimethyl-4-[(2S)-1-benzyloxycarbonyl-2-pyrrolidinylcarbonylamino]-2H-1-benzopyran-6-carbonitrile**

Following the procedure described in example 30, but starting from the product obtained in example 53 and using benzyl chloroformiate instead of methyl chloroformiate, the title compound of this example was obtained as a white solid (yield: 79%).
M.p.: 138.5-141.1 °C;
IR (KBr) ν: 3284, 2971, 2221, 1695, 1670, 1536, 1481, 1412, 1353, 1278, 1211, 1192, 1167, 1129 $cm^{-1}$;
$^1H$ NMR (80 MHz, $CDCl_3$) δ (TMS): 9.10 (m, 1H), 7.35 (m, 7H), 6.85 (d, J = 10Hz, 1H), 6.43 (s, 1H), 5.27 (m, 2H), 4.52 (m, 1H), 3.55 (m, 2H), 2,59 (m, 1H), 1.97 (m, 3H), 1.48 (s, 6H).
Analysis Calcd. for $C_{25}H_{25}N_3O_4$: C 69.59%; H 5.84%; N 9.74%. Found: C 69.43%; H 6.01%; N 9.37%.

**EXAMPLE 55**

***Trans* 3,4-dihydro-2,2-dimethyl-3-hydroxy-4-[(2S)-1-[(3-pyridyl)-methyloxycarbonyl]-2-pyrrolidinylcarbonylamino)-2H-1-benzopyran-6-carbonitrile**

Following the procedure described in example 13, but using N-(3-pyridyl)methyloxycarbonyl-L-proline (reference example 7) instead of N-*tert*-butoxycarbonylhomoproline, the title compound of this example was obtained as a white solid (yield: 28%).
M.p.: 193.8-195.7 °C;
IR (KBr) ν: 3600-3200, 2985, 2968, 2928, 2217, 1677, 1546, 1484, 1414, 1354, 1275, 1126 $cm^{-1}$;
$^1H$ NMR (80 MHz, $CDCl_3$) δ (TMS): 8.68 (m, 2H), 7.85 (m, 1H), 7.39 (m, 3H), 7.00 (broad s., 1H), 6.85 (d, J = 8Hz, 1H), 5.26 (s, 0.5 2H), 5.21 (s, 0.5 2H), 5.04(t, J = 8Hz, 1H), 4.45 (m, 1H), 3.57 (m, 3H), 3.20 (broad s., 1H), 2.5-1.8 (m, 4H), 1.50 (s, 3H), 1.27 (s, 3H).
Analysis Calcd. for $C_{24}H_{26}N_4O_5$: C 63.99%; H 5.82%; N 12.44%. Found: C 64.42%; H 6.14%; N 12.05%.

**EXAMPLE 56**

***Trans* 3,4-dihydro-2,2-dimethyl-3-hydroxy-4-[(2S)-1-[(2-pyridyl)-methyloxycarbonyl]-2-pyrrolidinylcarbonylamino)-2H-1-benzopyran-6-carbonitrile**

Following the procedure described in example 13, but using N-(2-pyridyl)methyloxycarbonyl-L-proline (reference example 8) instead of N-*tert*-butoxycarbonylhomoproline, the title compound of this example was obtained as a white solid (yield: 9%).
M.p.: 68.2-77.2 °C;

IR (KBr) ν: 3600-3200, 2971, 2929, 2221, 1694, 1658, 1605, 1483, 1437, 1410, 1353, 1263, 1125 $cm^{-1}$;
$^1$H NMR (80 MHz, $CDCl_3$) δ (TMS): 8.35 (m, 1H), 7.71 (m, 1H), 7.39 (m, 5H), 6.85 (d, J = 8Hz, 1H), 5.21 (m, 3H), 4.48 (t, J = 8Hz, 1H), 3.56 (m, 3H), 2.90 (broad s., 1H), 2.5-1.5 (m, 4H), 1.50 (s, 3H), 1.27 (s, 3H).
Analysis Calcd. for $C_{24}H_{26}N_4O_5$ . 0.5 $H_2O$: C 62.68%; H 5.88%; N 12.19%. Found: C 62.76%; H 6.15%; N 11.83%.

**EXAMPLE 57**

***Trans* 3,4-dihydro-2,2-dimethyl-3-hydroxy-4-[(2S)-1-[(4-pyridyl)-methyloxycarbonyl]-2-pyrrolidinylcarbonylamino)-2H-1-benzopyran-6-carbonitrile**

Following the procedure described in example 13, but using N-(4-pyridyl)methyloxycarbonyl-L-proline (reference example 9) instead of N-*tert*-butoxycarbonylhomoproline, the title compound of this example was obtained as a white solid (yield: 5%).
M.p.: 55.7-67.4 ° C;
IR (KBr) ν: 3600-3200, 2971, 2931, 2221, 1694, 1658, 1605, 1485, 1437, 1410, 1353, 1265, 1125 $cm^{-1}$;
$^1$H NMR (80 MHz, $CDCl_3$) δ (TMS): 8.65 (m, 2H), 7.39 (m, 5H), 6.85 (d, J = 8Hz, 1H), 5.20-4.80 (m, 4H), 4.40 (m, 1H), 4.0-3.4 (m, 3H), 2.5-1.5 (m, 4H), 1.48 (s, 3H), 1.25 (s, 3H).
Analysis Calcd. for $C_{24}H_{26}N_4O_5$ . 1.5 $H_2O$: C 60.31%; H 6.07%; N 11.73%. Found: C 60.26%; H 5.68%; N 11.53%.

**EXAMPLE 58**

***Trans* 3,4-dihydro-2,2-dimethyl-3-hydroxy-4-[(2S)-1-[(3-methoxyphenyl)methyloxycarbonyl]-2-pyrrolidinylcarbonylamino)-2H-1-benzopyran-6-carbonitrile**

Following the procedure described in example 13, but using N-(3-methoxyphenyl)methyloxycarbonyl-L-proline (reference example 10) instead of N-*tert*-butoxycarbonylhomoproline, the title compound of this example was obtained as a white solid (yield: 72%).
M.p.: 195-197 ° C;
IR (KBr) ν: 3600-3200, 2971, 2931, 2221, 1670, 1484, 1437, 1411, 1352, 1265, 1124 $cm^{-1}$;
$^1$H NMR (80 MHz, $CDCl_3$) δ (TMS): 7.5-6.8 (complex signal, 8H), 5.13 (m, 3H), 4.4 (m, 1H), 3.80 (s, 3H), 3.56 (m, 3H), 2.90 (broad s., 1H), 2.5-1.5 (m, 4H), 1.49 (s, 3H), 1.27 (s, 3H).
Analysis Calcd. for $C_{26}H_{29}N_3O_6$: C 65.12%; H 6.10%; N 8.76%. Found: C 64.89%; H 6.19%; N 8.28%.

**EXAMPLE 59**

***Trans* 3,4-dihydro-2,2-dimethyl-3-hydroxy-4-[(2S)-1-[(4-methoxyphenyl)methyloxycarbonyl]-2-pyrrolidinylcarbonylamino)-2H-1-benzopyran-6-carbonitrile**

Following the procedure described in example 13, but using N-(4-methoxyphenyl)methyloxycarbonyl-L-proline (reference example 11) instead of N-*tert*-butoxycarbonylhomoproline, the title compound of this example was obtained as a white solid (yield: 44%).
M.p.: 175.1-178 ° C;
IR (KBr) ν: 3434, 3325, 2968, 2225, 1691, 1674, 1539, 1482, 1359, 1265, 1120 $cm^{-1}$;
$^1$H NMR (80 MHz, $CDCl_3$) δ (TMS): 7.40 (m, 4H), 6.85 (m, 4H), 5.28 (s, 0.5 2H), 5.13 (s, 0.5 2H), 4.98 (t, J = 9Hz, 1H), 4.42 (m, 1H), 3.78 (s, 3H), 3.52 (m, 3H), 2.90 (broad s., 1H), 2.4-1.8 (m, 4H), 1.54 (s, 3H), 1.31 (s, 3H).
Analysis Calcd. for $C_{26}H_{29}N_3O_6$: C 65.12%; H 6.10%; N 8.76%. Found: C 65.43%; H 6.22%; N 8.76%.

**EXAMPLE 60**

***Trans* 3,4-dihydro-2,2-dimethyl-3-hydroxy-4-[(2S)-1-[(3-chlorophenyl)methyloxycarbonyl]-2-pyrrolidinylcarbonylamino)-2H-1-benzopyran-6-carbonitrile**

Following the procedure described in example 13, but using N-(3-chlorophenyl)methyloxycarbonyl-L-proline (reference example 12) instead of N-*tert*-butoxycarbonylhomoproline, the title compound of this example was obtained as a white solid (yield: 38%).

M.p.: 72.8-80.6 ° C;
IR (KBr) ν: 3600-3200, 2973, 2929, 1671, 1605, 1484, 1409, 1352, 1264, 1125 $cm^{-1}$;
$^1$H NMR (80 MHz, $CDCl_3$) δ (TMS): 7.40 (m, 6H), 7.0 (broad s., 1H), 6.85 (d, J = 8Hz, 1H), 5.17 (s, 0.5 2H), 5.14 (s, 0.5 2H), 5.03 (m, 1H), 4.48 (t, J = 8Hz, 1H), 3.55 (m, 3H), 2.90 (broad s., 1H), 2.5-1.5 (m, 4H), 1.49 (s, 3H), 1.27 (s, 3H).
Analysis Calcd. for $C_{25}H_{26}ClN_3O_5$: C 62.05%; H 5.42%; N 8.68%. Found: C 62.44%; H 5.63%; N 8.20%.

**EXAMPLE 61**

***Trans* 3,4-dihydro-2,2-dimethyl-3-hydroxy-4-[(2S)-1-[(2-phenyl)ethyloxycarbonyl]-2-pyrrolidinylcarbonylamino]-2H-1-benzopyran-6-carbonitrile**

Following the procedure described in example 13, but using N-(2-phenyl)ethyloxycarbonyl-L-proline (reference example 13) instead of N-*tert*-butoxycarbonylhomoproline, the title compound of this example was obtained as a white solid (yield: 75%).
M.p.: 81.0-94.7 ° C;
IR (KBr) ν: 3600-3200, 2971, 2925, 2221, 1665, 1484, 1415, 1365, 1264, 1194, 1125 $cm^{-1}$;
$^1$H NMR (80 MHz, $CDCl_3$) δ (TMS): 7.4 (m, 7H), 6.85 (d, J = 8Hz, 1H), 4.96 (m, 1H), 3.34 (t, J = 6Hz, 2H), 4.5-3.6 (m, 6H), 2.95 (t, J = 6Hz, 2H), 2.5-1.5 (m, 4H), 1.48 (s, 3H), 1.25 (s, 3H).
Analysis Calcd. for $C_{26}H_{29}N_3O_5$ . 0.5 $H_2O$: C 66.10%; H 6.36%; N 8.90%. Found: C 66.05%; H 6.48%; N 8.53%.

**EXAMPLE 62**

***Trans* 3,4-dihydro-2,2-dimethyl-3-hydroxy-4-[1-[(3-fluorophenyl)methyloxycarbonyl]-2-piperidinylcarbonylamino]-2H-1-benzopyran-6-carbonitrile**

Following the procedure described in example 15, but using 3-fluorophenylmethyl chloroformiate (prepared in situ by reacting 3-fluorobenzylic alcohol with bistrichloromethyl carbonate) instead of isopropyl chloroformiate, the title compound of this example was obtained as a white solid (yield: 5%).
M.p.: 152.2-164.8 ° C;
IR (KBr) ν: 3600-3200, 2936, 2859, 2221, 1695, 1643, 1537, 1483, 1415, 1266, 1255, 1123, 1069 $cm^{-1}$;
$^1$H NMR (80 MHz, $CDCl_3$) δ (TMS): 7.6-6.2 (m, 8H), 5.16 (s, 2H), 5.0 (m, 2H), 4.15 (m, 1H), 3.63 (m, 2H), 3.10 (m, 1H), 2.30 (m, 1H), 1.71 (m, 5H), 1.50 (s, 3H), 1.28 (s, 3H).
Analysis Calcd. for $C_{26}H_{28}FN_3O_5$: C 64.85%; H 5.86%; N 8.73%. Found: C 64.98%; H 6.02%; N 8.29%.

**EXAMPLE 63**

***Trans* 6-acetamido-3,4-dihydro-2,2-dimethyl-4-[(2S)-1-benzyloxycarbonyl-2-pyrrolidinylcarbonylamino]-7-nitro-2H-1-benzopyran-3-ol**

Following the procedure described in example 13, but using N-benzyloxycarbonyl-L-proline instead of N-*tert*-butoxycarbonylhomoproline and starting from 6-acetamido-4-amino-3,4-dihydro-2,2-dimethyl-7-nitro-2H-1-benzopyran-3-ol, the title compound of this example was obtained as a red solid (yield: 59%).
M.p.: 147.9-162.9 ° C;
IR (KBr) ν: 3600-3200, 2923, 1695, 1680, 1656, 1496, 1414, 1352, 1337, 1288, 1271, 1131 $cm^{-1}$;
$^1$H NMR (80 MHz, $CDCl_3$) δ (TMS): 9.88 (m, 1H), 8.52 (s, 1H), 7.85 (s, 1H), 7.31 (s, 5H), 6.90 (m, 1H), 5.10 (s, 2H), 4.98 (m, 1H), 4.38 (m, 1H), 3.56 (m, 3H), 3.09 (m, 1H), 2.5-1.8 (m, 4H), 2.18 (d, J = 3Hz, 3H), 1.47 (s, 3H), 1.26 (s, 3H).
Analysis Calcd. for $C_{26}H_{30}N_4O_8$ . 0.2 Hexane: C 60.09%; H 6.04%; N 10.30%. Found: C 60.42%; H 6.38%; N 9.91%.

**EXAMPLE 64**

***Trans* 6-amino-3,4-dihydro-2,2-dimethyl-4-[(2S)-1-benzyloxycarbonyl-2-pyrrolidinylcarbonylamino]-7-nitro-2H-1-benzopyran-3-ol**

Following the procedure described in example 13, but using N-benzyloxycarbonyl-L-proline instead of

N-*tert*-butoxycarbonylhomoproline and starting from 6-amino-4-amino-3,4-dihydro-2,2-dimethyl-7-nitro-2H-1-benzopyran-3-ol, the title compound of this example was obtained as a red solid (yield: 67%).
M.p.: 91.7-96.6 ° C;
IR (KBr) $\nu$: 3600-3200, 2969, 2925, 2665, 2492, 2416, 1247, 1135 $cm^{-1}$;
$^1H$ NMR (80 MHz, $CDCl_3$) $\delta$ (TMS): 7.33 (m, 7H), 6.70 (m, 2H), 5.15 (s, 2H), 4.97 (m, 1H), 4.43 (m, 3H), 3.57 (m, 3H), 2.4-1.8 (m, 4H), 1.45 (s, 3H), 1.21 (s, 3H).
Analysis Calcd. for $C_{24}H_{28}N_4O_7$ . 0.5 $H_2O$: C 58.42%; H 5.88%; N 11.36%. Found: C 58.70%; H 6.14%; N 11.00%.

## EXAMPLE 65

### *Trans*3,4-dihyro-2,2-dimethyl-4-[(2S)-1-benzyloxycarbonyl-2-pyrrolidinylcarbonylamino]-2H-1-benzopyran-3-ol

Following the procedure described in example 13, but using N-benzyloxycarbonyl-L-proline instead of N-*tert*-butoxycarbonylhomoproline and starting from 4-amino-3,4-dihydro-2,2-dimethyl-2H-1-benzopyran-3-ol, the title compound of this example was obtained as a white solid (yield: 78%).
M.p.: 64.2-68.1 ° C;
IR (KBr) $\nu$: 3600, 3200, 2969, 2925, 2871, 1679, 1480, 1448, 1415, 1354, 1247, 1122 $cm^{-1}$;
$^1H$ NMR (80 MHz, $CDCl_3$) $\delta$ (TMS): 7.33 (s, 5H), 7.4-6.5 (m, 5H), 5.15 (s, 2H), 5.00 (m, 1H), 4.41 (m, 1H), 3.52 (m, 4H), 2.4-1.8 (m, 4H, 1.47 (s, 3H), 1.25 (s, 3H).
Analysis Calcd. for $C_{24}H_{28}N_2O_5$: C 67.91%; H 6.65%; N 6.60%. Found: C 67.66%; H 6.91%; N 6.29%.

## Claims

**1.** A compound of formula **I**:

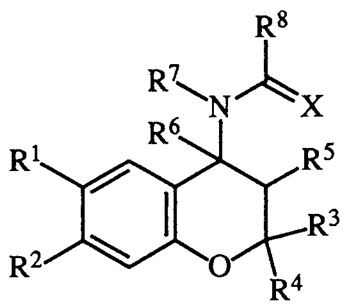

**I**

wherein:

$R^1$ and $R^2$ represent hydrogen, $C_{1-4}$ alkyl, hydroxyl, $C_{1-4}$ alkoxy, formyl, $C_{1-4}$ alkylcarbonyl, $C_{1-4}$ alkylthiocarbonyl, carboxyl, $C_{1-4}$ alkoxycarbonyl, $C_{1-4}$ alkoxythiocarbonyl, $C_{1-4}$ alkylcarbonyloxy, $C_{1-4}$ alkylthiocarbonyloxy, hydroxy-($C_{1-4}$) alkyl, mercapto-($C_{1-4}$) alkyl, perfluoro($C_{1-4}$)alkyl, nitro, amino, cyano, halogen, trifluoromethoxy, ethynyl, trimethylsilylethynyl, $C_{1-4}$ alkylsulphinyl, arylsulphinyl, $C_{1-4}$ alkylsulphonyl, arylsulphonyl, $C_{1-4}$ alkoxysulphinyl, $C_{1-4}$ alkoxysulphonyl, $C_{1-4}$ alkylcarbonylamino, $C_{1-4}$ alkoxycarbonylamino, aminosulphinyl, aminosulphonyl, aminocarbonyl, aminothiocarbonyl, $C_{1-4}$ alkylsulphinylamino, $C_{1-4}$ alkylsulphonylamino, $C_{1-4}$ alkoxysulphinylamino, $C_{1-4}$ alkoxysulphonylamino, ($C_{1-4}$ alkyl)carbonyl($C_{1-4}$ alkyl), nitro-($C_{1-4}$ alkyl), cyano-($C_{1-4}$ alkyl), ($C_{1-4}$ alkyl)C(=NOH), ($C_{1-4}$ alkyl)C(=$NNH_2$) or ($C_{1-4}$ alkoxy)C(=NH), being the above amino groups optionally substituted by one or two $C_{1-4}$ alkyl groups;

$R^3$ is hydrogen or $C_{1-4}$ alkyl; $R^4$ is $C_{1-4}$ alkyl, or $R^3$ and $R^4$ form a $C_{2-5}$ polymethylene chain;

$R^5$ is OH or $-OCOCH_3$ and $R^6$ is hydrogen, or $R^5$ and $R^6$ together form a bond;

$R^7$ is hydrogen or $C_{1-6}$ alkyl;

$R^8$ is a totally saturated $C_5$-$C_6$ cyclic or heterocyclic group selected from cyclopentyl, cyclohexyl, pyrrolidinyl, piperidinyl, piperazinyl, imidazolinyl, pyrazolidinyl, isothiazolidinyl, isoxazolidinyl, thiazolidinyl, oxazolidinyl or morpholinyl, which may be optionally substituted by one or two $R^9$ groups;

$R^9$ is a hydroxyl, oxo, $C_{1-6}$ alkyl, $C_{1-6}$ alkylcarbonyl, $C_{1-6}$ alkoxycarbonyl, $C_{1-6}$ alkoxycarbonylmethyl $C_{1-6}$ alkylaminocarbonyl, $R^{10}$-oxycarbonyl or $R^{10}$-($C_{1-4}$)alkoxycarbonyl group;

$R^{10}$ is a 5- or 6-membered monocyclic or a 9- or 10-membered bicyclic aryl or heteroaryl group, which may be optionally substituted by one or several fluorine, chlorine, bromine or iodine atoms, or hydroxyl, $C_{1-4}$ alkyl, $C_{1-4}$ alkoxy, cyano or nitro groups;

X is an oxygen or sulphur atom;

and the salts thereof.

**2.** A compound according to Claim 1 wherein:

$R^1$ is a cyano or nitro group;

$R^2$ is hydrogen;

$R^3$ and $R^4$ are methyl; and

$R^5$, $R^6$, $R^7$, $R^8$ and X have the previously defined meaning.

**3.** A compound according to Claim 1 wherein:

$R^1$ is a cyano group;

$R^2$ is hydrogen;

$R^3$ and $R^4$ are methyl;

$R^5$ is an hydroxyl group and $R^6$ is hydrogen; and

$R^7$, $R^8$ and X have the previously defined meaning.

**4.** A compound according to Claim 1 wherein:

$R^1$ is a cyano group;

$R^2$ is hydrogen;

$R^3$ and $R^4$ are methyl;

$R^5$ is an hydroxyl group and $R^6$ is hydrogen;

$R^7$ represents hydrogen or a methyl group;

$R^8$ represents a cyclohexyl, 1-piperazinyl, 1-piperidinyl, or 1-pyrrolidinyl group, which may be optionally substituted by a $C_{1-6}$ alkyl group; and

X has the previously defined meaning.

**5.** A compound according to Claim 1 wherein:

$R^1$ is a cyano group;

$R^2$ is hydrogen;

$R^3$ and $R^4$ are methyl;

$R^5$ is an hydroxyl group and $R^6$ is hydrogen;

$R^7$ represents hydrogen or a methyl group;

$R^8$ represents a 2-piperidinyl or 2-pyrrolidinyl group, which may be optionally substituted by an $R^9$ group, wherein $R^9$ is a $C_{1-6}$ alkoxycarbonyl, $R^{10}$-oxycarbonyl or $R^{10}$-($C_{1-4}$)alkoxycarbonyl group, being $R^{10}$ as above defined; and

X has the previously defined meaning.

**6.**

a) *Trans* 3,4-dihydro-2,2-dimethyl-3-hydroxy-4-(1-pyrrolidinyl-thiocarbonylamino)-2H-1-benzopyran-6-carbonitrile and the salts thereof;

b) *Trans* 3,4-dihydro-2,2-dimethyl-3-hydroxy-4-(1-piperidinyl-thiocarbonylamino)-2H-1benzopyran-6-carbonitrile and the salts thereof;

c) *Trans* 3,4-dihydro-2,2-dimethyl-3-hydroxy-4-(4-methyl-1-piperazinyl-thiocarbonylamino)-2H-1-benzopyran-6-carbonitrile and the salts thereof;

d) *Trans* 3,4-dihydro-2,2-dimethyl-3-hydroxy-4-(1-*tert*-butoxycarbonyl-2-piperidinylcarbonylamino)-2H-1-benzopyran-6-carbonitrile and the salts thereof;

e) *Trans* 3,4-dihydro-2,2-dimethyl-3-hydroxy-4-(1-phenyloxycarbonyl-2-piperidinylcarbonylamino)-2H-1-benzopyran-6-carbonitrile and the salts thereof;

f) *Trans* 3,4-dihydro-2,2-dimethyl-3-hydroxy-4-(1-benzyloxycarbonyl-2-piperidinylcarbonylamino)-2H-1-benzopyran-6-carbonitrile and the salts thereof;

g) *Trans* 3,4-dihydro-2,2-dimethyl-3-hydroxy-4-[(2S)-1-*tert*-butoxycarbonyl-2-pyrrolidinylcarbonylamino]-2H-1-benzopyran-6-carbonitrile and the salts thereof;

h) *Trans* 3,4-dihydro-2,2-dimethyl-3-hydroxy-4-[(2S)-1-isobutoxycarbonyl-2-pyrrolidinylcarbonylamino]-2H-1-benzopyran-6-carbonitrile and the salts thereof;
i) *Trans* 3,4-dihydro-2,2-dimethyl-3-hydroxy-4-[(2S)-1-phenoxycarbonyl-2-pyrrolidinylcarbonylamino)-2H-1-benzopyran-6-carbonitrile and the salts thereof;
j) *Trans* 3,4-dihydro-2,2-dimethyl-3-hydroxy-4-[(2S)-1-benzyloxycarbonyl-2-pyrrolidinylcarbonylamino)-2H-1-benzopyran-6-carbonitrile and the salts thereof;
k) *Trans* 3,4-dihydro-2,2-dimethyl-3-hydroxy-4-[(2S)-1-[(3-methoxyphenyl)methyloxycarbonyl]-2-pyrrolidinylcarbonylamino]-2H-1-benzopyran-6-carbonitrile and the salts thereof;
l) *Trans* 3,4-dihydro-2,2-dimethyl-3-hydroxy-4-[(2S)-1-[(3-chlorophenyl)methyloxycarbonyl]-2-pyrrolidinylcarbonylamino]-2H-1-benzopyran-6-carbonitrile and the salts thereof;
m) *Trans* 3,4-dihydro-2,2-dimethyl-3-hydroxy-4-[(2S)-1-[(2-phenyl)ethyloxycarbonyl]-2-pyrrolidinylcarbonylamino]-2H-1-benzopyran-6-carbonitrile and the salts thereof;
n) *Trans* 4-cyclohexylcarbonylamino-3,4-dihydro-2,2-dimethyl-3-hydroxy-2H-1-benzopyran-6-carbonitrile and the salts thereof.

**7.** A process for preparing a compound according to Claim 1 which comprises:
(a) when in a compound of general formula **I**, $R^5$ is OH, $R^6$ is hydrogen, X is oxygen and $R^8$ is bound to the C=X group by a carbon atom, reacting a compound of general formula **II**, wherein $R^{1'}$ and $R^{2'}$ are $R^1$ and $R^2$ as above defined or a group or atom convertible thereto, and $R^3$, $R^4$ and $R^7$ have the previously defined meaning,

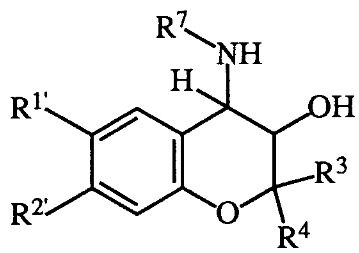

II

with an acid of general formula $R^{8a}COOH$ (wherein $R^{8a}$ means an $R^8$ group as above defined optionally substituted by one or two $R^9$ groups and which is bound to the C=X group by a carbon atom) in the presence of dicyclohexylcarbodiimide and 1-hydroxybenzotriazole in a suitable solvent such as dimethylformamide, or alternatively, reacting said compound **II** with an acid chloride of general formula $R^{8a}COCl$ (wherein $R^{8a}$ has the previously defined meaning) in the presence of a base in a suitable solvent such as chloroform, to give a compound of formula **I**, and optionally modifying its substituent $R^{8a}$ by standard chemical reactions involving the manipulation of amine protecting groups and the reaction of said amines with suitable electrophylic reagents; or
(b) when in a compound of general formula **I**, $R^5$ is OH, $R^6$ is hydrogen, X is oxygen and $R^8$ is bound to the C=X group by a nitrogen atom, reacting a compound of formula **II** with phenyl chloroformiate in a suitable solvent such as chloroform, to give a compound of general formula **XII**:

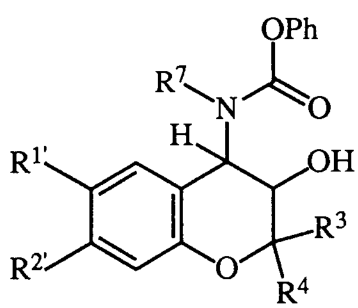

XII

wherein $R^{1'}$, $R^{2'}$, $R^3$, $R^4$ and $R^7$ have the previously defined meaning, and then reacting said compound **XII** with a compound of formula $R^{8d}$-H (**XIII**, wherein $R^{8d}$ is an $R^8$ group as above defined optionally substituted by one or two $R^9$ groups and which is bound to the C=X group by a nitrogen

atom) in a suitable solvent such as butanol or else using **XIII** itself as solvent; or

(c) when in a compound of general formula **I**, $R^5$ is OH, $R^6$ is hydrogen, X is sulphur and $R^8$ is bound to the C=X group by a nitrogen atom, reacting a compound of formula **II** with carbon sulphide in the presence of a base such as potassium hydroxide in a suitable solvent such as dimethylformamide, and subsequently treating the intermediate obtained with methyl iodide in a suitable solvent such as methanol, to give a compound of general formula **XIV**:

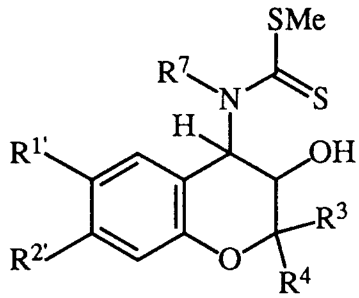

**XIV**

wherein $R^{1'}$, $R^{2'}$, $R^3$, $R^4$ and $R^7$ have the previously defined meaning, and finally reacting said compound **XIV** with a compound of general formula $R^{8d}$-H(**XIII**, wherein $R^{8d}$ has the previously defined meaning) in a suitable solvent such as butanol or else using **XIII** itself as solvent; or alternatively, in all cases wherein X is sulphur treating a compound of formula **I** wherein X is oxygen with a thiation agent such as Lawesson's reagent in a suitable solvent such as toluene;

(d) in all cases wherein $R^5$ is an acetoxy group and $R^6$ is hydrogen, reacting a compound of formula **I** wherein $R^5$ is hydroxyl and $R^6$ is hydrogen with acetic anhydride in the presence of a base such as pyridine;

(e) in all cases wherein $R^5$ and $R^6$ together form a bond, reacting a compound of general formula **I** wherein $R^5$ is OH and $R^6$ is hydrogen with a base such as sodium hydride or sodium hydroxide in a suitable solvent such as toluene or dioxane; or alternatively, reacting a compound of general formula **I** wherein $R^5$ is acetoxy and $R^6$ is hydrogen with a base such as 1,8-diazabicyclo(5.4.0)undec-7-ene (DBU) in an inert solvent such as toluene;

(f) optionally, interconverting the groups $R^1$, $R^2$, $R^{1'}$ and/or $R^{2'}$ in a compound of formula **I** or any intermediate of formula **II**, **XII** or **XIV** into other groups $R^1$ and/or $R^2$;

(g) and optionally, reacting a compound of formula **I** with an acid to give its corresponding acid addition salt.

**8.** A pharmaceutical composition comprising an effective amount of at least one compound of formula **I** or a pharmaceutically acceptable salt thereof in admixture with a pharmaceutically acceptable excipient.

**9.** The use of at least one compound of formula **I** as defined in Claim 1 for the manufacture of a medicament for the treatment and/or prevention of the diseases related with the regulation of the smooth muscle contraction at hte cardiovascular, respiratory and cerebrovascular systems, and at the gastrointestinal, urinary and uterus tracts, and particularly for the treatment and/or prevention of hypertension and asthma in mammals, including man.

**Claims for the following Contracting State : GR**

**1.** A process for preparing a compound of formula **I**:

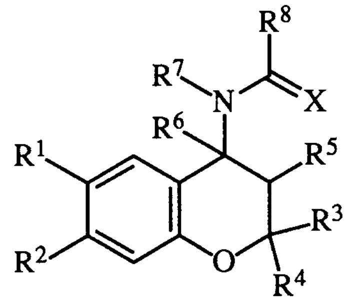

I

wherein:

$R^1$ and $R^2$ represent hydrogen, $C_{1-4}$ alkyl, hydroxyl, $C_{1-4}$ alkoxy, formyl, $C_{1-4}$ alkylcarbonyl, $C_{1-4}$ alkylthiocarbonyl, carboxyl, $C_{1-4}$ alkoxycarbonyl, $C_{1-4}$ alkoxythiocarbonyl, $C_{1-4}$ alkylcarbonyloxy, $C_{1-4}$ alkylthiocarbonyloxy, hydroxy-($C_{1-4}$) alkyl, mercapto-($C_{1-4}$) alkyl, perfluoro($C_{1-4}$)alkyl, nitro, amino, cyano, halogen, trifluoromethoxy, ethynyl, trimethylsilylethynyl, $C_{1-4}$ alkylsulphinyl, arylsulphinyl, $C_{1-4}$ alkylsulphonyl, arylsulphonyl, $C_{1-4}$ alkoxysulphinyl, $C_{1-4}$ alkoxysulphonyl, $C_{1-4}$ alkylcarbonylamino, $C_{1-4}$ alkoxycarbonylamino, aminosulphinyl, aminosulphonyl, aminocarbonyl, aminothiocarbonyl, $C_{1-4}$ alkylsulphinylamino, $C_{1-4}$ alkylsulphonylamino, $C_{1-4}$ alkoxysulphinylamino, $C_{1-4}$ alkoxysulphonylamino, ($C_{1-4}$ alkyl)carbonyl($C_{1-4}$ alkyl), nitro-($C_{1-4}$ alkyl), cyano-($C_{1-4}$ alkyl), ($C_{1-4}$ alkyl)C(=NOH), ($C_{1-4}$ alkyl)C(=$NNH_2$) or ($C_{1-4}$ alkoxy)C(=NH), being the above amino groups optionally substituted by one or two $C_{1-4}$ alkyl groups;

$R^3$ is hydrogen or $C_{1-4}$ alkyl; $R^4$ is $C_{1-4}$ alkyl, or $R^3$ and $R^4$ form a $C_{2-5}$ polymethylene chain;

$R^5$ is OH or -$OCOCH_3$ and $R^6$ is hydrogen, or $R^5$ and $R^6$ together form a bond;

$R^7$ is hydrogen or $C_{1-6}$ alkyl;

$R^8$ is a totally saturated $C_5$-$C_6$ cyclic or heterocyclic group selected from cyclopentyl, cyclohexyl, pyrrolidinyl, piperidinyl, piperazinyl, imidazolinyl, pyrazolidinyl, isothiazolidinyl, isoxazolidinyl, thiazolidinyl, oxazolidinyl or morpholinyl, which maybe optionally substituted by one or two $R^9$ groups;

$R^9$ is a hydroxyl, oxo, $C_{1-6}$ alkyl, $C_{1-6}$ alkylcarbonyl, $C_{1-6}$ alkoxycarbonyl, $C_{1-6}$ alkoxycarbonylmethyl $C_{1-6}$ alkylaminocarbonyl, $R^{10}$-oxycarbonyl or $R^{10}$-($C_{1-4}$)alkoxycarbonyl group;

$R^{10}$ is a 5- or 6-membered monocyclic or a 9- or 10-membered bicyclic aryl or heteroaryl group, which may be optionally substituted by one or several fluorine, chlorine, bromine or iodine atoms, or hydroxyl, $C_{1-4}$ alkyl, $C_{1-4}$ alkoxy, cyano or nitro groups;

X is an oxygen or sulphur atom;

and the salts thereof,

which comprises:

(a) when in a compound of general formula **I**, $R^5$ is OH, $R^6$ is hydrogen, X is oxygen and $R^8$ is bound to the C=X group by a carbon atom, reacting a compound of general formula **II**, wherein $R^{1'}$and $R^{2'}$ are $R^1$ and $R^2$ as above defined or a group or atom convertible thereto, and $R^3$, $R^4$ and $R^7$ have the previously defined meaning,

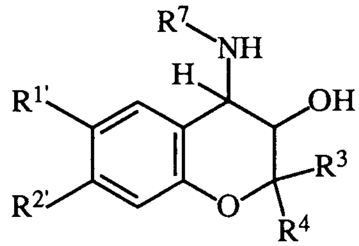

II

with an acid of general formula $R^{8a}COOH$ (wherein $R^{8a}$ means an $R^8$ group as above defined optionally substituted by one or two $R^9$ groups and which is bound to the C=X group by a carbon atom) in the presence of dicyclohexylcarbodiimide and 1-hydroxybenzotriazole in a suitable solvent

such as dimethylformamide, or alternatively, reacting said compound **II** with an acid chloride of general formula $R^{8a}COCl$ (wherein $R^{8a}$ has the previously defined meaning) in the presence of a base in a suitable solvent such as chloroform, to give a compound of formula **I**, and optionally modifying its substituent $R^{8a}$ by standard chemical reactions involving the manipulation of amine protecting groups and the reaction of said amines with suitable electrophylic reagents; or

(b) when in a compound of general formula **I**, $R^5$ is OH, $R^6$ is hydrogen, X is oxygen and $R^8$ is bound to the C=X group by a nitrogen atom, reacting a compound of formula **II** with phenyl chloroformiate in a suitable solvent such as chloroform, to give a compound of general formula **XII**:

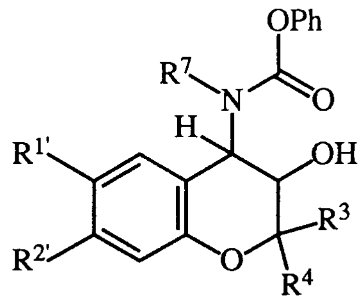

**XII**

wherein $R^{1'}$, $R^{2'}$, $R^3$, $R^4$ and $R^7$ have the previously defined meaning, and then reacting said compound **XII** with a compound of formula $R^{8d}$-H (**XIII**, wherein $R^{8d}$ is an $R^8$ group as above defined optionally substituted by one or two $R^9$ groups and which is bound to the C=X group by a nitrogen atom) in a suitable solvent such as butanol or else using **XIII** itself as solvent; or

(c) when in a compound of general formula **I**, $R^5$ is OH, $R^6$ is hydrogen, X is sulphur and $R^8$ is bound to the C=X group by a nitrogen atom, reacting a compound of formula **II** with carbon sulphide in the presence of a base such as potassium hydroxide in a suitable solvent such as dimethylformamide, and subsequently treating the intermediate obtained with methyl iodide in a suitable solvent such as methanol, to give a compound of general formula **XIV**:

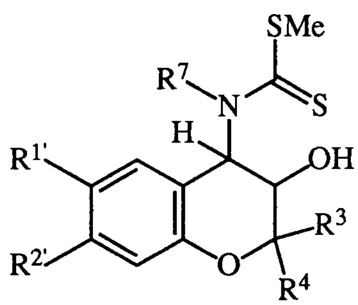

**XIV**

wherein $R^{1'}$, $R^{2'}$, $R^3$, $R^4$ and $R^7$ have the previously defined meaning, and finally reacting said compound **XIV** with a compound of general formula $R^{8d}$-H(**XIII**, wherein $R^{8d}$ has the previously defined meaning) in a suitable solvent such as butanol or else using **XIII** itself as solvent; or alternatively, in all cases wherein X is sulphur treating a compound of formula **I** wherein X is oxygen with a thiation agent such as Lawesson's reagent in a suitable solvent such as toluene;

(d) in all cases wherein $R^5$ is an acetoxy group and $R^6$ is hydrogen, reacting a compound of formula **I** wherein $R^5$ is hydroxyl and $R^6$ is hydrogen with acetic anhydride in the presence of a base such as pyridine;

(e) in all cases wherein $R^5$ and $R^6$ together form a bond, reacting a compound of general formula **I** wherein $R^5$ is OH and $R^6$ is hydrogen with a base such as sodium hydride or sodium hydroxide in a suitable solvent such as toluene or dioxane; or alternatively, reacting a compound of general formula **I** wherein $R^5$ is acetoxy and $R^6$ is hydrogen with a base such as 1,8-diazabicyclo(5.4.0)undec-7-ene (DBU) in an inert solvent such as toluene;

(f) optionally, interconverting the groups $R^1$, $R^2$, $R^{1'}$ and/or $R^{2'}$ in a compound of formula **I** or any intermediate of formula **II**, **XII** or **XIV** into other groups $R^1$ and/or $R^2$;

(g) and optionally, reacting a compound of formula **I** with an acid to give its corresponding acid addition salt.

**2.** A process according to Claim 1 in which the reagents and reaction conditions are so chosen as to prepare a compound of formula **I** wherein:
$R^1$ is a cyano or nitro group;
$R^2$ is hydrogen;
$R^3$ and $R^4$ are methyl; and
$R^5$, $R^6$, $R^7$, $R^8$ and X have the previously defined meaning.

**3.** A process according to Claim 1 in which the reagents and reaction conditions are so chosen as to prepare a compound of formula **I** wherein:
$R^1$ is a cyano group;
$R^2$ is hydrogen;
$R^3$ and $R^4$ are methyl;
$R^5$ is an hydroxyl group and $R^6$ is hydrogen; and
$R^7$, $R^8$ and X have the previously defined meaning.

**4.** A process according to Claim 1 in which the reagents and reaction conditions are so chosen as to prepare a compound of formula **I** wherein:
$R^1$ is a cyano group;
$R^2$ is hydrogen;
$R^3$ and $R^4$ are methyl;
$R^5$ is an hydroxyl group and $R^6$ is hydrogen;
$R^7$ represents hydrogen or a methyl group;
$R^8$ represents a cyclohexyl, 1-piperazinyl, 1-piperidinyl, or 1-pyrrolidinyl group, which may be optionally substituted by a $C_{1-6}$ alkyl group; and
X has the previously defined meaning.

**5.** A process according to Claim 1 in which the reagents and reaction conditions are so chosen as to prepare a compound of formula **I** wherein:
$R^1$ is a cyano group;
$R^2$ is hydrogen;
$R^3$ and $R^4$ are methyl;
$R^5$ is an hydroxyl group and $R^6$ is hydrogen;
$R^7$ represents hydrogen or a methyl group;
$R^8$ represents a 2-piperidinyl or 2-pyrrolidinyl group, which may be optionally substituted by an $R^9$ group, wherein $R^9$ is a $C_{1-6}$ alkoxycarbonyl, $R^{10}$-oxycarbonyl or $R^{10}$-($C_{1-4}$)alkoxycarbonyl group, being $R^{10}$ as above defined; and
X has the previously defined meaning.

**6.**

a) A process according to Claim 1 in which the reagents and reaction conditions are so chosen as to prepare *Trans* 3,4-dihydro-2,2-dimethyl-3-hydroxy-4-(1-pyrrolidinylthiocarbonylamino)-2H-1-benzopyran-6-carbonitrile and the salts thereof;
b) A process according to Claim 1 in which the reagents and reaction conditions are so chosen as to prepare *Trans* 3,4-dihydro-2,2-dimethyl-3-hydroxy-4-(1-piperidinyl-thiocarbonylamino)-2H-1-benzopyran-6-carbonitrile and the salts thereof;
c) A process according to Claim 1 in which the reagents and reaction conditions are so chosen as to prepare *Trans* 3,4-dihydro-2,2-dimethyl-3-hydroxy-4-(4-methyl-1-piperazinyl-thiocarbonylamino)-2H-1-benzopyran-6-carbonitrile and the salts thereof;
d) A process according to Claim 1 in which the reagents and reaction conditions are so chosen as to prepare *Trans* 3,4-dihydro-2,2-dimethyl-3-hydroxy-4-(1-*tert*-butoxycarbonyl-2-piperidinylcarbonylamino)-2H-1-benzopyran-6-carbonitrile and the salts thereof;
e) A process according to Claim 1 in which the reagents and reaction conditions are so chosen as to prepare *Trans* 3,4-dihydro-2,2-dimethyl-3-hydroxy-4-(1-phenyloxycarbonyl-2-piperidinylcarbonylamino)-2H-1-benzopyran-6-carbonitrile and the salts thereof;
f) A process according to Claim 1 in which the reagents and reaction conditions are so chosen as to

prepare *Trans* 3,4-dihydro-2,2-dimethyl-3-hydroxy-4-(1-benzyloxycarbonyl-2-piperidinylcarbonylamino)-2H-1-benzopyran-6-carbonitrile and the salts thereof;

g) A process according to Claim 1 in which the reagents and reaction conditions are so chosen as to prepare *Trans* 3,4-dihydro-2,2-dimethyl-3-hydroxy-4-[(2S)-1-*tert*-butoxycarbonyl-2-pyrrolidinylcarbonylamino]-2H-1-benzopyran-6-carbonitrile and the salts thereof;

h) A process according to Claim 1 in which the reagents and reaction conditions are so chosen as to prepare *Trans* 3,4-dihydro-2,2-dimethyl-3-hydroxy-4-[(2S)-1-isobutoxycarbonyl-2-pyrrolidinylcarbonylamino]-2H-1-benzopyran-6-carbonitrile and the salts thereof;

i) A process according to Claim 1 in which the reagents and reaction conditions are so chosen as to prepare *Trans* 3,4-dihydro-2,2-dimethyl-3-hydroxy-4-[(2S)-1-phenoxycarbonyl-2-pyrrolidinylcarbonylamino)-2H-1-benzo pyran-6-carbonitrile and the salts thereof;

j) A process according to Claim 1 in which the reagents and reaction conditions are so chosen as to prepare *Trans* 3,4-dihydro-2,2-dimethyl-3-hydroxy-4-[(2S)-1-benzyloxycarbonyl-2-pyrrolidinylcarbonylamino)-2H-1-benzopyran-6-carbonitrile and the salts thereof;

k) A process according to Claim 1 in which the reagents and reaction conditions are so chosen as to prepare *Trans* 3,4-dihydro-2,2-dimethyl-3-hydroxy-4-[(2S)-1-[(3-methoxy-phenyl)methyloxycarbonyl]-2-pyrrolidinylcarbonylamino]-2H-1-benzopyran-6-carbonitrile and the salts thereof;

l) A process according to Claim 1 in which the reagents and reaction conditions are so chosen as to prepare *Trans* 3,4-dihydro-2,2-dimethyl-3-hydroxy-4-[(2S)-1-[(3-chloro-phenyl)methyloxycarbonyl]-2-pyrrolidinylcarbonylamino]-2H-1-benzopyran-6-carbonitrile and the salts thereof;

m) A process according to Claim 1 in which the reagents and reaction conditions are so chosen as to prepare *Trans* 3,4-dihydro-2,2-dimethyl-3-hydroxy-4-[(2S)-1-[(2-phenyl)ethyloxycarbonyl]-2-pyrrolidinylcarbonylamino]-2H-1-benzopyran-6-carbonitrile and the salts thereof;

n) A process according to Claim 1 in which the reagents and reaction conditions are so chosen as to prepare *Trans* 4-cyclohexylcarbonylamino-3,4-dihydro-2,2-dimethyl-3-hydroxy-2H-1-benzopyran-6-carbonitrile and the salts thereof.

**7.** The use of at least one compound of formula **I** as defined in Claim 1 for the manufacture of a medicament for the treatment and/or prevention of the diseases related with the regulation of the smooth muscle contraction at hte cardiovascular, respiratory and cerebrovascular systems, and at the gastrointestinal, urinary and uterus tracts, and particularly for the treatment and/or prevention of hypertension and asthma in mammals, including man.

**Claims for the following Contracting State : ES**

**1.** A process for preparing a compound of formula **I**:

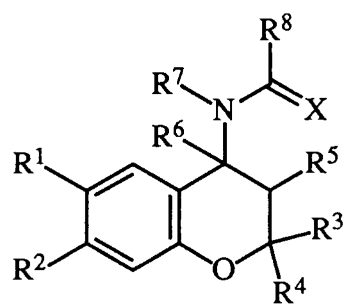

**I**

wherein:

$R^1$ and $R^2$ represent hydrogen, $C_{1-4}$ alkyl, hydroxyl, $C_{1-4}$ alkoxy, formyl, $C_{1-4}$ alkylcarbonyl, $C_{1-4}$ alkylthiocarbonyl, carboxyl, $C_{1-4}$ alkoxycarbonyl, $C_{1-4}$ alkoxythiocarbonyl, $C_{1-4}$ alkylcarbonyloxy, $C_{1-4}$ alkylthiocarbonyloxy, hydroxy-($C_{1-4}$) alkyl, mercapto-($C_{1-4}$) alkyl, perfluoro($C_{1-4}$)alkyl, nitro, amino, cyano, halogen, trifluoromethoxy, ethynyl, trimethylsilylethynyl, $C_{1-4}$ alkylsulphinyl, arylsulphinyl, $C_{1-4}$ alkylsulphonyl, arylsulphonyl, $C_{1-4}$ alkoxysulphinyl, $C_{1-4}$ alkoxysulphonyl, $C_{1-4}$ alkylcarbonylamino, $C_{1-4}$ alkoxycarbonylamino, aminosulphinyl, aminosulphonyl, aminocarbonyl, aminothiocarbonyl, $C_{1-4}$

alkylsulphinylamino, $C_{1-4}$ alkylsulphonylamino, $C_{1-4}$ alkoxysulphinylamino, $C_{1-4}$ alkoxysulphonylamino, ($C_{1-4}$ alkyl)carbonyl($C_{1-4}$ alkyl), nitro-($C_{1-4}$ alkyl), cyano-($C_{1-4}$ alkyl), ($C_{1-4}$ alkyl)C(=NOH), ($C_{1-4}$ alkyl)C(=$NNH_2$) or ($C_{1-4}$ alkoxy)C(=NH), being the above amino groups optionally substituted by one or two $C_{1-4}$ alkyl groups;

$R^3$ is hydrogen or $C_{1-4}$ alkyl; $R^4$ is $C_{1-4}$ alkyl, or $R^3$ and $R^4$ form a $C_{2-5}$ polymethylene chain;

$R^5$ is OH or -$OCOCH_3$ and $R^6$ is hydrogen, or $R^5$ and $R^6$ together form a bond;

$R^7$ is hydrogen or $C_{1-6}$ alkyl;

$R^8$ is a totally saturated $C_5$-$C_6$ cyclic or heterocyclic group selected from cyclopentyl, cyclohexyl, pyrrolidinyl, piperidinyl, piperazinyl, imidazolinyl, pyrazolidinyl, isothiazolidinyl, isoxazolidinyl, thiazolidinyl, oxazolidinyl or morpholinyl, which may be optionally substituted by one or two $R^9$ groups;

$R^9$ is a hydroxyl, oxo, $C_{1-6}$ alkyl, $C_{1-6}$ alkylcarbonyl, $C_{1-6}$ alkoxycarbonyl, $C_{1-6}$ alkoxycarbonylmethyl $C_{1-6}$ alkylaminocarbonyl, $R^{10}$-oxycarbonyl or $R^{10}$-($C_{1-4}$)alkoxycarbonyl group;

$R^{10}$ is a 5- or 6-membered monocyclic or a 9- or 10-membered bicyclic aryl or heteroaryl group, which may be optionally substituted by one or several fluorine, chlorine, bromine or iodine atoms, or hydroxyl, $C_{1-4}$ alkyl, $C_{1-4}$ alkoxy, cyano or nitro groups;

X is an oxygen or sulphur atom;

and the salts thereof,

which comprises:

(a) when in a compound of general formula **I**, $R^5$ is OH, $R^6$ is hydrogen, X is oxygen and $R^8$ is bound to the C=X group by a carbon atom, reacting a compound of general formula **II**, wherein $R^{1'}$and $R^{2'}$ are $R^1$ and $R^2$ as above defined or a group or atom convertible thereto, and $R^3$, $R^4$ and $R^7$ have the previously defined meaning,

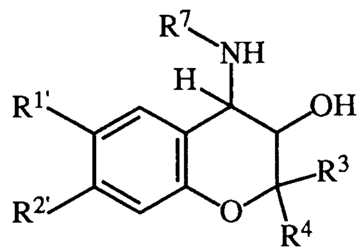

**II**

with an acid of general formula $R^{8a}COOH$ (wherein $R^{8a}$ means an $R^8$ group as above defined optionally substituted by one or two $R^9$ groups and which is bound to the C=X group by a carbon atom) in the presence of dicyclohexylcarbodiimide and 1-hydroxybenzotriazole in a suitable solvent such as dimethylformamide, or alternatively, reacting said compound **II** with an acid chloride of general formula $R^{8a}COCl$ (wherein $R^{8a}$ has the previously defined meaning) in the presence of a base in a suitable solvent such as chloroform, to give a compound of formula **I**, and optionally modifying its substituent $R^{8a}$ by standard chemical reactions involving the manipulation of amine protecting groups and the reaction of said amines with suitable electrophylic reagents; or

(b) when in a compound of general formula **I**, $R^5$ is OH, $R^6$ is hydrogen, X is oxygen and $R^8$ is bound to the C=X group by a nitrogen atom, reacting a compound of formula **II** with phenyl chloroformiate in a suitable solvent such as chloroform, to give a compound of general formula **XII**:

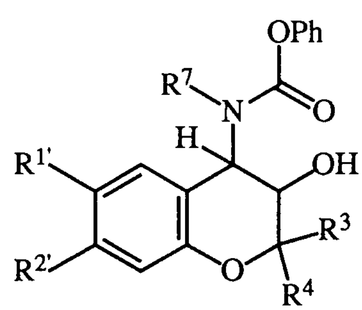

**XII**

wherein $R^{1'}$, $R^{2'}$, $R^3$, $R^4$ and $R^7$ have the previously defined meaning, and then reacting said compound **XII** with a compound of formula $R^{8d}$-H (**XIII**, wherein $R^{8d}$ is an $R^8$ group as above defined optionally substituted by one or two $R^9$ groups and which is bound to the C=X group by a nitrogen atom) in a suitable solvent such as butanol or else using **XIII** itself as solvent; or

(c) when in a compound of general formula **I**, $R^5$ is OH, $R^6$ is hydrogen, X is sulphur and $R^8$ is bound to the C=X group by a nitrogen atom, reacting a compound of formula **II** with carbon sulphide in the presence of a base such as potassium hydroxide in a suitable solvent such as dimethylformamide, and subsequently treating the intermediate obtained with methyl iodide in a suitable solvent such as methanol, to give a compound of general formula **XIV**:

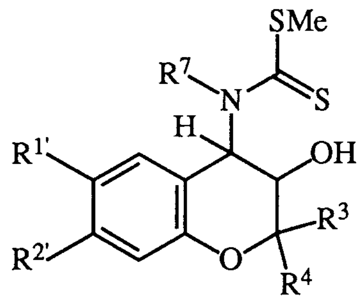

XIV

wherein $R^{1'}$, $R^{2'}$, $R^3$, $R^4$ and $R^7$ have the previously defined meaning, and finally reacting said compound **XIV** with a compound of general formula $R^{8d}$-H(**XIII**, wherein $R^{8d}$ has the previously defined meaning) in a suitable solvent such as butanol or else using **XIII** itself as solvent; or alternatively, in all cases wherein X is sulphur treating a compound of formula **I** wherein X is oxygen with a thiation agent such as Lawesson's reagent in a suitable solvent such as toluene;

(d) in all cases wherein $R^5$ is an acetoxy group and $R^6$ is hydrogen, reacting a compound of formula **I** wherein $R^5$ is hydroxyl and $R^6$ is hydrogen with acetic anhydride in the presence of a base such as pyridine;

(e) in all cases wherein $R^5$ and $R^6$ together form a bond, reacting a compound of general formula **I** wherein $R^5$ is OH and $R^6$ is hydrogen with a base such as sodium hydride or sodium hydroxide in a suitable solvent such as toluene or dioxane; or alternatively, reacting a compound of general formula **I** wherein $R^5$ is acetoxy and $R^6$ is hydrogen with a base such as 1,8-diazabicyclo(5.4.0)undec-7-ene (DBU) in an inert solvent such as toluene;

(f) optionally, interconverting the groups $R^1$, $R^2$, $R^{1'}$ and/or $R^{2'}$ in a compound of formula **I** or any intermediate of formula **II**, **XII** or **XIV** into other groups $R^1$ and/or $R^2$;

(g) and optionally, reacting a compound of formula **I** with an acid to give its corresponding acid addition salt.

**2.** A process according to Claim 1 in which the reagents and reaction conditions are so chosen as to prepare a compound of formula **I** wherein:
$R^1$ is a cyano or nitro group;
$R^2$ is hydrogen;
$R^3$ and $R^4$ are methyl; and
$R^5$, $R^6$, $R^7$, $R^8$ and X have the previously defined meaning.

**3.** A process according to Claim 1 in which the reagents and reaction conditions are so chosen as to prepare a compound of formula **I** wherein:
$R^1$ is a cyano group;
$R^2$ is hydrogen;
$R^3$ and $R^4$ are methyl;
$R^5$ is an hydroxyl group and $R^6$ is hydrogen; and
$R^7$, $R^8$ and X have the previously defined meaning.

**4.** A process according to Claim 1 in which the reagents and reaction conditions are so chosen as to prepare a compound of formula **I** wherein:

$R^1$ is a cyano group;
$R^2$ is hydrogen;
$R^3$ and $R^4$ are methyl;
$R^5$ is an hydroxyl group and $R^6$ is hydrogen;
$R^7$ represents hydrogen or a methyl group;
$R^8$ represents a cyclohexyl, 1-piperazinyl, 1-piperidinyl, or 1-pyrrolidinyl group, which may be optionally substituted by a $C_{1-6}$ alkyl group; and
X has the previously defined meaning.

**5.** A process according to Claim 1 in which the reagents and reaction conditions are so chosen as to prepare a compound of formula **I** wherein:
$R^1$ is a cyano group;
$R^2$ is hydrogen;
$R^3$ and $R^4$ are methyl;
$R^5$ is an hydroxyl group and $R^6$ is hydrogen;
$R^7$ represents hydrogen or a methyl group;
$R^8$ represents a 2-piperidinyl or 2-pyrrolidinyl group, which may be optionally substituted by an $R^9$ group, wherein $R^9$ is a $C_{1-6}$ alkoxycarbonyl, $R^{10}$-oxycarbonyl or $R^{10}$-($C_{1-4}$)alkoxycarbonyl group, being $R^{10}$ as above defined; and
X has the previously defined meaning.

**6.**
a) A process according to Claim 1 in which the reagents and reaction conditions are so chosen as to prepare *Trans* 3,4-dihydro-2,2-dimethyl-3-hydroxy-4-(1-pyrrolidinylthiocarbonylamino)-2H-1-benzopyran-6-carbonitrile and the salts thereof;
b) A process according to Claim 1 in which the reagents and reaction conditions are so chosen as to prepare *Trans* 3,4-dihydro-2,2-dimethyl-3-hydroxy-4-(1-piperidinyl-thiocarbonylamino)-2H-1-benzopyran-6-carbonitrile and the salts thereof;
c) A process according to Claim 1 in which the reagents and reaction conditions are so chosen as to prepare *Trans* 3,4-dihydro-2,2-dimethyl-3-hydroxy-4-(4-methyl-1-piperazinyl-thiocarbonylamino)-2H-1-benzopyran-6-carbonitrile and the salts thereof;
d) A process according to Claim 1 in which the reagents and reaction conditions are so chosen as to prepare *Trans* 3,4-dihydro-2,2-dimethyl-3-hydroxy-4-(1-*tert*-butoxycarbonyl-2-piperidinylcarbonylamino)-2H-1-benzopyran-6-carbonitrile and the salts thereof;
e) A process according to Claim 1 in which the reagents and reaction conditions are so chosen as to prepare *Trans* 3,4-dihydro-2,2-dimethyl-3-hydroxy-4-(1-phenyloxycarbonyl-2-piperidinylcarbonylamino)-2H-1-benzopyran-6-carbonitrile and the salts thereof;
f) A process according to Claim 1 in which the reagents and reaction conditions are so chosen as to prepare *Trans* 3,4-dihydro-2,2-dimethyl-3-hydroxy-4-(1-benzyloxycarbonyl-2-piperidinylcarbonylamino)-2H-1-benzopyran-6-carbonitrile and the salts thereof;
g) A process according to Claim 1 in which the reagents and reaction conditions are so chosen as to prepare *Trans* 3,4-dihydro-2,2-dimethyl-3-hydroxy-4-[(2S)-1-*tert*-butoxycarbonyl-2-pyrrolidinylcarbonylamino]-2H-1-benzopyran-6-carbonitrile and the salts thereof;
h) A process according to Claim 1 in which the reagents and reaction conditions are so chosen as to prepare *Trans* 3,4-dihydro-2,2-dimethyl-3-hydroxy-4-[(2S)-1-isobutoxycarbonyl-2-pyrrolidinylcarbonylamino]-2H-1-benzopyran-6-carbonitrile and the salts thereof;
i) A process according to Claim 1 in which the reagents and reaction conditions are so chosen as to prepare *Trans* 3,4-dihydro-2,2-dimethyl-3-hydroxy-4-[(2S)-1-phenoxycarbonyl-2-pyrrolidinylcarbonylamino)-2H-1-benzo pyran-6-carbonitrile and the salts thereof;
j) A process according to Claim 1 in which the reagents and reaction conditions are so chosen as to prepare *Trans* 3,4-dihydro-2,2-dimethyl-3-hydroxy-4-[(2S)-1-benzyloxycarbonyl-2-pyrrolidinylcarbonylamino)-2H-1-benzopyran-6-carbonitrile and the salts thereof;
k) A process according to Claim 1 in which the reagents and reaction conditions are so chosen as to prepare *Trans* 3,4-dihydro-2,2-dimethyl-3-hydroxy-4-[(2S)-1-[(3-methoxy-phenyl)methyloxycarbonyl]-2-pyrrolidinylcarbonylamino]-2H-1-benzopyran-6-carbonitrile and the salts thereof;
l) A process according to Claim 1 in which the reagents and reaction conditions are so chosen as to prepare *Trans* 3,4-dihydro-2,2-dimethyl-3-hydroxy-4-[(2S)-1-[(3-chloro-phenyl)methyloxycarbonyl]-2-pyrrolidinylcarbonylamino]-2H-1-benzopyran-6-carbonitrile and the salts thereof;

m) A process according to Claim 1 in which the reagents and reaction conditions are so chosen as to prepare *Trans* 3,4-dihydro-2,2-dimethyl-3-hydroxy-4-[(2S)-1-[(2-phenyl)ethyloxycarbonyl]-2-pyrrolidinylcarbonylamino]-2H-1-benzopyran-6-carbonitrile and the salts thereof;
n) A process according to Claim 1 in which the reagents and reaction conditions are so chosen as to prepare *Trans* 4-cyclohexylcarbonylamino-3,4-dihydro-2,2-dimethyl-3-hydroxy-2H-1-benzopyran-6-carbonitrile and the salts thereof.

European Patent Office

# EUROPEAN SEARCH REPORT

Application Number

EP 91 12 0417

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 126 311 (BEECHAM)<br>* page 2 - page 3; claims 1,25,26 *<br>--- | 1,8,9 | C07D311/68<br>C07D405/12<br>C07D405/14 |
| D,A | EP-A-0 095 316 (BEECHAM)<br>* page 5 - page 6; claims 1,23,24 *<br>--- | 1,8,9 | A61K31/35<br>A61K31/40<br>A61K31/445 |
| A | PATENT ABSTRACTS OF JAPAN<br>vol. 14, no. 439 (C-76)19 September 1990<br>& JP-A-2 172 984 ( YAMANOUCHI ) 4 July 1990<br>* abstract *<br>----- | 1,8,9 | A61K31/45<br>A61K31/50<br>A61K31/535 |
| | | | TECHNICAL FIELDS SEARCHED (Int. Cl.5)<br>C07D |

The present search report has been drawn up for all claims

| Place of search | Date of completion of the search | Examiner |
|---|---|---|
| THE HAGUE | 07 FEBRUARY 1992 | ENGLISH R.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)